# EUROPEAN PATENT APPLICATION

(11) **EP 2 289 484 A1**
(43) Date of publication of application: **02.03.2011**
(21) Application number: 09750409.6
(22) Date of filing: 03.03.2009
(51) Int. Cl.: A61K 8/85, A61K 8/02, A61K 8/19, A61K 8/27, A61K 8/29, A61K 8/87, A61Q 1/00, A61Q 5/00, A61Q 17/04, A61Q 19/00, C01B 13/14, C09C 3/10

(54) **SURFACE-TREATED POWDER AND COSMETIC COMPRISING THE SAME**

(30) Priority: 21.05.2008 JP 2008133521
(71) Applicant: Miyoshi Kasei, Inc., Saitama-shi Saitama 336-0975 (JP); Daikin Industries, Ltd., Osaka 530-8323 (JP)
(72) Inventor: HASEGAWA, Yukio, Saitama-shi Saitama 336-0975 (JP); TAKEUCHI, Yasushi, Saitama-shi Saitama 336-0975 (JP); IMAZEKI, Masafumi, Saitama-shi Saitama 336-0975 (JP); TSUBOYAMA, Hiroshi, Saitama-shi Saitama 336-0975 (JP); NOGUCHI, Koji, Saitama-shi Saitama 336-0975 (JP); MAEDA, Masahiko, Settu-shi Osaka 566-0044 (JP); FUKUDA, Teruyuki, Settu-shi Osaka 566-0044 (JP); MARUYAMA, Shigeru, Settu-shi Osaka 566-0044 (JP)
(74) Representative: Walcher, Armin
(86) International application number: PCT/JP2009/054377
(87) International publication number: WO 2009/142047

(57) **Abstract**

Provided is a surface-treated powder having excellent usability and adhesion to skin. The powder is coated with a surface-treating agent including (a) a fluorine-containing monomer of a general formula (I) and (b) an alkoxy group-containing monomer of a general formula (II). The powder is used for various cosmetics.

[Chemical formula 3] CH2=C(-X)-C(=O)-Y-[-(CH2)m-Z-]p-(CH2)n-Rf (I)

[Chemical formula 4) CH2=C(X1)-C(=O)-O-(RO)q-X2 (II)

## Description

### [Technical Field]

The present invention relates to a surface-treated powder, and more particularly a surface-treated powder in which at least a part of surfaces of powdery particles are coated with a compound containing at least a specific fluorine-containing copolymer, and cosmetics containing the same.

The surface-treated powders of the present invention are excellent in terms of usability and adhesion to skin, and have excellent affinity to other ingredients blended into cosmetic formulations. The cosmetics in which such surface-treated powders are blended are excellent in usability, cosmetic finish, long-lasting property, quality stability, and safety to living bodies and environment.

Further, the surface-treated powders of the present invention can be used preferably as a surface-treated powder for cosmetics. However, the surface-treated powders of the present invention can be applied to not only the cosmetics but also various fields including powdery fillers to be blended into inks, paints, resin master batches, papers and the like, ceramic materials, magnetic materials, rare earths, optical materials, electroconductive materials, voltage materials and the like.

### [Background Art]

Heretofore, powders surface-treated with perfluoroalkyl phosphoric acid esters and perfluoroalkyl silanes have been disclosed as fluorine-containing compound treated powders to be used for cosmetics (Patent Documents 1 and 2). Although such treated powders have water-repellent and oil-repellent properties and resistance against sweat and sebum secreted from skins, they have poor affinity with other raw materials for cosmetics, so that there is a considerable restriction in making formulations, and usability and adhesion to skin have been still insufficient. In addition, although such treated powders repel the sebum being present as an oil in air, there was a problem in the long-lasting property due to weakening of the oil-repellent property (sebum resistance) in a system in which sweat and sebum are coexistent (under an environment in the summer in which a man is likely to get sweat). As one having solved the above problem, surface-treated powders with fluorine-containing copolymers of a perfluoroalkyl acrylate and a silicone macromer or a polyalkylene glycol acrylate are disclosed (Patent document 3). However, there was a drawback that the oil-repellent property was deteriorated by the introduction of a silicone chain in the molecule. In addition, Patent document 4 discloses a surface-treated powder with a copolymer of a perfluoroalkyl acrylate and a polyalkylene glycol acrylate, and discloses that a combination of a fluoroalkyl group having six or less carbon atoms and HEMA gives a weak oil-repellent property in water (Patent document 4).

It is considered that compounds having fluoroalkyl groups with eight or more carbon atoms may produce PFOA as a fluorine-containing carboxylic acid through decomposition or metabolism, so that a fear of environmental accumulation has become apparent. Under the circumstances, the fluoroalkyl compounds to be used for treating powders have begun to be replaced by short-chain compounds having six or less carbon atoms. For example, a fluorine-containing copolymer having a fluoroalkyl group with six or less carbon atoms and excellent water-repellent and oil-repellent properties and a fluoroalkyl silane are disclosed (Patent Documents 5 and 6).

A graft copolymer having a fluorine segment and a hydrophilic segment in a molecule is disclosed for the purpose of water-repellent and oil-repellent purpose. Furthermore, polymers having sufficient solubility to water and being excellent in water resistance, softness and film strength are disclosed (Patent documents 7 and 8).

Patent Document 1: JP-A 62-250074
Patent Document 2: JP-A 2-218603
Patent Document 3: JP-A 2008-50620
Patent Document 4: JP-A 2000-290640
Patent Document 5: JP-A 2007-210939
Patent Document 6: JP-A 2007-238690
Patent Document 7: WO95/18194
Patent Document 8: JP-A 2005-213485

### [Disclosure of the Invention]

### [Problems to be solved by the invention]

However, there were problems in that the conventional fluorine-treated powders exhibiting water-repellent and oil-repellent properties were poor in solubility for other cosmetic materials, emulsifiability and dispersibility and had narrow variations in making formulations. Further, the conventional fluorine-containing compound treated powders are still insufficient in that usability are good while water-repellent and oil-repellent properties are possessed, adhesion to the skins is excellent, usability is excellent when used in cosmetics, and both cosmetic finish and long-lasting property are realized. In addition, there has not been a powder which is treated with a fluorine-containing compound having a fluoroalkyl group with six or less carbon atoms and which pays attention to safety for the living bodies and environment. Furthermore, there has not been a powder which is treated with a fluorine-containing compound having a fluoroalkyl group with six or less carbon atoms and which has excellent affinity for other ingredients to be blended into cosmetic formulations. Accordingly, a surface-treated powder has been sought, which has excellent affinity for other ingredients to be blended into cosmetic formulations and excellent oil-repellent property and usability in cosmetics, realizes both cosmetic finish and long-lasting property, and further pays attention to living bodies and environment.

The present invention is aimed at providing a fluorine-containing compound treated powder which has excellent usability and adhesion to skin, and the invention is aimed at providing a cosmetic into which the surface-treated powder of the present invention is blended and which has excellent usability, cosmetic finish, long-lasting property, quality stability, and safety to the living bodies and the environment.

### [Measures to solve the problems]

Having strenuously investigated to solve the above-mentioned problems, the present inventors discovered that a surface-treated powder coated with a compound containing at least a specific fluorine-containing copolymer can attain the object of the present invention. The above specific fluorine-containing copolymer is obtained by copolymerizing monomers essentially comprising (a) a fluorine-containing monomer expressed by the following general formula (I) with (b) a monomer essentially containing an alkoxy group-containing monomer expressed by the following general formula (II).

(1) That is, the present invention is directed to a surface-treated powder in which surfaces of particles of a powder to be surface-treated are coated with a surface-treating agent, characterized in that the surface-treating agent is a fluorine-containing copolymer obtained by copolymerizing a monomer essentially comprising (a) a fluorine-containing monomer expressed by the following general formula (I) with (b) an alkoxy group-containing monomer expressed by the following general formula (II).

(Chemical formula 1) CH2=C(-X)-C(=O)-Y [-(CH2)m-Z-]p-(CH2)n-Rf (I)

In the formula, X is a hydrogen atom, a methyl group, a fluorine atom, a chlorine atom, a bromine atom, an iodine atom, a CFX1X2 in which X1 and X2 are a hydrogen atom, a fluorine atom or a chlorine atom, a cyano group, a straight-chain or branched-chain fluoroalkyl group having 1 to 20 carbon atoms, a substituted or non-substituted benzyl group, or a substituted or non-substituted phenyl group;
Y is -O- or -NH-;
Z is a direct bond, -S- or -SO2-;
Rf is a fluoroalkyl group having 1 to 6 carbon atoms;
m is 1 to 10, n is 0 to 10, and p is 0 or 1.

(Chemical structure 2) CH2=C(X1)-C(=O)-O-(RO)_{q}-X2 (II)

In the formula, X1 is a hydrogen atom or a methyl group;
X2 is a hydrogen atom or an unsaturated or saturated hydrocarbon group having 1 to 22 carbon atoms;
R is an alkylene group having 2 to 4 carbon atoms in which a part of 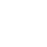 or an entire part of hydroxyl atoms may be replaced by a hydroxyl group or hydroxyl groups; and
q is an integer of 1 to 50.

The following are recited as preferred embodiments of the surface-treated powder according to the present invention.
(2) The surface-treated powder set forth in (1), wherein X 2 in the above formula (II) is a hydrogen atom.
(3) The surface-treated powder set forth in claim (1) or (2), wherein the surface-treated powder has water-repellent and oil-repellent properties.
(4) The surface-treated powder set forth in claim (1) or (2), wherein the surface-treated powder has hydrophilic and oil-repellent properties.
(5) The surface-treated powder set forth in any one of (1) to (4), wherein a crosslinkable monomer represented by the following general formula (III) is contained in constituting component components of the fluorine-containing copolymer.

(Chemical structure 3) CH2=C(X3)-C(=O)-O-(R1O)_{q}-C(=O)-C(X3)=CH2 (III)

In the formula, each of the X3 groups is a hydrogen atom or a methyl group; R1 is an alkylene group having 2 to 10 carbon atoms in which a part or an entire part of hydrogen atoms may be replaced by a hydroxyl group or hydroxyl groups; and q is an integer of 1 to 50.
(6) The surface-treated powder set forth in any one of claims (1) to (5), wherein the weight average molecular weight of the fluorine-containing copolymer is about 1,000 to about 1,000,000.
(7) The surface-treated powder set forth in any one of (1) to (6), wherein the alkoxy group-containing monomer (b) is 10 to 400 parts by weight relative to 100 parts by weight of the fluorine-containing monomer (a) in the fluorine-containing copolymer.
(8) The surface-treated powder set forth in any one of (1) to (7), wherein a coated amount of the fluorine-containing copolymer is 0.01 to 40 parts by weight relative to 100 parts by weight of the powder to be surface-treated.
(9) The surface-treated powder set forth in any one of (1) to (8), wherein the surface-treating agent further contains one or more kinds of compounds selected from the group consisting of the following compounds:
   other fluorine-containing compound or compounds rather than the fluorine-containing copolymer, an organopolysiloxane, an alkylsilane, a polyether-modified silane, an organic titanate, a polyolefin, a hydrogenated lecithin (including a form of a salt), an acylated amino acid (including a form of a salt or a composition), an acidic ester oil, a fatty acid (including a form of a salt) and a dextrin fatty acid ester.
(10) The surface-treated powder set forth in (9), wherein the fluorine-containing compound is at least one kind of a perfluoroalkyl phosphoric acid ester and a perfluoroalkyl silane having six or less carbon atoms, perfluoropolyether phosphoric acid ester and a perfluoropolyether silane.

(11) The surface-treated powder set forth in any one of (1) to (10), wherein 0.01 to 39.99 parts by weight of the fluorine-containing copolymer and 39.99 to 0.01 parts by weight of one or more kinds of the group of the above-mentioned compounds are coated for 100 parts by weight of the powder to be surface-treated.
(12) The surface-treated powder set forth in any one of (1) to (11), wherein the powder to be surface-treated is a powder usable for cosmetics.
(13) The surface-treated powder set forth in (12), wherein the powder usable for the cosmetics is an inorganic powder usable for the cosmetics.
(14) The surface-treated powder set forth in (13), wherein the inorganic powder is any one or more kinds of sericite, mica, kaolin, talc, silica, barium sulfate, boron nitride, titanium oxide, zinc oxide, iron oxide and a pearl pigment.
(15) The surface-treated powder set forth in (12), wherein the powder usable for the cosmetics is an organic powder usable for the cosmetics.

According to another aspect of the present invention, a cosmetic can be provided, which is characterized by containing the above surface-treated powder, that is, the surface-treated powder of the present invention. The cosmetic according to the present invention preferably further contains at least one of an oily ingredient, an aqueous ingredient and a surface-active agent as constituting ingredients besides the above surface-treated powder.

Furthermore, the cosmetic of the present invention is preferably any of a skin-care cosmetic, a hair cosmetic, a makeup cosmetic and an UV protecting cosmetic. A formulation of a product is preferably any of a liquid form, an emulsion form, a creamy form, a solid form, a paste form, a gel form, a powdery form, a multi-layer form, a mousse form and a spray form.

### [Effects of the invention]

The surface-treated powder according to the present invention is provided, which has excellent usability and adhesion to the skins and excellent affinity for other ingredients to be blended into the cosmetic formulations, and particularly the surface-treated powder for the cosmetics is provided. The surface-treated powder according to the present invention can be made water-repellent and oil-repellent or hydrophilic and oil-repellent. Here, the water-repellent and oil-repellent properties are intended to mean that the surface-treated powder is made to exhibit the water-repellent property and the oil-repellent property by changing the conformation of the polymer molecules based on a coating method or a coating amount of the specific fluorine-containing copolymer. The surface-treated agent can be made water-repellent and oil-repellent or the hydrophilic and oil-repellent, depending upon the purpose, the use and so on. The hydrophilic and oil-repellent properties are intended to mean that the surface-treated powder is made to exhibit the hydrophilic property and the oil-repellent property by changing the conformation of the polymer molecules based on the coating method and the coated amount of the specific fluorine-containing copolymer. The coated amount which exhibits the oil-repellent property or the hydrophilic property differs depending on the kind of the powder to be coated and the treating condition. The hydrophilic and oil-repellent powder of the present invention can be not only blended into aqueous ingredients but also applied as an emulsifier aid. Therefore, variations in making the formulations are largely expanded.

The cosmetics having good usability, cosmetic finish and long-lasting property can be easily and simply produced by blending the surface-treated powder of the present invention. In addition, the cosmetic into which the surface-treated powder is blended has excellent sebum resistance and transfer resistance, and exhibits a powder flocculation-preventing effect, a catalyst activity-suppressing effect, an effect for preventing an odor change and coloring with an interaction with UV absorbing organic powders and the like, a crystal deposition-preventing effect, an effect for preventing a reactivity with a water-soluble tackifier, a moisture loss suppressing effect and so on. Furthermore, the quality stability and the safety of the cosmetics can be largely improved by these effects. Therefore, the present invention is extremely industrially usable in the cosmetic field.

### [Best mode for carrying out the invention]

In the following, the best mode for carrying out the present invention will be explained in detail, while centering cases in which the surface-treated powders according to the present invention are used in the cosmetics, but the invention is not limited thereto. Note that the powder to be treated includes any form of the powder and the particles in the present invention.

### (Surface-treated powder according to the present invention)

The surface-treated powder according to the present invention is a surface-treated powder, preferably a surface-treated powder for cosmetics, and at least a part of surfaces of the particles of the powder to be surface-treated is coated with a surface-treating agent comprising a compound containing at least a specific fluorine-containing copolymer.

The surface-treating agent to be used in the present invention is the specific fluorine-containing copolymer. This fluorine-containing copolymer is obtained by copolymerizing monomers essentially comprising (a) a fluorine-containing monomer represented by the following general formula (I) with (b) an alkoxy group-containing monomer represented by the following general formula (II).

### Fluorine-containing monomer (a):

(Chemical formula 4) CH2=C(-X)-C(=O)-Y [-(CH2)ₘ-Z-]ₚ-(CH2)ₙ-Rf (I)

In the formula, X is a hydrogen atom, a methyl group, a straight-chain or branched-chain alkyl group having 1 to 21 carbon atoms, a fluorine atom, a chlorine atom, a bromine atom, an iodine atom, a CFX1X2 group in which X1 and X2 are a hydrogen atom, a fluorine atom, a chlorine atom, a bromine atom or an iodine atom, a cyano group, a straight-chain or branched-chain fluoroalkyl group having 1 to 20 carbon atoms, or a substituted or non-substituted benzyl group, a substituted or non-substituted phenyl group;
Y is -O- or -NH-;
Z is a single bond, -S- or -SO2-;
Rf is a fluoroalkyl group having 1 to 6 carbon atoms;
m is 1 to 10, n is 0 to 10, and p is 0 or 1.
The alkoxy group containing monomer (b) is expressed by the following general formula (II).

(Chemical formula 5) CH2=C(X1)-C(=O)-O-(R1O)_{q}-X2 (II)

In the formula, X1 is a hydrogen atom or a methyl group;
X2 is a hydrogen atom or a saturated or unsaturated hydrocarbon group having 1 to 22 carbon atoms;
R1 is an alkylene group having 2 to 4 carbon atoms in which a part or an entire part of hydrogen atoms may be replaced by a hydroxyl group or hydroxyl groups; and
q is an integer of 1 to 50.

In the above general formula (I), p is preferably 0. A preferable example of X is a hydrogen atom.

The fluorine-containing monomer (a) generally has a perfluoroalkyl group and/or a partially fluorinated fluoroalkyl group. The perfluoroalkyl group is preferred. The number of carbons of the Rf group is 1 to 6. The number of carbons of the Rf is preferably 4, 5 or 6, and particularly 6 is more preferred. Examples of the Rf group are -CF3,-CF2CF3, -CF2CF2CF3, -CF(CF3)2, -CF2CF2CF2CF3, -CF2CF(CF3)2, -C(CF3)3, -(CF2)4CF3, -(CF2)2CF(CF3)2, -CF2C(CF3)3, -CF(CF3)CF2CF2CF3, -(CF2)5CF3 and the like.

The fluorine-containing monomer (a) may be used of course singly, or two or more kinds may be used in a mixture.

As the fluorine-containing monomer (a), the following are recited.
CH2=C(-X)-C(=O)-O-(CH2)m-S-(CH2)n-Rf,
CH2=C(-X)-C(=O)-O-(CH2)ₘ-SO2-(CH2)ₙ-Rf
CH2=C(-X)-C(=O)-O-(CH2)n-Rf
CH2=C(-X)-C(=O)-NH-(CH2)ₙ-Rf
In the above formulae, X is a hydrogen atom, a methyl group, a fluorine atom, a chlorine atom, a bromine atom, an iodine atom, a CFX1X2 group in which X1 and X2 are a hydrogen atom, a fluorine atom or a chlorine atom, a cyano group, a straight-chain or branched-chain fluoroalkyl group having 1 to 20 carbon atoms, a substituted or non-substituted benzyl group or a substituted or non-substituted phenyl group; Rf is a fluoroalkyl group having 1 to 6; m is 1 to 10, and n is 0 to 10.

As concrete examples of the fluorine-containing monomer (a), the following can be recited, for example, but it is not limited thereto.
CH2=C(-H)-C(=O)-O-(CH2)2-S-Rf
CH2=C(-H)-C(=O)-O-(CH2)2-S(CH2)2-Rf
CH2=C(-H)-C(=O)-O-(CH2)3-SO2-Rf
CH2=C(-H)-C(=O)-O-(CH2)2-SO2-(CH2)2-Rf
CH2=C(-H)-C(=O)-O-(CH2)2-Rf
CH2=C(-H)-C(=O)-NH-(CH2)2-Rf
CH2=C(-H)-C(=O)-OCH2CH2N(C2H5)SO2-Rf
CH2=C(-H)-C(=O)-OCH2CH2N(CH3)SO2-Rf
CH2=C(-H)-C(=O)-OCH2CH(OCOCH3)CH2-Rf
CH2=C(-CH3)-C(=O)-O-(CH2)2-S-Rf
CH2=C(-CH3)-C(=O)-O-(CH2)2-S-(CH2)2-Rf
CH2=C(-CH3)-C(=O)-O-(CH2)3-SO2-Rf
CH2=C(-CH3)-C(=O)-O-(CH2)2-SO2-(CH2)2-Rf
CH2=C(-CH3)-C(=O)-O-(CH2)2-Rf
CH2=C(-CH3)-C(=O)-NH-(CH2)2-Rf
CH2=C(-CH3)-C(=O)-OCH2CH2N(C2H5)SO2-Rf
CH2=C(- CH3) - C(=O)-OCH2CH2N(CH3)SO2-Rf
CH2=C(-CH3)-C(=O)-OCH2CH(OCOCH3)CH2-Rf

CH2=C(-F)-C(=O)-O-(CH2)2-S-Rf
CH2=C(-F)-C(=O)-O-(CH2)2-S-(CH2)2-Rf
CH2=C(-F)-C(=O)-O-(CH2)2-SO2-Rf
CH2=C(-F)-C(=O)-O-(CH2)2-SO2-(CH2)2-Rf
CH2=C(-F)-C(=O)-O-(CH2)2-Rf
CH2=C(- F)-C(=O)-NH-(CH2)2-Rf
CH2=C(-Cl)-C(=O)-O-(CH2)2-S-Rf
CH2=C(-Cl)-C(=O)-O-(CH2)2-S-(CH2)2-Rf
CH2=C(-Cl)-C(=O)-O-(CH2)2-SO2-Rf
CH2=C(-Cl)-C(=O)-O-(CH2)2-SO2-(CH2)2-Rf
CH2=C(-Cl)-C(=O)-O-(CH2)2-Rf
CH2=C(-Cl)-C(=O)-NH-(CH2)2-Rf

CH2=C(-CF3)-C(=O)-O-(CH2)2-S-Rf
CH2=C(-CF3)-C(=O)-O-(CH2)2-S-(CH2)2-Rf CH2=C(-CF3)-C(=O)-O-(CH2)2-SO2-Rf
CH2=C(-CF3)-C(=O)-O-(CH2)2-SO2-(CH2)2-Rf CH2=C(-CF3)-C(=O)-O-(CH2)2-Rf
CH2=C(-CF3)-C(=O)-NH-(CH2)2-Rf
CH2=C(-CF2H)-C(=O)-O-(CH2)2-S-Rf
CH2=C(-CF2H)-C(=O)-O-(CH2)2-S-(CH2)2-Rf
CH2=C(-CF2H)-C(=O)-O-(CH2)2-SO2-Rf
CH2=C(-CF2H)-C(=O)-O-(CH2)2-SO2-(CH2)2-Rf
CH2=C(-CF2H)-C(=O)-O-(CH2)2-Rf
CH2=C(-CF2H)-C(=O)-NH-(CH2)2-Rf
CH2=C(-CN)-C(=O)-O-(CH2)2-S-Rf
CH2=C(-CN)-C(=O)-O-(CH2)2-S-(CH2)2-Rf
CH2=C(-CN)-C(=O)-O-(CH2)2-SO2-Rf
CH2=C(-CN)-C(=O)-O-(CH2)2-SO2-(CH2)2-Rf
CH2=C(-CN)-C(=O)-O-(CH2)2-Rf
CH2=C(-CN)-C(=O)-NH-(CH2)2-Rf

CH2=C(-CF2CF3)-C(=O)-O-(CH2)2-S-Rf
CH2=C(-CF2CF3)-C(=O)-O-(CH2)2-S-(CH2)2-Rf
CH2=C(-CF2CF3)-C(=O)-O-(CH2)2-SO2-Rf
CH2=C(-CF2CF3)-C(=O)-O-(CH2)2-SO2-(CH2)2-Rf
CH2=C(-CF2CF3)-C(=O)-O-(CH2)2-Rf
CH2=C(-CF2CF3)-C(=O)-NH-(CH2)2-Rf
CH2=C(-F)-C(=O)-O-(CH2)3-S-Rf
CH2=C(-F)-C(=O)-O-(CH2)3-S-(CH2)2-Rf
CH2=C(-F)-C(=O)-O-(CH2)3-SO2-Rf
CH2=C(-F)-C(=O)-O-(CH2)3-SO2-(CH2)2-Rf
CH2=C(- F) - C(=O)-O- (CH2)3 - Rf
CH2=C(- F) - C(=O)- NH- (CH2)3 -Rf

CH2=C(-Cl)-C(=O)-O-(CH2)3-S-Rf
CH2=C(-Cl)-C(=O)-O-(CH2)3-S-(CH2)2-Rf
CH2=C(-Cl)-C(=O)-O-(CH2)3-SO2-Rf
CH2=C(-Cl)-C(=O)-O-(CH2)3-S02-(CH2)2-Rf
CH2=C(-CF3)-C(=O)-O-(CH2)3-S-Rf
CH2=C(-CF3)-C(=O)-O-(CH2)3-S-(CH2)2-Rf
CH2=C(-CF3)-C(=O)-O-(CH2)3-SO2-Rf
CH2=C(-CF3)-C(=O)-O-(CH2)3-SO2-(CH2)2-Rf
CH2=C(-CF2H)-C(=O)-O-(CH2)3-S-Rf
CH2=C(-CF2H)-C(=O)-O-(CH2)3-S-(CH2)2-Rf
CH2=C(-CF2H)-C(=O)-O-(CH2)3-SO2-Rf
CH2=C(-CF2H)-C(=O)-O-(CH2)3-SO2-(CH2)2-Rf

CH2=C(-CN)-C(=O)-O-(CH2)3-S-Rf
CH2=C(-CN)-C(=O)-O-(CH2)3-S-(CH2)2-Rf
CH2=C(-CN)-C(=O)-O-(CH2)3-SO2-Rf
CH2=C(-CN)-C(=O)-O-(CH2)3-SO2-(CH2)2-Rf
CH2=C(-CF2CF3)-C(=O)-O-(CH2)3-S-Rf
CH2=C(-CF2CF3)-C(=O)-O-(CH2)3-S-(CH2)2-Rf
CH2=C(-CF2CF3)-C(=O)-O-(CH2)3-SO2-Rf
CH2=C(-CF2CF3)-C(=O)-O-(CH2)2-SO2-(CH2)2-Rf
In the above formula, Rf is a fluoroalkyl group having 1 to 6.

The alkoxy group-containing monomer (b) is a non-fluorine monomer, which is a compound (alkylene glycol (metha)acrylate) represented by the following general formula (II).

(Chemical formula 6) CH2=C(X 1)-C(=O)-O-(RO)q-X 2 (II)

In the formula, X1 is a hydrogen atom or a methyl group, X2 is a hydrogen atom or an unsaturated or saturated hydrocarbon group having 1 to 22 carbon atoms, R is an alkylene group having 2 to 4 carbon atoms in which a part or an entire part of hydrogen atoms may be replaced by a hydroxyl group or hydroxyl groups, and q is an integer of 1 to 50.

In the alkoxy group-containing monomer (b), q is preferably 1 to 30, more preferably 2 to 10, and particularly preferably 2 to 5.

In the general formula (II), it is preferably that R is ethylene or propylene, and particularly ethylene. R in the general formula (II) may be a combination of two or more kinds of alkylenes. In this case, at least one of Rs is preferably ethylene. As the combination of Rs, a combination of an ethylene group/a propylene group, or a combination of an ethylene group/a butylene group may be recited.

The alkoxy group-containing monomer (b) may be a mixture of two or more kinds.

As concrete examples of the alkoxy group-containing monomer (b), the following can be exemplified, for example, but it is not limited thereto.
CH2=CHCOO-(CH2CH2O)9-H
CH2=C(CH3)COO-(CH2CH2O)9-H
CH2=C(CH3)COO-(CH2CH2O)23-H
CH2=C(CH3)COO-(CH2CH2O)50-H CH2=C(CH3)COO-(CH2CH(CH3)O)9-H
CH2=CHCOO-(CH2CH(CH3)O)9-H
CH2=C(CH3)COO-(CH2CH(CH3)O)9-H
CH2=C(CH3)COO-(CH2CH2O)5-(CH2CH(CH3)O)2-H
CH2=C(CH3)COO-(CH2CH2O)8-(CH2CH(CH3)O)6-H
CH2=CX1-(CH2CH2O)n-CH3
CH2=CX1COO-(CH2CH(CH3)O)n-CH3
CH2=CX1COO-(CH2CH2O)5-(CH2CH(CH3)O)3-CH3

The fluorine-containing copolymer may contain a crosslinkable monomer. The crosslinkable monomer can be a compound which has at least two reactive groups and/or carbon-carbon double bonds and contains no fluorine. The crosslinkable monomer can be a compound which has at least two carbon-carbon double bonds, or a compound which has at least one carbon-carbon double bond and at least one reactive group. Examples of the reactive group are a hydroxyl group, an epoxy group, a chloromethyl group, a blocked isocyanate, a carboxyl group or the like. In the present invention, a monomer having an amino group is not used.

The crosslinkable monomer is preferably a non-fluorine crosslinkable monomer, and a di(metha)acrylate is more preferable.

The crosslinkable monomer is particularly preferably a compound (alkylene glycol di(metha)acrylate) represented by a general formula:

(Chemical formula 7) CH2=C(X 3)-C(=O)-O-(R 1 O)q- C(=O) -C(X 3)=CH2 (III)

A compound expressed by the chemical formula (an alkylene glycol di-(metha)acrylate) is particularly preferable, in which each X3 is a hydrogen atom or a methyl group;
R1 is an alkylene group having 2 to 10 carbon atoms in which a part or an entire part of hydrogen atoms may be replaced by a hydroxyl group or hydroxyl groups; and
q is an integer of 1 to 50.
Meanwhile, the number of carbon atoms of R1 is 2 to 10, for example, 2 to 6, and particularly preferably 2 to 4, for examples. R1 is preferably an ethylene group.

When the molecular weight and the mole ratio of each of the concerned monomers constituting the fluorine-containing copolymer are controlled, a coating characteristic and a film-forming power of the fluorine-containing copolymer upon the powder particles, a powder particle-dispersing ability, hygroscopic/moisture-retaining properties and so forth can be afforded. The weight average molecular weight of the fluorine-containing copolymer of the present invention can be set to around 1,000 to 1,000,000, and preferably around 5,000 to 500,000. If it is less than 1,000, the fluorine-containing copolymer is not suitable for the surface treatment with the effects due to weak coating film-forming ability, while if it is 1,000,000, not only a solubility of the polymer gets poorer, but also the dispersibility of the surface-treating powder get worse. Meanwhile, this weight average molecular weight is a value determined by a gel permeation chromatography when converted to polystyrene.

For example, the fluorine-containing copolymer may be such that assuming that the fluorine-containing monomer (a) is contained in an amount of 100 parts by weight, the amount of the alkoxy group-containing monomer (b) can be set to 10 to 400 parts by weight, preferably 25 to 150 parts by weight, more preferably 43 to 100 parts by weight relative to 100 parts by weight of the fluorine-containing monomer (a). If it is less than 10 parts by weight, the hydrophilic property is not obtained, while if it is more than 400 parts by weight, the oil repellency drops. If the crosslinkable monomer is contained, the amount of the crosslinkable monomer can be set to not more than 30 parts by weight, for example, 0.1 to 20 parts by weight, particularly 0.5 to 10 parts by weight. If it is more than 30 parts by weight, a coated film becomes get hard, which gives poor aesthetics. Note that the fluorine-containing copolymer to be used in the present invention can be synthesized by a method disclosed in JP-A 2000-290640, for example, but the invention is not limited thereto. The polymerization of the fluorine-containing copolymer is not limited, and for example, various polymerization methods such as a mass polymerization, a solution polymerization, an emulsion polymerization, a radiation polymerization and so on can be selected. For example, the solution polymerization using an organic solvent or the emulsion polymerization using water or an organic and water in combination is generally selected. A treating liquid is generally prepared by dilution with water or by emulsification in water with addition of an emulsifier after the polymerization.

A surface-treating agent to be used in the present invention is preferably in a form of a solution, an emulsion or an aerosol. The surface-treating agent contains a fluorine-containing copolymer and a medium (for example, a liquid medium such as an organic solvent or water), for example. The concentration of the fluid-containing copolymer in the surface-treating agent can be set to 0.01 to 50 weight%, for example.

The coating amount for the powder to be surface-treated in the above varies, depending upon the chemical composition, the particle diameters, presence or absence of the porousness, an oil absorption amount, a water absorption amount and a specific surface area of the powder to be surface-treated, and it is preferable that the coating amount of the above specific fluorine-containing copolymer is 0.1 to 40 parts by weight relative to 100 parts by weight of the powder to be surface-treated. More preferably, it is 0.5 to 35 parts by weight. If it is less than that, sufficient effects are not obtained, while if it is more than that, flocculation of the powder particles occurs with the polymer, so that not only performance of the surface-treated powder is deteriorated and the effects in the cosmetic becomes insufficient, but also it tends to be uneconomical.

In order that improvement on aesthetics and adhesion to skin of the surface-treated powder coated with the specific fluorine-containing copolymer, improvement on affinity to other ingredients to be blended into cosmetic preparations, and further aesthetics, cosmetic finish and long-lasting property of the cosmetics in which such surface-treated powders are blended may be more effectively exhibited, it is effective that the surface treatment is carried out with the above specific fluorine-containing copolymer and a compound other than the above specific fluorine-containing copolymer in a composite manner, that is, it is effective that the surface-treating agent further contains other compound than the above specific fluorine-containing copolymer.

Other compound than the above specific fluorine-containing copolymers is one or more kinds compounds selected from among, for example, fluorine-containing compounds such as a perfluoroalkyl phosphoric acid ester, a perfluoropolyether phosphoric acid ester, a perfluoropolyether silane, a perfluoroalkyl silane and so forth, an organopolysiloxane, an alkyl silane, an organic titanate, a polyolefin, a hydrogenarated lecithin (including its salts), an N-acyl amino acid (including its salts or compositions), an acidic ester oil, a fatty acid (including its salts) and dextrin fatty acid ester.

In the present invention, as those other fluorine-containing compounds than the above specific fluorine-containing copolymer, which effects the surface treatment together with the specific fluorine-containing copolymer in the composite manner, a perfluoroalkyl phosphoric acid ester, a perfluoropolyether phosphoric acid ester, a perfluoropolyether silane, a perfluoroalkyl silane, a perfluoropolyether-modified polyurethane, a perfluoropolyether-modified acrylate, a perfluoroalkyl-modified silicone, a perfluoropolyether-modified silicone and the like are recited.

As the perfluoroalkyl phosphoric acid ester, a publicly known compound of the following formula (1) can be used, and a preferred compound is a compound expressed by the following formula (1) in which the number of carbon atoms in Rf is not more than 6.

(Chemical formula 8) [RfCnH2nO]yPO[OM]3-y (1)

In the formula, Rf denotes a perfluoroalkyl group or a perfluorooxyalkyl group having 1 to 6 carbon atoms, which may be a straight chain or branched-chain form and single-chain long or multiple-chain long, n denotes an integer of 1 to 12, y denotes an integer of 1 to 3, and M denotes hydrogen, an alkali metal, ammonium or substituted ammonium.

As the perfluoropolyether phosphoric acid ester, publicly known compounds can be used, and a preferred compound is a compound represented by the following formula (2).

(Chemical Compound 9) [F(CF2)3O[CF(CF3)CF2O]mCF(CF3)CnH2nO]wP(O)(OH)3-w (2)

In the formula, m is an integer of 0 to 50, n is an integer of 1 to 16, and w is 1 or 2. A phosphoric acid portion may be in a form of a salt, an amine salt or an ammonium salt of an alkali metal such as Na, K or the like. In addition, one modified at both end modified with phosphoric acid (FOMBLIN HC/P2-1000:Solvay Solexis S.p.A.) may be used.

As the perfluoropolyether silane, publicly known compounds described in WO 97/07155 and JP-A 2008-214229 can be used.

As the perfluoroalkyl silane, publicly known compounds having the following formula (3) can be used, and preferred compounds are those expressed by the following formula (3) in which the number of carbon atoms in Rf is not more than 6.

(Chemical formula 10) (RfCnH2n)aSiX4-a (3)

In the formula, Rf is a perfluoroalkyl group or a perfluorooxyalkyl group having 1 to 6 carbon atoms, which may be in a straight chain or branched-chain form and single-chain long or multiple-chain long; n is an integer of 1 to 12; a is an integer of 1 to 3; X is an alkoxy group, a hydroxyl group; an amino group or a halogen atom.

DYNASYLAN F-8261 (Evonik Degussa Company) or the like is recited as a commercial product.

As the perfluoropolyether-modified polyurethane, FOMBLIN AN-5 and FOMBLIN CAT-5 commercially available from Solvay Solexis S. p. A., etc. are recited. As other fluorine-containing compounds useful as a surface-treating agent, a carboxyl-modified perfluoropolyether, an alcohol-modified perfluoropolyether, an isocyanate-modified perfluoropolyether, an ester-modified perfluoropolyether, a trifluoroalkyl dimethyltrimethylsiloxy silicate, a fluoropropyl methicone, etc. are recited.

As the organopolysiloxane, an organopolysiloxane may be selected, which may have or may not have a functional group. As an organopolysiloxane having a functional group, an organopolysiloxane can be selected, which possesses a linear structure in which side chains or both ends or one end is provided with a functional group, and a compound represented by the following formula (4) are recited.

(Chemical Formula 11) (R53SiO)(R42SiO)m(R42SiO)n(SiR53) (4)

In the above formula (4), R4s are all mutually independent, and each of them denotes a lower alkyl group having 1 to 4 carbon atoms or a hydrogen atom. R5s present in plural are also all mutually independent and each denote any of a hydrogen atom, a hydroxyl group and a lower alkoxy group having 1 to 4 carbon atoms; m and n denote an integer of 1 or more, respectively, and m + n is an integer of 2 to 1000.

As the organopolysiloxane, KF99, KF9901, X-24-9171, X-24-9221, etc. commercially available from Shin-Etsu Chemical Co., Ltd. are concretely recited. As other organopolysiloxanes, silicone, acryl silicone, silicone acryl, etc. having trimethylsiloxy silicate, a reactive group such as a dimethyl group or an alkyl group at a side chain can be used. Those commercially available from Shin-Etsu Chemical Co., Ltd. are KF-96 series, KF-9908 (triethoxysilyl ethyl polydimethylsiloxy ethyl methicone), KF-9909 (triethoxysilyl ethyl polydimethylsiloxyethyl hexyl dimethicone), KP-541, KP-545, KP-575, KP-561, KP-574, KF-7312F, KF-7312J, KF-7312K, KF-9001, et., and as those commercially available from Dow Corning Company. Ltd., SH1107C, BY11-018, DC593, FA-4001, CM Silicone Acrylate, and FA-4002 ID Silicone Acrylate can be also used. In addition, cyclic methyl hydrogen silicone such as tetrahydro tetramethyl cyclotetrasiloxane or the like, methyl hydrogen silicone, amino-modified silicone, carboxyl-modified silicone, silicone alkoxy oligomer, triethoxysilyl fluoride-modified silicone, triethoxy nonamethyl penta siloxane, triethoxy triisopropyl disiloxane, triethoxy butyl dimethyl disiloxane or the like can be also used.

As the alkylsilane to be used in the present invention, one having a fundamental skeleton with an RnSiX4-n structure is selected. Here, R is a straight-chain or branched-chain alkyl group, X is hydrogen or an alkoxy group, amino group or a halogen atom. The length of the alkyl chain may be 1 to 18 carbon atoms, and octyltriethoxysilane, octadecyltriethoxysilane and the like are concretely recited. Further, polyether-modified alkoxysilane in which an ether group is intramolecularly introduced may suffice. As commercial goods, Z-6341 of Dow Corning Corporation, DYNASYLAN4140 of Degussa GmbH, KBM-641 and KBE-13 of Shin-Etsu Chemical Co., Ltd., Prosil 9202 of Clariant Company Ltd., etc. are recited.

As the organic titanate to be used in the present invention, one having a fundamental skeleton with a structure of (C n H2n-1COO) a T i (O C mH2m-1) b is selected. In this case, n is an integer of 1 to 26, m is an integer of 1 to 12, and b are each an integer of 1 to 3, and a + b = 4. Note that the alkyl group shown here may be a straight-chain or branched-chain form, and may be single-chain long or composite-chain long. As commercially available one, isopropyltriisostealoyl titanate (Plenacto KR-TTS:Ajonomoto Co., Inc.)

As the polyolefin to be used in the present invention, a polyolefin resin such as polyethylene, polypropylene or the like which has at least one carboxyl group in a molecule can be recited. For example, a low molecular polyethylene as a publicly known chemical compound described in JP-A 63-179972 and having a molecular weight of 500 to 20,000 with a melting point of 40°C or more, an oxidized polyethylene obtained by the oxidation of polypropylene, maleinated polyethylene, oxidized polypropylene and the like are recited.

Hydrogenerated lecithin to be used in the present invention has only to be a glyceride having a phosphate group, and as such a hydrogenerated lecithin, natural lecithin extracted from egg yolk, soybean, corn, oilseed rape or the like, lysolecithin, and a hydrogenerated synthetic lysolecithin having an iodine value of 15 or less can be recited. The hydrogenerated lecithin to be used in the present invention may be in a form of a salt. The salt form is preferably a water-insoluble hydrogenerated lecithin metal salt of such Al, Mg, Ca, Zn, Zr, Ti or the like is preferred. The hydrogenerated lecithin having a melting point of 50° C or more (including forms of salts) is particularly preferable. For example, publicly known compounds described in JP-A 60-184571, JP-A 60-190705, and JP-B 4-58443, e.g., hydrogenerated egg yolk No. 5 of Asahi Kasei Corporation, hydrogenerated soybean phosphatide-basis LS-60HR of Nisshin Olio Group, Ltd, etc. are recited. As other compounds having phosphate group, LIPIDURE Series commercial available from NOF Corporation, such as methacryloyloxyethyl phosphorylcholine is recited.

Acylated amino acid to be used in the present invention is an acylated compound of an amino acid selected from a saturated fatty acid, asparagic acid and glutamic acid, alanine having 12 to 20 carbon atoms, lysine, glycine, sarcosine, proline and hydroxyproline, fully-hydrolyzed products of peptide or cyclopeptide originated from plants such as wheat, green beans, proso millet and the like, peptides originated from animals, etc., and carboxyl groups of the amino acids may be free or in a form of a salt of K, Na, Fe, Zn, Ca, Mg, Al, Zr, Ti or the like. Specifically, Amisoft HS-21P, Amihope LL (lauroyl lysine), etc. commercially available from Ajinomoto Co., Ltd., Soypon SLP, Soypon SCA and Arapon AMP commercially available from Kawaken Fine Chemicals Co., Ltd., SEPILIFT DPHP, etc. commercially available from France SEPPIC Co., ltd., Sarcosinate MN, etc. commercially available from Nikko Chemicals Co., Ltd., Pellicer amino foamer, amino surfactant, amino coat, etc. commercially available from Asahi Kasei Corporation can be recited. These acylated amino acids may be in the form of compositions with fatty acids. As the acylated lipoamino acid composition, SEPIFEEL ONE (a composition composed of four ingredients of palmitoyl proline, palmitoyl sarcosine, palmitoyl glutamic acid and palmitic acid) commercially available from SEPPIC Co., Ltd. is recited.

The acidic ester oil to be used in the present invention includes an ester compound having the total carbon atoms of not less than 16, which can be obtained by reacting one or more kinds of alcohols having 1 to 36 carbon atoms with one or more kinds of carboxylic acids having 1 to 36 carbon atoms, and the ester compound having an acid value of 15 or more is preferable. As publicly known compounds described in JP-A 2004-51945, Salacos MIS (isostearyl sebacate), Salacos MOD (azelaic acid octyldodecanol), Salacos 1A (adipic acid octyl dodecanol), Salacos HD (dimer acid octyl dodecanol), etc. are recited.

As the fatty acids to be used in the present invention, straight-chain or branched-chain saturated or unsaturated fatty acids having 12 to 22 carbon atoms are recited, and for example, fatty acids such as lauric acid, myristic acid, palmitic acid, stearic acid, oleic acid, linoleic acid, palmitoleic acid, behenic acid, lignoceric acid, 2-ethylhexanoic acid, isotridecanoic acid, isomyristic acid, isopalmitic acid, isostearic acid, behenic acid and the like, or their alts of metals such as Ca, Mg, Zn, Zr, Al, Ti and the like are recited.

In addition, as the compound for the surface treatment in a composite manner with the specific fluorine-containing copolymer, which is to be used in the present invention, an alkyl phosphoric acid and its salt, an alkyl carboxylic acid and its salt, an alkyl sulfuric acid and its salt, an amido sulfonic acid and its salt, an alkyl ether carboxylic acid and its salt, a polyoxyethylene alkyl ether phosphoric acid and its salt, a polyoxyethylene alkyl ether sulfuric acid and its salt, methyl silanol/triPEG-8 palm oil fatty acid glyceride, dextrin fatty acid ester and the like are recited. As commercially available products, there are AKYPO RLM45NV, ECT-3NEX and so on commercially available from NIKKOL Co., Ltd. monosiliol C and so on commercially available from EXSYMOL Co., Ltd., but they are not restrictive. These compounds can be combined for treatment, depending upon an aimed effect of a surface-treated powder.

Regarding the specific fluorine-containing copolymer (herein referred to as a treating agent A) and other ingredient (referred to as a treating agent B) to be used for the surface treatment in the composite manner together with that, B being one or more kinds of the other compounds selected from among e.g., other fluorine-containing compound than the above-mentioned fluorine-containing copolymer such as a perfluoroalkyl phosphoric acid ester, a perfluoropolyether phosphoric acid ester, a perfluoropolyether silane, a perfluoroalkyl silane, etc., an organopolysiloxane, an alkyl silane, an organic titanate, a polyolefin, hydrogenerated lecithin (including its salt), an N-acylamino acid (including its salt or composition), an acidic ester oil, a fatty acid (including its salt), a dextrin fatty acid ester, the blending ratio between A and B is preferably A:B = 39.0 to 0.1 parts : 0.1 to 39.0 parts. More preferably, A:B is 34.5 to 0.5 parts: 0.5 to 34.5 parts. The blending ratio differs, depending upon the kind of the powder, the kind of the cosmetic and the blending amount for the cosmetic, but if it is out of the above ratio, the effects with the cosmetic tends to decrease.

As the powder to be used in the present invention, powders usable in various fields, that is, powders usable in the cosmetics can be selected when they are used in the cosmetics. In this case, either inorganic powders or organic powders may be selected.

For example, as the inorganic powders, recitation is made of boron nitride, sericite, natural mica, fired mica, synthetic mica, synthetic sericite, alumina, mica, talc, kaolin, bentonite, smectite, calcium carbonate, magnesium carbonate, calcium phosphate, silicic anhydride, magnesium oxide, tin oxide, iron oxide, yttrium oxide, chromium oxide, titanium oxide, zinc oxide, cerium oxide, aluminum oxide, magnesium oxide, chromium hydroxide, iron blue, ultramarine blue, calcium phosphate, aluminum hydroxide, barium sulfate, magnesium sulfate, silicic acid, aluminum magnesium silicate, calcium silicate, barium silicate, magnesium silicate, aluminum silicate, strontium silicate, silicate carbide, magnesium fluoride, metal tungstate salts, magnesium aluminate, magnesium methasilicate aluminate, chlorohydroxyl aluminum, clay, zeolite, hydroxyapatite, ceramic powder, spinel, mullite, cordierite, aluminum nitride, titanium nitride, silicon nitride, lanthanum, samarium, tantalum, terbium, europium, neodymium, Mn-Zn ferrite, Ni-Zn ferrite, silicon carbide, cobalt titanate, barium titanate, iron titanate, lithium cobalt titanate, cobalt aluminate, antimony-containing tin oxide, tin-containing indium oxide, magnetite, aluminum powder, gold powder, silver powder, platinum powder, copper powder, precious metal colloid, iron powder, zinc powder, cobalt blue, cobalt violet, cobalt green, low level titanium oxide, finely particulate titanium oxide, butterfly-shaped barium sulfate, flower petal-shaped zinc oxide, tetrapod-shaped zinc oxide, and finely particulate zinc oxide. As pearl pigments, pearl pigments such as titanium oxide-coated mica, titanium oxide-coated mica, titanium oxide-coated synthetic mica, titanium oxide-coated silica, titanium oxide-coated synthetic mica, titanium oxide-coated talc, zinc oxide-coated silica, titanium oxide-coated colored mica, colcothar-coated mica titanium, colcothar/black iron oxide-coated mica titanium, carmine-coated mica titanium, iron blue-coated mica titanium, and so on are recited. Concretely, recitation is made of IRIODIN (registered trade mark) series, TIMIRON (registered trade mark) COLORONA series (registered trade mark), DICHRONA (registered trade mark) series, XIRONA (registered trade mark) series and RONASTAR (registered trade mark) series of MERCK & Co., Ltd., DESERT REFLECTIONS series, TIMICA series, FLAMENCO series, CLOIZONNE series, DUOCROME series, GEMTONE series, CELLINI series, MEARLMAID series, REFLECKS series, CHROMA-LITE series and COSMICA series of BASF Co., Ltd., PRESTIGE (registered trade mark) series, VISIONAIRE (registered trade mark) series and MIRAGE series of ECKART Co., Ltd., Metashine (registered trade mark) of NSG Group Co., Ltd., and PROMINENCE (registered trade mark) of NIHON KOKEN KOGYO Co., Ltd., Cosmetica White Pearl series, sharon Pearl series and so on of CQV Co., Ltd., Precioso White Pearlescent Pigments series and so on of Taizu Co., Ltd. Recitation is made of effect pigments such as aluminum flake, silica flake, alumina flake, glass flake and so on, colcothar-coated mica, carmine, titanium oxide-coated borosilicic acid (sodium/calcium), titanium oxide-coated borosilicic acid (calcium/aluminum), bismuth oxychloride, fish argentine, stainless powder, tourmaline powder, powders of crushed jewels such as sapphire, ruby, etc., mango violet, glass fibers, carbon fibers, silicon carbide fibers, alumina fibers, beta-Wollastonite, Zonolite, potassium titanate fibers, aluminum borate fibers, basic magnesium sulfate fibers, silicon nitride fibers, etc.

As organic powders, for example, recitation is made of metal soaps, an N-mono long-chain acyl basic amino acid, an amido sulfonic acid polyvalent metal salt, amber powder, carbon black, chelated tar dye, chelated natural colors , polyamide powder, polyester powder, polyethylene powder, polyurethane powder, polypropylene powder, polystyrene powder, benzoguanamine powder, polymethyl benzoguanamine powder, PTFE powder, cellulose powder, silk powder, silicone powder, synthetic resin powders of such as divinylbenzene-styrene copolymer, vinyl resin, urea resin, phenol resin, fluorine resin, silicon resin, acryl resin, melamine resin, epoxy resin, polycarbonate resin, etc., finely crystalline fiber powders such as nylon fibers, polyester fibers, etc., starch powder, CI pigment yellow, CI pigment orange, etc. As the tar dye, recitation is made of Red No. 3, Red No. 10, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, Orange No. 207, etc.; as the natural colors, recitation is made of powders of natural powders of such as carmine, laccaic acid, carthamin, brazilin, crocin, chlorophyll, beta-carotene, safflower powder, etc. Forms of the powders may be powdery or fibrous. When cosmetics are selected as usage, particle diameters may be in a range of around 1nm to 2,000 µ m provided that the powders can be blended into the cosmetics.

Meanwhile, in the present invention, the above-mentioned powder (the powder before the surface treatment) may be a powder in which two or more kinds are combined in complex. For example, recitation is made of a powder in which aluminum hydroxide is combined with surfaces of particles of mica or a pearl pigment (Excel mica JP-2 or Excel mica: Miyoshi Kasei, Inc.), a powder in which hydroxyapatite and zinc oxide are combined with surfaces of sericite or a pearl pigment (Powder Lavie: Miyoshi Kasei, Inc.), a powder in which finely particulate titanium oxide and finely particulate zinc oxide are dispersed and mixed (TZ-POWDER TYPE 1: Miyoshi Kasei), a powder in which talc, fine zinc flower and finely particulate titanium oxide are dispersed and mixed (TZ-POWDER TYPE 2: Miyoshi Kasei, Inc.), a mixture of dimethicone/vinyldimethicone and silica (Dow Corning 9701 Cosmetic Powder), a mixture of dimethicone/vinyldimethicone and titanium oxide (Dow Corning EP-9261 TI Cosmetic Powder), a mixture of dimethicone/vinyldimethicone and alumina (Dow Corning EP-9293 AL Cosmetic Powder), a mixture of dimethicone/vinyldimethicone and lauroyl lysine (Dow Corning EP-9289 LL Cosmetic Powder), COVERLEAF series and CONCELIGHT series commercially available from JGC C&C, and so on.

Further, in order to improve affinity or firm adhesion with the surface-treating agent, the powder to be surface-treated in the present invention may be preliminarily coated with at least one kind of oxides or hydrous oxides of aluminum, potassium, magnesium, cerium, silicon, zirconium, titanium, zinc, iron, cobalt, manganese, nickel and tin, for example. These compounds may be preliminarily coated before the surface treatment is performed with the surface-treating agent in the present invention or when the surface treatment is performed. In addition, particles of the oxide or hydrous oxide may be coated or may be precipitated on surfaces of the powder particles to be surface-treated. The coating amount of such a third substance is preferably a minimum amount required to exhibit the function of the fluorine-containing compound by the surface-treated powder.

In the present invention, a method by which the powder is coated with the surface-treating agent, particularly the specific fluorine-containing copolymer is not specifically limited, and the coating can be carried out by a publicly known method. The surface-treating method is broadly classified into a dry type method and a wet type method. The treatment is carried out by mixing and contacting the surface-treating agent and the powder to be used in the present invention for a given time period with use of a stirring device, a milling device, a mixer or a dispersing device such as a henschel mixer, a ball mill, a jet mill, a kneader, a planetary mixer, a pony mixer, a sand mill, an attritor, a ribbon blender, a dispermixer, a homo mixer, an extruder or the like. The treatment may be carried out, while energy such as a mechanochemical mechanical force, plasma, flame, UV rays, electron beams, overheated steam, laser beams, electromagnetic waves or the like is being applied. As the wet type method, the treatment can be done by dispersing the powder and the surface-treating agent in water, a solvent or a supercritical fluid (water, CO2 or the like), mixing and contacting them and thereafter evaporating the solvent.

Further, when the surface treatment is carried out in complex with the specific fluorine-containing copolymer to be used in the present invention and at least one or more kinds of compounds selected from among fluorine-containing compounds such as perfluoroalkyl phosphoric acid, a perfluoropolyether phosphoric acid ester, a perfluoropolyether silane, a perfluoroalkyl silane and the like, an organopolysiloxane, an alkyl silane, an organic titanate, a polyolefin, a hydrogenerated lecithin (including a salt thereof, N-acylamino acid (including a salt or a composition thereof), an acidic ester oil, a fatty acid (including a salt thereof) and a dextrin fatty acid ester, the surface treatment can be carried out by mixing and contacting the powder with the surface-treating agent further containing the above compound in the same manner as in the case where the surface treatment is carried out by the above specified fluorine-containing copolymer alone. In this case, an optimum coating production method may be selected, depending upon properties (liquid, solid, gel or the like) and physical properties (melting point, boiling point, glass transition temperature, solubility, reactivity, etc.) of a compound selected for the surface treatment in complex, and any particular limitation is not imposed by the producing method.

When the surface is treated in a composite manner, a method in which after the surface treatment is first carried out by a specific fluorine-containing copolymer, coating is performed with other surface-treating ingredient than the specific fluorine-containing copolymer, a method in which coating is performed simultaneously with the specific fluorine-containing copolymer and other surface-treating ingredient than the specific fluorine-containing copolymer, a method in which coating is performed first with other surface-treating ingredient than the specific fluorine-containing copolymer, followed by coating with the specific fluorine-containing copolymer, etc. are recited.

For either of the case where the surface treatment is carried out with the above specific fluorine-containing copolymer alone and the case in which the surface treatment is carried out in the complex manner, a preferred coating method is a method in which after the particles of the powder to be coated are preliminarily dispersed in air or a liquid or in coexistence with other powder, followed by coating, or a method in which coating is simultaneously performed.

Since the specific fluorine-containing copolymer of the present invention possesses oil-repellent groups and hydrophilic groups in its molecule, water-repellent and oil-repellent properties are exhibited by exposing only the oil-repellent groups from the surfaces of the particles of the powder. In order to obtain excellent affinity and dispersion with other ingredients to be blended into a cosmetic formulation of the surface-treated powder having the oil-repellent property, the hydrophilic and oil-repellent properties are advantageous. Since both the oil-repellent groups and the hydrophilic groups can be exposed onto the surfaces of the particles of the powder by the fluoride-containing copolymer of the present invention, depending upon the coating method and the coating amount, the hydrophilic and oil-repellent properties are exhibited thereby.

Regarding a coating method to afford the hydrophilic and oil-repellent properties, the surface-treated powder having the hydrophilic and oil-repellent properties is obtained by the surface treatment with a coating amount greater than that capable of obtaining the water-repellent and oil-repellent properties in one coating process. The specific fluorine-containing copolymer to be used in the present invention has high hydrogen bond force and film-forming force and enables easy treatment for the base material of the powder, when an optimum amount of the polyalkylene glycol is introduced into the molecule. However, if the coated amount for the powder particles at this time is relatively small, the water-repellent and oil-repellent properties are exhibited, whereas if it becomes larger, free polyalkylene glycol chains become excess, so that the surface-treated powder exhibits the hydrophilic property upon contact with water due to the mobility of the molecules depending upon the environment in which the powder particles are placed. In addition, although depending upon the kind of the powder, the hydrophilic and oil-repellent properties can be obtained by a method in which the treatment is performed, while pH of the powder is set to an acidic or alkaline side at the time of the surface treatment, a method in which the temperature at the time of the treatment is controlled, or a method in which after the powder is coated with a conventional fluorine-containing compound, it is coated with the specific fluorine-containing copolymer of the present invention.

Regarding one more coating method to obtain the hydrophilic and oil-repellent properties, such properties are obtained by coating at plural times. That is, such properties are obtained by further coating the surface-treated powder, to which the water-repellent and oil-repellent properties are afforded by coating, with a specific fluorine-containing copolymer, with the identical or different specific fluorine-containing copolymer. When fluoro chains are exposed on the outermost surfaces of first treated layers of particles of a surface-treated powder exhibiting the water-repellent and oil-repellent properties and those surfaces are further coated such that fluoro chains of a polymer in a second treated layer are opposed to those in the first treated layer, the polyalkylene glycol chains are exposed on the outermost surface of the particles, exhibiting the hydrophilic and oil-repellent properties. The hydrophilic and oil-repellent properties and the water-repellent and oil-repellent properties can be controlled by repeating such operations.

As an evaluation method for the water-repellent and oil-repellent properties and the hydrophilic and oil-repellent properties, there is a method for measuring a contact angle with water or squalane, and this method can be employed. According to this method, the water-repellent and oil-repellent properties of the surface-treated powder of the present invention are preferably such that a contact angle with water or squalane is not less than 80°. The hydrophilic and oil-repellent properties of the surface-treated powder according to the present invention is preferably such that the contact angle with water is not more than 20°, the contact angle with squalane is not less than 80°, more preferably the contact angle with water is not more than 10° and the contact angle with squalane is not less than 100°.

The contact angle of such a surface-treated powder with squalane is an angle which is formed between a drop of squalane, which is dropped on a surface of a molded powder obtained by packing the powder into a metal bowl and molding it under application of a pressure of 6MPa for 10 seconds, and the powder surface. The larger this angle, the larger is an effect of repelling that liquid drop, thus exhibiting the liquid-repelling property, whereas the smaller the angle, the more is liquid affinity property exhibited.

As another evaluation method for the hydrophilic property, after water is put into a beaker, a small amount of a powder is put thereinto and the resultant is stirred with a spatula 50 times at a rate of 2 times per one second, evaluation can be made by observing the states of the powder floating on the surface of water and the powder dispersed in a water layer. The surface-treated powder having the hydrophilic and oil-repellent properties according to the present invention is dispersed in the water layer without almost floating on the surface of water.

A cosmetic containing a powder which is coated with a compound containing at least a specific fluorine-containing copolymer of the present invention and possess the hydrophilic and oil-repellent properties exhibits more excellent affinity with other ingredients blended into a cosmetic formulation as compared with a cosmetic containing a powder treated with a conventional fluorine-containing compound, so that the former is more excellent in terms of usability, cosmetic finish, long-lasting property and quality stability in the cosmetic.

Since the surface-treated powder according to the present invention exhibits the hydrophilic and oil-repellent properties, the powder can be dispersed in aqueous ingredients such as a polyol and the like blended into the cosmetic formulation. After the dispersion liquid is applied to a skin and the aqueous ingredient is evaporated, the coated film of the surface-treated powder exhibits the oil-repellent property. This means that the conventional surface-treated powder exhibiting the water-repellent and oil-repellent properties exhibits poor solubility, emulsifiability and dispersability for the oil ingredient and the aqueous ingredient of the cosmetic and gives poor adhesion to the skin, and the power of the present invention can improve such properties. The present invention can solve the problem that variations of formulations into which is blended the powder treated with the conventional fluorine-containing compound are limited. One example is that the surface-treated powder is designed such that the entire formulation exhibits the water-repellent and oil-repellent properties by blending the surface-treated powder exhibiting such hydrophilic and oil-repellent properties into an aqueous component and blending an oil-repellent ingredient into an oily component. Consequently, it is possible to make formulations having functions beyond conventional ones and novel formulations different from those in the prior art.

One or more kinds of the surface-treated powders obtained in the present invention can be blended into a cosmetic.

### (Cosmetics of the present invention)

The cosmetics of the present invention are cosmetics in which the above-mentioned surface-treated powders are contained. That is, the surface-treated powder can be prepared as mentioned above.

In the present invention, the cosmetic formulations are free from particular difficulty, and a target cosmetic can be obtained by a conventionally used technique, particularly based on a technique (for example, emulsification or the like) for incorporating the surface-treated powder into the cosmetic.

When the surface-treated powder of the present invention is to be blended into the cosmetic, although the blended amount is different depending upon the kind and the formulation of the cosmetic, it is preferably 0.1 to 100 weight% in the entire cosmetic composition. Further, the surface-treated powder of the present invention can form a dispersion with an aqueous component, and its blended amount is not particularly limited in a case where the powder is blended into the cosmetic.

As other ingredients to be added into the cosmetics of the present invention, an oily ingredient, an aqueous ingredient and a surface-active agent are recited, for example.

As the oily ingredients, recitation is made of, for example, oils and fats, such as safflower oil, soybean oil, evening primrose oil, grape seed oil, rose hips oil, candlenut oil, almond oil, sesame oil, wheat germ oil, corn oil, cottonseed oil, avocado oil, olive oil, camellia oil, persic oil, castor oil, peanut oil, hazelnut oil, macadamia nut oil, meadow-foam oil, cacao butter, shea butter, Japanese wax, palm oil, palm oil, palm kernel oil, beef tallow, horse fat, mink oil, milk fat, egg yolk oil, turtle oil, etc.; waxes such as honey wax, whale wax, lanolin, carnauba wax, candelilla Wax, jojoba oil, etc.; hydrocarbons such as hydrocarbons such as liquid paraffin, liquid isoparaffin, squalane, squalene, petrolatum, paraffin, seresin, microcrystalline wax, alpha-olefin oligomer, etc.; fatty acids such as lauric acid, myristic acid, palmitic acid, stearic acid, behenic acid, oleic acid, linoleic acid, undecylenic acid, hydroxy stearic acid, lanolin fatty acid, etc.: higher alcohols such as myristyl alcohol, cetyl alcohol, cetostearyl alcohol, stearyl alcohol, aralkyl alcohol, behenyl alcohol, oleyl alcohol, jojoba alcohol, batyl alcohol, cholesterol, phytosterol, lanolin alcohol, isostearyl alcohol, etc.; lanosterols such as cholesterol, sitosterol, phytosterol, etc.; sterols such as lanosterol, etc.; esters such as ethyl oleate, isopropyl myristate, cetyl octanoate, diisostearyl malate, glyceryl tricaprylate, etc.: silicones such as dimethyl polysiloxane, methylphenyl polysiloxane, alkyl-modified silicone, cyclic silicone pentamer, cyclic silicone hexamer, methyl trimethicon, caprylyl methicon, diethicon, trimechikon, alkylmethicon, tocophenyl trisiloxane, fluorocarbon silicone, etc.; fluorine-based oily agents such as perfluoropolyether oil, perfluorocarbon, hydrofluoroether, etc.; organic solvents such as ethyl acetate, butyl acetate, toluene, etc..

As the aqueous ingredients, recitation is made of lower alcohols such as ethanol, isopropanol, etc.: polyvalent alcohols such as 1,3-butylene glycol, propylene glycol, polyethylene glycol, glycerin, diglycerin, polyglycerin, trehalose, erythritol, mannitol, xylitol, sorbitol, maltose, etc.; plant-based water-soluble polymers such as gum arabic, tragacanth, galactan, carob gum, guar gum, karaya gum, carrageenan, pectine, agar, quince seed (quince), starch (rice, corn, potato, wheat), dextrin, dextoran, argecolloid, trant gum, locust bean gum, etc.; microorganism-based water-soluble polymers such as xanthane gum, dextran, succinoglucan, pullulan, etc.; animal-based water-soluble polymers such as collagen, casein, albumin, gelatin, etc.; starch-based water-soluble polymers such as carboxymethyl starch, methylhydroxypropyl starch, etc.; cellulose-based water soluble polymers such as methyl cellulose, ethyl cellulose, methylhydroxypropyl cellulose, carboxymethyl cellulose, hydroxymethyl cellulose, hydroxypropyl cellulose, nitrocellulose, cellulose sodium sulfate, carboxymethyl cellulose sodium, crystalline cellulose and cellulose powder; alginic acid-based water-soluble polymers such as sodium alginate, alginic acid propylene glycol ester, etc.; vinyl-based water-soluble polymers such as polyvinylmethyl ether, carboxyvinyl polymer, etc.; polyoxyethylene-based water-soluble polymer, polyoxyethylene polyoxypropylene copolymer-based water-soluble polymer, acryl-based water soluble polymers such as polyacrylic acid sodium, polyethyl acrylate, polyacrylamide, etc.; other synthetic water-soluble polymers such as polyethylene imine, cation polymer, etc.; and inorganic water-soluble polymers such as bentonite, magnesium aluminum silicate, montmorillonite, beidellite, nontronite, saponite, hectorite, silicic anhydride, etc.. In addition, these water-soluble polymers include film-forming agents such as polyvinyl alcohol, polyvinyl pyrolidone, etc.

As the surface active agent, recitation is made of fatty acid soaps such as fatty acid soaps such as sodium stearate, triethanolamine palmitate, etc.; alkyl ether carboxylic acids and salts thereof, condensation salts between amino acids and fatty acids, alkane sulfonic acid salts, alkene sulfonic acid salts, sulfonic acid salts of fatty acid esters, sulfonic acid salts of fatty acid amides, sulfonic acid salts of formalin condensation series, sulfuric acid ester salts such as alkyl sulfuric acid ester salts, secondary higher alcohol sulfuric acid ester salts, alkyl and allyl ether sulfuric acid ester salts, sulfuric acid ester salts of fatty acid esters, sulfuric acid ester salts of fatty acid alkylol amides, tote oil, etc. anionic surface active agents such as alkyl phosphoric acid salts, ether phosphoric acid salts, alkylallyl ether phosphoric acid salts, amido phosphoric acid salts, N-acylamino acid-based active agents, etc.; amine salts such as alkyl amine salts, polyamine and amino alcohol fatty acid derivatives, etc.; cationic surface active agents such as alkyl quaternary ammonium salts, aromatic quaternary ammonium salts, pyridium salts, imidazolium salts, etc.; nonionic surface active agents such as sorbitan fatty acid esters, glycerin fatty acid esters, polyglycerin fatty acid esters, propylene glycol fatty acid esters, polyethylene glycol fatty acid esters, sucrose fatty acid esters, polyoxyethylene alkyl ethers, polyoxypropylene alkyl ethers, polyoxyethylene alkyl phenyl ethers, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene cater oil, polyoxyethylene hydrogenated cater oil, polyoxyethylene phytostanol ethers, polyoxyethylene phytosterol ethers, polyoxyethylene cholestanol ethers, polyoxyethylene cholesteryl ethers, polyoxyalkylene-modified organopolysiloxane, polyoxyalkylene-alkyl co-modified organopolysiloxane, polyglycerin-modified organopolysiloxane, alkanol amides, saccharo ethers, saccharo amides, etc.; and ampholytic surface active agents such as betaine, amino carboxylic acid salts, imidazoline derivatives, etc.

So long as aesthetics, cosmetic finish, long-lasting property, excellent affinity with other ingredients to be blended into the cosmetic formulations, in addition quality stability, safety to living bodies, safety to the environment or the like is not lost, the cosmetic of the present invention can be appropriately blended with a pigment dispersant, an oily agent, a surface active agent, a UV absorber, antiseptic, antioxidant, film formers, moisturizing agents, thickeners, dyes, pigments, various medicines (vitamins, astaxanthin, alpha-lipoic acid, coenzyme Q10, etc.), perfumes, etc.

No limitation is particularly imposed upon formulations of the cosmetics according to the present invention. As the formulations of the cosmetics, for example, conventionally publicly known formulations such as an emulsion form, a creamy form, a solid form, a paste form, a gel form, a powdery form, a multilayer from, a moose form, a spray form, etc. can be selected. Specifically, recitation can be made of, as makeup cosmetic, makeup base, powder foundation, liquid foundation, oily foundation, stick foundation, pressed powder, face powder, white powder, lip stick, lip stick overcoat, lip gloss, concealer, blusher, eye shadow, eyebrow, eyeliner, mascara, aqueous nail enamel, oily nail enamel, emulsion type nail enamel, enamel top coat, enamel base coat and the like; as skincare cosmetics, emollient cream, cold cream, whitening cream, emulsion, lotion, beauty essence, pack, carmine lotion, liquid face wash, face wash foam, face wash cream, face wash powder, makeup cleansing, body gloss, UV control cosmetics, lotion and the like such as sunscreen, sunburn cream and the like; as hair cosmetics, hair gloss, hair cream, hair shampoo, hair conditioner, hair color, hair brushing agent and the like; as antiperspirant cosmetics, cream, lotion, powder, spray, roll-on type deodorant product and the like; as others, emulsion, soap, bath agent, perfume and the like.

### (Uses other than cosmetics)

The above-mentioned surface-treated powders (the surface-treated powders according to the present invention) can be applied to various fields of not only cosmetics but also inks, paints, resin master batches, powder fillers to be blended into papers and the like, powdery fillers, ceramic materials, magnetic materials, rare earths, optical materials, electroconductive materials, piezoelectric materials and the like.
Particularly, when the powders usable in the cosmetics are used in other fields, the surface-treated powders explained for the cosmetic purpose can be also employed in these other fields.

In the following, the present invention will be explained in detail by taking Examples and Comparative Examples, but the invention is not limited to those Examples.

### (Examples)

### (Producing Examples 1-1 to 1-3)

Producing Examples of the fluorine-containing copolymer compounds to be used in the present invention were shown below.

### (Producing Example 1-1)

Into a 100ml four-necked flask equipped with a reflex cooling tube, a nitrogen introduction tube, a thermometer and a stirrer were charged 18.6g of a fluorine-containing monomer CH2 = CHC(=O)O-CH2CH2C6F13 (hereinafter, referred to as C6SFA(a)), 11.4g of polyethylene glycol acrylate CH2=CHC(=O)O-(CH2CH2O)n-H (BLEMMER AE 90, manufactured by NOF Corporation, the average value of n is 2, hereinafter referred to as AE90(b)), 0.3g of 2-mercaptoethanol and 45g of methylethyl ketone (hereinafter referred to as MEK), followed by bubbling with nitrogen for 30 minutes. The internal temperature was raised to 50 to 65° C under nitrogen stream, and 0.4g of perbutyl PV (hereinafter referred to as PV) was added to perform a reaction at 60 to 65°C for 6 hours. MEK was distilled off from the resulting solution at about 70°C under a reduced pressure condition, thereby obtaining a residue of a light yellow polymer. Then, after 122.4g of water was added and the internal temperature was kept at about 80°C for not less than 1 hour, a water dispersion having about 20 wt% of a solid concentration was prepared by cooling.

### (Producing Example 1-2)

A similar polymerization reaction as in Producing Example 1-1 was carried out provided that AE90(b) in Producing Example 1-1 was replaced by polyethylene glycol methacrylate CH2=C(CH3)C(=O)O-(CH2CH2O)n-H (BLEMMER PE350, manufactured by NOF CORPORATION, the average value of n is 8, hereinafter referred to as PE350(b)), thereby preparing a water dispersion having about 20 wt% of a solid concentration.

### (Producing Example 1-3)

Into a 100ml four-necked flask equipped with a reflex cooling tube, a nitrogen introduction tube, a thermometer and a stirrer were charged 18.6g of C6SFA(a), 5.7g of AE 90(b), 5.7g of polyethylene glycol acrylate CH2=CHC(=O)O-(CH2CH2O)n-H (BLEMMER AE200, manufactured by NOF CORPORATION, the average value of n is 4.5, hereinafter referred to as AE200(b)), 0.3g of 2-mercaptoethanol and 45g of MEK, and a similar polymerization reaction as in Producing Example 1-1 were carried out, thereby preparing a water dispersion having about 20 wt% of a solid concentration.

### (Producing Example 1-4)

Into a 100ml four-necked flask equipped with a reflex cooling tube, a nitrogen introduction tube, a thermometer and a stirrer were charged 18.6g of C6SFA(a), 10.5g of AE350(b), 0.9g of polyethylene glycol diacrylate CH2=CHC(=O)O - (CH2CH2O)n - C(=O)CH=CH2 (BLEMMER ADE300, manufactured by NOF CORPORATION, the average value of n is 7), 0.3g of dodecanethiol and 45g of isopropanol, and a similar polymerization reaction as in Example 1 was carried out, thereby preparing a water dispersion having about 20 wt% of a solid concentration.

### (Table 1-1)

**Table 1-1**

| Compound | Structure | Remarks |
|---|---|---|
| (a1) | Fluorine-containing copolymer in Production Example 1-1 | To be used in the present invention |
| (b1) | Fluorine-containing copolymer in Production Example 1-2 | To be used in the present invention |
| (c1) | Fluorine-containing copolymer in Production Example 1-3 | To be used in the present invention |
| (d1) | Fluorine-containing copolymer (17FA/HEMA) disclosed in Production Example 1 of JP-A 2000-290640 | Conventional treating agent (Comparative Example) |
| (e1) | [CF3(CF3)7-15CH2CH2O]2P(O)OH | Conventional treating agent (Comparative Example) |

### (Examples 1-1 to 1-14 and Comparative Examples 1-1 to 1-14)

(a1) to (c1) are fluorine-containing copolymers to be used in the present invention, and a copolymer shown in (d1) and a perfluoroalkyl phosphoric acid ester shown in (e1) are conventional compounds (Comparative Example). A powder surface-treated with each of these compounds was produced, and physical properties of these surface-treated powders were evaluated. In the following, Producing Examples of the surface-treated powders and evaluation items and methods were shown.

### (Example 1-1 and Comparative Example 1-1)

Into a high-speed mixer was charged 100g of sericite FSE (Sanshin Mining Ind. Co., Ltd.), and 3g as a solid content of each of fluorine-containing compounds shown in Table 1, 50g of a mixed solution of IPA (isopropyl alcohol) and water (50:50wt%) were added, followed by kneading for 30 minutes. After the kneaded matter was dried at 80°C for 3 hours, it was dried at 110°C for 10 hours. A sericite treated with each of the fluorine-containing compound was obtained by pulverizing with an atomizer.

### (Example 1-2 to Comparative Example 1-2)

The sericite used in Example 1-1 and Comparative Example 1-1 was replaced by Mica Y-2300 (Yamaguchi Mica Co., Ltd.), and surface treatment was carried out similarly to Example 1-1 and Comparative Example 1-1, thereby obtaining each of micas treated with the fluorine-containing compounds.

### (Examples 1 - 3 and Comparative Examples 1 - 3)

Talc surface-treated with each of the fluorine-containing compounds and a perfluoroalkyl phosphoric acid ester in complex was obtained by surface treatment in the same manner as in Example 1-1 and Comparative Example 1-1, provided that the sericite used in Example 1-1 and Comparative Example 1-1 was replaced by Talc JA-46R (Asada Milling, Co., Ltd.) and the fluorine-containing compound/the perfluoroalkyl phosphoric acid ester [CF3(CF3)5CH2CH2O]2P(O)OH shown in Table 1-1 were in 5g/2g, respectively, as a solid content.

### (Example 1-4 and Comparative Example 1-4)

Titanium CR-50 (Ishihara Sangyo Kaisha, Ltd.), 100g, was added with each of the fluorine-containing compounds diluted in 10ml of IPA at a solid content of 5g, which was mixed with a mixer for 15 minutes, and steam overheated at 250°C was introduced thereinto. At a point of time when the interior of the mixer reached 200°C, stirring was stopped, thereby obtaining each of fluorine-containing compound-treated powders.

### (Example 1-5 to 1-7 and Comparative Examples 1-5 to 1-7)

Each of fluorine-containing compounds was obtained by surface treatment in the same manner as in Example 1-1 and Comparative Example 1-1, provided that the sericite in Example 1-1 and Comparative Example 1-1 was replaced by each of Yellow LL-100P (Titan Kogyo, Ltd.), Red R-516PS (Titan Kogyo, Ltd.) and Black BL-100P (Titan Kogyo, Ltd.), respectively.

### (Example 1-8 and Comparative Example 1-8)

Fine particles of titanium oxide (MT-100TV. TAYCA Corporation), 100g, was put into 1500ml of deionized water, and each of fluorine-containing compounds which was diluted at a solid content of 5g with 500ml IPA was added thereinto. The mixture was dispersed under circulation for 15 minutes with a sand grinder (DYNO-Mill: 1.4L Zirconia Vessel & Blade, 0.5mm in diameter zirconia beads at a packed rate 85%). The dispersion liquid was heated up to 80°C under stirring, and a 10% HCl aqueous solution wad added dropwise to adjust pH at 4.5. After the resultant was dewatered by centrifugal separation, the residue was dried at 120°C for 16 hours, and pulverized with a JET atomizer, thereby obtaining finely particulate titanium oxide treated with each of the fluorine-containing compounds.

### (Example 1-9 and Comparative Example 1-9)

Finely particulate titanium oxide surface-treated with each of fluorine-containing compounds was obtained by the producing method as shown in Example 1-1 and Comparative Example 1-1 provided that the solid content of each of the fluorine-containing compounds in Example 1-8 and Comparative Example 1-8 was changed to 8g and the solvent was an IPA solution only.

### (Example 1-10 and Comparative Example 1-10)

Surface-treated finely particulate titanium oxide double coated with each of the fluorine-containing compounds was obtained by the surface treatment of each of the fluorine-containing compound-treated powders obtained in Example 1-8 and Comparative Example 1-8 in the same manner as in Example 1-1 and Comparative Example 1-1 provided that the solvent used in the method of Example 1-1 and Comparative Example 1-1 was replaced by HFE and the solid content to the powder of each of the fluorine-containing compound shown in Table 1-1 was set to 1%.

### (Example 1-11 and Comparative Example 1-11)

HFE (hydrofluoroether), 15g and 50g of IPA were added to 100g of finely particulate zinc oxide (MZ-300: TAYCA Corporation), 5g of each of the fluorine-containing compounds shown in Table 1-1 and 3g of a straight-chain dimethyl polysiloxane having a triethoxy group at one end with a polymerization degree of 15, which was kneaded with a kneader for 30 minutes. Further, after 10g of deionized water was added and the mixture was kneaded for 30 minutes, it was dried at 105°C for 16 hours, and then finely particulate zinc oxide treated in combination with the fluorine-containing compound and the silicone was obtained by pulverizing with a JET atomizer.

### (Example 1-12 and Comparative Example 1-12)

A pearl pigment (Flamenco Gold: Angel Heart Co., Ltd.), 100g, was added to 1,000ml of deionized water, and 1.5 g of disodium N-stearoyl glutamate (Ajinomoto Co., Inc.: Amisoft HS-21P) was added thereto, followed by dissolving and dispersing. Further, 3g of each of the fluorine-containing compounds shown in Table 1-1 was added thereto, which was heated to 80°C and kneaded with a kneader for 30 minutes. The kneaded matter was dried at 130°C for 8 hours, thereby obtaining the pearl pigment treated in combination with each of the fluorine-containing compounds and the acylated amino acid.

### (Example 1-13 and Comparative Example 1-13)

A mixed liquid of IPA/HFE = 15g/10g was added to 100g of Amihope LL (lauroyl lysin: Ajinomoto Co., Inc.), 3g of each of the fluorine-containing treating agents shown in Table 1-1 was added thereto, and the surface treatment was carried out by the same method as in Example 1-12 and Comparative Example 1-12, thereby obtaining the Amihope LL treated with each of the fluorine-containing compounds.

### (Example 1-14 and Comparative Example 1-14)

Red No. 202 (Kishi Kasei Co., Ltd.), 100g, was put into a mixed solution of IPA/HFE/deionized water = 40g/15g/100g, which was dispersed with a ultrasonic homogenizer (Japan Siber Hegner & Co.), while being stirred with a propeller stirrer. After 5g of each of the fluorine-containing treating agents shown in Table 1-1 and 5g of IPA were dropwise added thereto and the mixture was aged, the solvent was distilled off by heating under vacuum, and the resultant was dried and pulverized at 105°C for 16 hours, thereby Red No. 202 treated with each of the fluorine-containing compounds was obtained by milling.

Examples 1-1 to 1-14 and Comparative Examples 1-1 to 1-14 were shown in list in Table 1-2.

### (Table 1-2)

**Table 1-2: A list of Examples and Comparative Examples**

| Powder to be treated | Compound (a1) | Compound (b1) | Compound (c1) | Compound (d1) | Compound (e1) |
|---|---|---|---|---|---|
| Sericite FSE | Example 1-1 | Example 1-1 | Example 1-1 | Comparative Example 1-1 | Comparative Example 1-1 |
| Mica Y-2300 | Example 1-2 | Example 1-2 | Example 1-2 | Comparative Example 1-2 | Comparative Example 1-2 |
| Talc JA-46R | Example 1-3 | Example 1-3 | Example 1-3 | Comparative Example 1-3 | Comparative Example 1-3 |
| Titanium CR-50 | Example 1-4 | Example 1-4 | Example 1-4 | Comparative Example 1-4 | Comparative Example 1-4 |
| Yellow-LL-100P | Example 1-5 | Example 1-5 | Example 1-5 | Comparative Example 1-5 | Comparative Example 1-5 |
| Red R-516PS | Example 1-6 | Example 1-6 | Example 1-6 | Comparative Example 1-6 | Comparative Example 1-6 |
| Black BL-100P | Example 1-7 | Example 1-7 | Example 1-7 | Comparative Example 1-7 | Comparative Example 1-7 |
| Finely particulate titanium oxide | Example 1-8 | Example 1-8 | Example 1-8 | Comparative Example 1-8 | Comparative Example 1-8 |
| Finely particulate titanium oxide | Example 1-9 | Example 1-9 | Example 1-9 | Comparative Example 1-9 | Comparative Example 1-9 |
| Finely particulate titanium oxide | Example 1-10 | Example 1-10 | Example 1-10 | Comparative Example 1-10 | Comparative Example 1-10 |
| Finely particulate zinc oxide | Example 1-11 | Example 1-11 | Example 1-11 | Comparative Example 1-11 | Comparative Example 1-11 |
| Pearl pigment | Example 1-12 | Example 1-12 | Example 1-12 | Comparative Example 1-12 | Comparative Example 1-12 |
| Amihope LL | Example 1-13 | Example 1-13 | Example 1-13 | Comparative Example 1-13 | Comparative Example 1-13 |
| Red No. 202 | Example 1-14 | Example 1-14 | Example 1-14 | Comparative Example 1-14 | Comparative Example 1-14 |

### (Tests on contact angles of water and squalane)

A double-faced tape is attached to a slide glass, and each of the surface-treated powders is coated onto the other face of the tape with a cosmetic puff until adhesivity of the tape face loses adhesion. A liquid was dropwise fallen on the powder-coated surface (a dropped amount 10µl), and 20 second later, an angle (contact angle) between the liquid drop and the powder-coated face was measured with a contact angle meter (CA-D) manufactured by Kyowa Interface Science Co., Ltd. (n times was set to 5).

### (Tests on usability and adhesion)

Usability and adhesion of each surface-treated powders for the identical powder were evaluated by 15 panelists capable of conducting organoleptic examinations of the surface-treated powders. Evaluations were performed by rubbing with balls of fingers or coating the powders on backs of hands and brachial regions. Evaluations were performed by absolute evaluations with each panelist, and their marks were defined as follows. A: Very good, B: good, C: ordinary, D: bad.

### (Evaluation methods for water-repellency and hydrophilicity)

About 50cc of purified water is put into a 100cc glass beaker. About 0.1g of the treated powder is put on a water surface, which is stirred by a spatula at a rate of about 2 times per one second. Turbidity degrees of the water phase after stirring 50 times, 100 times and 150 times were evaluated according to 5-mark scale.

### (Evaluation standard)

5 - No turbidity is observed in the water phase (strong water repellency)
4 - Slight turbidity is observed in the water phase
3 - Clear turbidity is observed in the water
2 - Most powder is moved into the water phase
1 - All powder is moved into the water phase (Strong hydrophilicity)

### (Evaluation results)

Evaluations were shown in lists in Table 1-3 to Table 1-5 for the surface-treated powders in Examples 1-1 to 1-14 and Comparative Examples 1-1 to 1-14.

### (Table 1-3)

**Table 1-3: Test results on contact angles of water/squalane**

| Powder to be treated | Compound (a1) | Compound (b1) | Compound (c1) | Compound (d1) | Compound (e1) |
|---|---|---|---|---|---|
| Sericite FSE | 135°/130° | 133°/133° | 140°/130° | 124°/120° | 141°/135° |
| Mica Y-2300 | 138°/130° | 132°/130° | 137°/133° | 126°/129° | 145°/137° |
| Talc JA-46R | 134°/127° | 130°/125° | 136°/130° | 126°/129° | 147°/130° |
| Titanium CR-50 | 130°/130° | 132°/133° | 135°/130° | 136°/139° | 146°/139° |
| Yellow-LL-100P | 137°/130° | 133°/131° | 138°/132° | 136°/120° | 145°/135° |
| Red R-516PS | 136°/130° | 135°/123° | 135°/128° | 136°/130° | 145°/130° |
| Black BL-100P | 130°/132° | 132°/134° | 133°/130° | 136°/130° | 142°/131° |
| Finely particulate titanium oxide | 132°/130° | 132°/133° | 137°/124° | 136°/131° | 145°/130° |
| Finely particulate titanium oxide | 8°/130° | 10°/132° | 10°/133° | 136°/130° | 145°/140° |
| Finely particulate titanium oxide | 9°/130° | 12°/133° | 8°/130° | 136°/138° | 143°/141° |
| Finely particulate zinc oxide | 130°/131° | 132°/130° | 130°/132° | 136°/130° | 145°/130° |
| Pearl pigment | 133°/130° | 132°/133° | 139°/131° | 136°/137° | 144°/130° |
| Amihope LL | 10°/125° | 13°/135° | 10°/128° | 136°/133° | 145°/131° |
| Red No. 202 | 135°/130° | 132°/133° | 140°/133° | 136°/131° | 144°/136° |

### (Table 1-4)

**Table 1-4: Test results on usability/adhesion**

| Powder to be treated | Compound (a1) | Compound (b1) | Compound (c1) | Compound (d1) | Compound (e1) |
|---|---|---|---|---|---|
| Sericite FSE | B/A | B/A | B/A | C/C | D/D |
| Mica Y-2300 | B/A | B/A | B/A | C/C | D/D |
| Talc JA-46R | A/A | A/A | A/A | B/D | C/D |
| Titanium CR-50 | B/A | B/A | B/A | C/D | D/D |
| Yellow-LL-100P | B/A | B/A | B/A | C/D | D/D |
| Red R-516PS | B/A | B/A | B/A | C/D | D/D |
| Black BL-100P | B/A | B/A | B/A | C/D | D/D |
| Finely particulate titanium oxide | B/A | B/A | B/A | B/C | D/D |
| Finely particulate titanium oxide | A/A | A/A | A/A | B/D | C/D |
| Finely particulate titanium oxide | A/A | A/A | A/A | B/D | C/D |
| Finely particulate zinc oxide | B/A | B/A | B/A | C/C | D/D |
| Pearl pigment | A/A | A/A | A/A | C/C | D/D |
| Amihope LL | A/A | A/A | A/A | C/C | D/D |
| Red No. 202 | B/A | B/A | B/A | C/C | D/D |

### (Table 1-5)

**Table 1-5: Test results on water repellency and hydrophilicity**

| Powder to be treated | Compound (a1) | Compound (b1) | Compound (c1) | Compound (d1) | Compound (e1) |
|---|---|---|---|---|---|
| Sericite FSE | 5 | 5 | 5 | 5 | 5 |
| Mica Y-2300 | 4 | 4 | 4 | 5 | 5 |
| Talc JA-46R | 5 | 5 | 5 | 5 | 5 |
| Titanium CR-50 | 5 | 5 | 5 | 5 | 5 |
| Yellow-LL-100P | 5 | 5 | 5 | 5 | 5 |
| Red R-516PS | 5 | 5 | 5 | 5 | 5 |
| Black BL-100P | 5 | 5 | 5 | 5 | 5 |
| Finely particulate titanium oxide | 5 | 5 | 5 | 5 | 5 |
| Finely particulate titanium oxide | 1 | 1 | 1 | 5 | 5 |
| Finely particulate titanium oxide | 1 | 1 | 1 | 5 | 5 |
| Finely particulate zinc oxide | 5 | 5 | 5 | 5 | 5 |
| Pearl pigment | 5 | 5 | 5 | 5 | 5 |
| Amihope LL | 1 | 1 | 1 | 5 | 5 |
| Red No. 202 | 4 | 4 | 4 | 5 | 5 |

The surface-treated powders obtained in Examples 1-9 and 1-10 and Example 1-13 had the hydrophilic and oil-repellent properties. Each of the surface-treated powders obtained in the respective Examples exhibits high usability and adhesion.

### (Examples 1-15 to 1-32 and Comparative Examples 1-15 to 1-32)

Next, Examples of cosmetics into which the surface-treated powders of the present invention were blended will be explained. Various cosmetics were produced based on the respective compositions in the following Tables by the following producing methods.

Meanwhile, 20 special panelists were prepared for respective evaluation items on usability, cosmetic finish and long-lasting property regarding cosmetics obtained in the respective Examples and Comparative Examples, and they used them for one day. They made evaluations according to evaluation standards shown in Table 1-6 to Table 1-8, and evaluation marks were obtained by dividing the total marks of the entire panelists with 20.

### (Table 1-6)

**Table 1-6: Evaluation of usability**

| Standard | Mark |
|---|---|
| Very good usability | A |
| Good usability | B |
| Ordinary usability | C |
| Bad usability | D |
| Very bad usability | E |

### (Table 1-7)

**Table 1-7: Evaluation of cosmetic finish**

| Standard | Mark |
|---|---|
| High effect felt | A |
| Effect felt | B |
| Effect somewhat felt | C |
| Effect merely slightly felt | D |
| No effect felt | E |

Regarding the finish items, finish uniformity, covering power, natural glossy feeling and cosmetic film uniformity were evaluated for a makeup and a skincare cosmetic, absence of stickiness and absence of oiliness were evaluated for an antiperspirant cosmetic, and glossy feeling, silky feeling, smoothness and combing easiness were evaluated for hair cosmetics.

### (Table 1-8)

**Table 1-8: Evaluation of long-lasting property**

| Standard | Mark |
|---|---|
| High effect felt | A |
| Effect felt | B |
| Effect somewhat felt | C |
| Effect merely slightly felt | D |
| No effect felt | E |

Three items of color dullness prevention, secondary attachment (color transfer) prevention and shinning prevention were taken for items of the long-lasting property. Meanwhile, peeling difficulty was evaluated for a nail manicure.

### (Example 1-15 and Comparative Example 1-15) Production of powder foundation

Powder foundations having a composition shown in Table 1-9 were produced by the following method. Evaluation results were shown in Table 1-10.

### (Table 1-9)

**Table 1-9**

| Ingredients | Parts by weight |
|---|---|
| (1) Sericite (Example 1-1 & Comparative Example 1-1) | 35.0 |
| (2) Talc (Example 1-3 & Comparative Example 1-3) | balance |
| (3) titanium oxide (Example 1-4 & Comparative Example 1-4) | 8.5 |
| (4) Yellow iron oxide (Example 1-5 & Comparative Example 1-5) | 3.5 |
| (5)Red iron oxide (Example 1-6 & Comparative Example 1-6) | 1.8 |
| (6) Black iron oxide (Example 1-7 & Comparative Example 1-7) | 0.2 |
| (7) Octyldodecyl myristate | 4.0 |
| (8) Squalane | 3.5 |
| (9) Methylphenyl polysiloxane | 1.5 |
| (10) Antiseptic | approp. amount |
| (11) Perfume | approp. amount |

### (Producing method)

The above ingredients (1) to (6) were mixed and pulverized through a grinding mill. The resultant was moved to a high speed blender, and ingredients (7) to (11) were mixed and homogenized under heating, and added thereinto, followed by further mixing and homogenizing. After the resultant was passed through the mill and screened to adjust particle sizes, the powder was compression molded in an aluminum bowl under a surface press pressure of 10 MPa, thereby producing a 2-way powder foundation.

### (Table 1-10)

**Table 1-10**

| Evaluation item | Compound (a1) | Compound (b1) | Compound (c1) | Compound (d1) | Compound (e1) |
|---|---|---|---|---|---|
| Usability | B | B | A | D | D |
| Uniform finish | B | B | A | C | D |
| long-lasting property | A | A | A | B | B |

### (Example 1-16 and Comparative Example 1-16) Production of emulsion type foundations

W/O type liquid foundations having a composition shown in Table 1-11 were produced by the following method. Evaluation results were shown in Table 1-12.

### (Table 1-11)

**Table 1-11**

| Ingredients | Parts by weight |
|---|---|
| (1) Decamethylcyclopentasiloxane | 20.0 |
| (2) Vaseline | 0.5 |
| (3) Methylphenyl polysiloxane | 2.3 |
| (4) Squalane | 4.2 |
| (5) Isotridecyl isonanoate | 4.5 |
| (6) Dimethylpolysiloxane polyoxyalkylene polymer (HLB=4.5) | 3.0 |
| (7) Red iron oxide (Example 1-6 and Comparative Example 1-6) | 1.3 |
| (8) Yellow iron oxide (Example 1-5 and Comparative Example 1-5) | 2.4 |
| (9) Black iron oxide (Example 1-7 and Comparative Example 1-7) | 0.1 |
| (10) Titanium oxide (Example 1-4 and Comparative Example 1-4) | 8.0 |
| (11) Talc (Example 1-3 and Comparative Example 1-3) | 2.5 |
| (12) Ethanol | 5.0 |
| (13) 1,3-Butylene glycol | 5.0 |
| (14) Sodium chloride | 2.0 |
| (15) Purified water | balance |
| (16) Amihope LL (Example 1-13 and Comparative Example 1-13) | 5.0 |
| (17) Antiseptic | approp.amount |
| (18) Perfume | approp.amount |

### (Producing method)

The above ingredients (7) to (11) were preliminarily mixed and pulverized. The preliminarily pulverized mixture of the ingredients (7) to (11) was added into an oily phase in which ingredients (1) to (6) were homogeneously dissolved and mixed at 70 °C, and the resultant was homogeneously dispersed with a homodisperser. A water phase in which ingredients (12) to (17) were homogeneously mixed and dissolved at 70°C was gradually added into the above oily phase, which was homogeneously dispersed with the homo mixer. Then, the mixture was cooled, and an ingredient (18) was added thereto, and emulsion particles were adjusted, thereby producing a liquid foundation.

### (Table 1-12)

**Table 1-12**

| Evaluation item | Compound (a1) | Compound (b1) | Compound (c1) | Compound (d1) | Compound (e1) |
|---|---|---|---|---|---|
| Usability | A | B | A | D | D |
| Uniform finish | B | A | A | E | D |
| long-lasting property | A | A | A | D | D |
| Quality Stability*1 | good | good | good | bad | bad |

| | | | | | |
|---|---|---|---|---|---|
| *1 Regarding the quality stability, the emulsion was left in a thermostatic bath at 50°C for one week, and viscosity, separation and precipitation were observed. One with no outer appearance change was taken as good. Cosmetics into which the treated powders of Compounds (d) and (e) in Comparative Examples were blended caused separation the next day, and had bad quality stability. | | | | | |

### (Example 1-17 and Comparative Example 1-17) Production of eye shadows

Eye shadows having a composition shown in Table 1-13 were produced by the following method. Evaluation results were shown in Table 1-14.

### (Table 1-13)

**Table 1-13**

| Ingredients | Parts by weight |
|---|---|
| 1. Decamethylcyclopentasiloxane | 15.0 |
| 2. Dimethylphenyl polysiloxane (6cs) | 10.0 |
| 3. Polyether-modified silicone *2 | 2.0 |
| 4. PEG (1) lauryl ether | 0.5 |
| 5. Titanium oxide (Example 1-4 and Comparative Example 1-4) | 2.5 |
| 6. Red iron oxide (Example 1-6 and Comparative Example 1-6) | 3.5 |
| 7. Pearl pigment (Example 1-12 and Comparative Example 1-12) | 6.0 |
| 8. Sodium chloride | 2.0 |
| 9. Propylene glycol | 8.0 |
| 10. Antiseptic | approp.amount |
| 11. Perfume | approp.amount |
| 12. Purified water | balance |

| | |
|---|---|
| *2 KF6026: manufactured by Shin-Etsu Chemical Co., Ltd. | |

### (Producing method)

(1) Ingredients 1 to 4 are mixed, and ingredients 5 to 7 are added thereto, followed by homogeneously dispersing.
(2) Ingredients 8 to 10 and an ingredient 12 are dissolved homogeneously.
(3) The mixture obtained in (1) was emulsified by gradually adding the mixture obtained in (2) thereby under stirring, and an eyen shadow was obtained by adding an ingredient 11 thereto.

### (Table 1-14)

**Table 1-14**

| Evaluation item | Compound (a1) | Compound (b1) | Compound (c1) | Compound (d1) | Compound (e1) |
|---|---|---|---|---|---|
| Usability | A | B | B | D | E |
| Uniform finish | B | A | A | D | D |
| Long-lasting property | A | A | A | C | B |

### (Example 1-18 and Comparative Example 1-18) Production of oily solid foundations

Oily solid foundations having a composition shown in Table 1-15 were produced by the following method. Evaluation results were shown in Table 1-16.

### (Table 1-15)

**Table 1-15**

| Ingredients | Parts by weight |
|---|---|
| (1) Isopropyl palmitate | 20.0 |
| (2) Cetanol | 7.0 |
| (3) Squalane | 15.5 |
| (4) Polyglyceryl triisostearate | 5.0 |
| (5) Volatile fluid isoparaffin | 10.0 |
| (6) Xylene wax | 3.8 |
| (7) Candelilla wax | 5.5 |
| (8) Red iron oxide (Example 1-6 and Comparative Example 1-6) | 0.8 |
| (9) Titanium oxide (Example 1-4 and Comparative Example 1-4) | 11.5 |
| (10)Talc (Example 1-3 and Comparative Example 1-3) | 8.0 |
| (11) Antiseptic | approp. amount |
| (12) Perfume | approp. amount |

### (Producing method)

The above ingredients (8) to (10) were preliminarily mixed and pulverized. The preliminarily milled ingredients (8) to (11) were added to an oily phase in which ingredients (1) to (7) were mixed and dissolved at 85° C, which was dispersed homogeneously with the homodisperser. A perfume was added to the resultant, which was packed in a metal bowl, and an oily solid foundation was produced by cooling.

### (Table 1-16)

**Table 1-16**

| Evaluation item | Compound (a1) | Compound (b1) | Compound (c1) | Compound (d1) | Compound (e1) |
|---|---|---|---|---|---|
| Usability | A | B | B | D | E |
| Uniform finish | B | A | A | C | E |
| Long-lasting property | A | A | A | C | B |

### (Example 1-19 and Comparative Example 1-19) Production of emulsion type sunscreen creams

Emulsion type sunscreen creams having a composition shown in Table 1-17 were produced by the following method. Evaluation results were shown in Table 1-18.

### (Table 1-17)

**Table 1-17**

| Ingredients | Parts by weight |
|---|---|
| 1. Volatile fluid isoparaffin (isodecane) | 15.0 |
| 2. Dimethicone(6cs) | 2.0 |
| 3. Isononyl isononanoate | 3.5 |
| 4. Cetanol | 1.0 |
| 5. Squalane | 5.0 |
| 6. Monostearic acid polyethylene glycol (4EO) | 1.0 |
| 7. Hexaglyceryl ricinoleate | 3.5 |
| 8. Finely particulate Titanium oxide HFE *3 | 10.0 |
| 9. Finely particulate of zinc oxide (Ex. 1-11 & Com.Examplel-11) | 15.0 |
| 10. Purified water | balance |
| 11. Glycerin | 5.0 |
| 12. 1,3-butylene glycol | 5.0 |
| 13. Pyrrolidone carboxylate | 2.5 |
| 14. Antiseptic | approp.amount |
| 15. Perfume | approp.amount |

| | |
|---|---|
| *3 A dispersion was obtained by dispersing the finely particulate titanium oxide in Example 1-8 and Comparative Example 1-8 and hydrofluoroether (HFE7200: Sumitomo 3M Ltd.) at a composition of 30:70 (parts by weight) with a sand grinder. The surface-treated powder coated with the specific fluorine-containing copolymer according to the present invention had high pigment dispersability and storage stability. | |

Oily ingredients (1) to (9) except ingredient (8) were dissolved at 75° C. A water phase component of ingredients (10) to (14) was dissolved at 75° C, and the homogenized one was added to the oily-phase component, followed by emulsification with a homo mixer. Finally, the emulsion was added with ingredients (8) and (15), which was cooled, thereby producing a sunscreen cream.

### (Table 1-18)

**Table 1-18**

| Evaluation item | Compound (a1) | Compound (b1) | Compound (c1) | Compound (d1) | Compound (e1) |
|---|---|---|---|---|---|
| Usability | A | B | B | D | E |
| Uniform finish | B | A | A | C | E |
| Long- lasting property | A | A | A | C | B |
| SPPF value of formulation*4 | 23 | 25 | 26 | 20 | 15 |

| | | | | | |
|---|---|---|---|---|---|
| *4 An in-vitro SPF value is a figure that indicates a shielding effect of UV rays (UVB), and the greater the figure, the higher is the UV rays shielding effect. A surgical tape of 3M Corporation was bonded onto a quartz glass plate, a cream was applied to a tape face at a rate of 0.08g/cm2, and the in-vitro SPF value was measured with an SPF analyzer of US Optometrics, Inc. | | | | | |

### (Example 1-20 and Comparative Example 1-20) Production of eyeliners

Eyeliners having a composition shown in Table 1-19 were produced by the following method. Evaluation results were shown in Table 1-20.

### (Table 1-19)

**Table 1-19**

| Ingredients | Parts by weight |
|---|---|
| (1) Decamethylcyclopentasiloxane | 25.0 |
| (2) Dimethylpolysiloxane (6cs) | 4.0 |
| (3) Jojoba oil | 2.5 |
| (4) Polyether-modified silicone *5 | 1.0 |
| (5) Black iron oxide (Example 1-7 & Comparative Example 1-7) | 20.0 |
| (6) Ethanol | 5.0 |
| (7) Antiseptic | approp. amount |
| (8) Purified water | balance |

| | |
|---|---|
| *5 KF6026 manufactured by Shin-Etsu Chemical Co., Ltd. | |

### (Producing method)

(1) Ingredients 1 to 4 are mixed at 70°C. and an ingredient 5 was added and homogeneously dispersed thereinto.
(2) Ingredients 6 to 8 were heated to 70°C, and mixed and dissolved.
(3) The mixture obtained in (2) was gradually added to the mixture obtained in (1) under stirring, which was emulsified, thereby obtaining an eyeliner.

### (Table 1-20)

**Table 1-20**

| Evaluation item | Compound (a1) | Compound (b1) | Compound (c1) | Compound (d1) | Compound (e1) |
|---|---|---|---|---|---|
| Usability | B | B | B | D | E |
| Uniform finish | B | A | A | D | E |
| Long-lastin g property | A | A | A | D | B |

### (Example 1-21 and Comparative Example 1-21) Production of lipsticks

Lipsticks having a composition shown in Table 1-21 were produced by the following method. Evaluation results were shown in Table 1-22.

### (Table 1-21)

**Table 1-21**

| Ingredients | Parts by weight |
|---|---|
| 1. Diisostearyl Malate | 15.0 |
| 2. Glyceryl octanoate | balance |
| 3. Phytosterol glutamate octyl dodecanol | 15.0 |
| 4. Carnauba wax | 3.5 |
| 5. Squalane | 5.5 |
| 6. Candelilla wax | 5.0 |
| 7. Titanium oxide (Example 1-4 and Comparative Example 1-4) | 8.0 |
| 8. Red No. 202 (Example 1-14 and Comparative Example 1-14) | 0.7 |
| 9. Black iron oxide (Example 1-7 and Comparative Example 1-7) | 0.2 |
| 10. Antiseptic | approp. amount |

### (Producing method)

(1) Ingredients 7 to 9 were added to an ingredient 1, which was kneaded and homogeneously dispersed with rollers.
(2) After the other ingredients were heated, mixed and dissolved, the ingredients in (1) were added thereto, which was homogeneously dispersed with the homo mixer.
(3) After deaeration, the dispersion was poured into a mold, thereby obtaining a stick-shaped lipstick.

### (Table 1-22)

**Table 1-22**

| Evaluation item | Compound (a1) | Compound (b1) | Compound (c1) | Compound (d1) | Compound (e1) |
|---|---|---|---|---|---|
| Usability | B | B | B | C | C |
| Uniform finish | B | A | A | D | E |
| Long-lastin g property | A | A | A | D | B |

### (Example 1-22 and Comparative Example 1-22) Production of lipstick overcoats

Lipstick overcoats having a composition shown in Table 1-23 were produced by the following method. Evaluation results were shown in Table 1-24.

### (Table 1-23)

**Table 1-23**

| Ingredients | Parts by weight |
|---|---|
| (1) Perfluoropolyether (FOMBLIN HC-04) | 91.5 |
| (2) Silica (Aerosil R-972) *6 | 2.0 |
| (3) Finely particulate titanium oxide (Example 1-8 and Comparative Example 1-8) | 6.0 |
| (4) Glycerin | 1.0 |

| | |
|---|---|
| *6 Silica treated with each of fluorine-containing compounds (a1) to (e1) at 5% by the method in Example 1-1 and Comparative Example 1-1 was blended. | |

### (Producing method)

After ingredients (2) and (3) were added to an ingredient (1) and the resultant was homogeneously dispersed, and a lipstick overcoat was produced by adding an ingredient (4) thereto.

### (Table 1-24)

**Table 1-24**

| Evaluation item | Compound (a1) | Compound (b1) | Compound (c1) | Compound (d1) | Compound (e1) |
|---|---|---|---|---|---|
| Usability | B | B | B | B | C |
| Uniform finish | B | A | A | D | E |
| Long-lasting property | A | A | A | D | B |

### (Example 1-23 and Comparative Example 1-23) Production of emulsion type liquid foundations

O/W type liquid foundations having a composition shown in Table 1-25 were produced by the following method. Evaluation results were shown in Table 1-26.

### (Table 1-25)

**Table 1-25**

| Ingredients | Parts by weight |
|---|---|
| (1) Stearic acid | 1.5 |
| (2) Cetanol | 1.0 |
| (3) Polyoxyethylene sorbitan monooleate (3EO) | 1.0 |
| (4) Sorbitan sesquioleate | 1.0 |
| (5) Fluid paraffin | 5.0 |
| (6) 2-Ethyl glyceryl hexanoate | 5.0 |
| (7) Glycerin | 5.0 |
| (8) 1,3-butylene glycol | 5.0 |
| (9) Alkyl (C10-30) acrylate-methacrylate copolymer | 0.05 |
| (10) Locust bean gum | 0.2 |
| (11) Triethanol gum | 0.8 |
| (12) Antiseptic | approp. amount |
| (13) Purified water | balance |
| (14) Titanium oxide (Example 1-4 and Comparative Example 1-4) | 5.0 |
| (15) Yellow iron oxide (Example 1-5 and Comparative Example 1-5) | 1.0 |
| (16) Red iron oxide (Example 1-6 and Comparative Example 1-6) | 0.3 |
| (17) Black iron oxide (Example 1-7 and Comparative Example 1-7) | 0.1 |
| (18) Amihope LL (Example 1-13 and Comparative Example 1-13) | 5.0 |

### (Producing method)

A: Ingredients (1) to (6) were heated and dissolved.
B: Ingredients (7) to (13) were heated and dissolved.
C: The mixture obtained in B was added to the mixture obtained in A, and ingredients (14) to (18) were added thereto after cooling, which was homogeneously mixed, thereby producing a liquid foundation.

### (Table 1-26)

**Table 1-26**

| Evaluation item | Compound (a1) | Compound (b1) | Compound (c1) | Compound (d1) | Compound (e1) |
|---|---|---|---|---|---|
| Usability | B | B | B | B | C |
| Uniform finish | B | A | A | D | E |
| Long-last ing property | A | A | A | D | B |

### (Example 1-24 and Comparative Example 1-24) Production of skin color emulsions

Skin color emulsions having a composition shown in Table 1-27 were produced by the following method. Evaluation results were shown in Table 1-28.

### (Table 1-27)

**Table 1-27**

| Ingredients | Parts by weight |
|---|---|
| (1) N-stearoyl-L-glutamate | 0.5 |
| (2) Cetanol | 0.5 |
| (3) Polyoxyethylene (10mol) monostearate | 0.8 |
| (4) Decaglyceryl monoisostearate | 1.0 |
| (5) Sorbitan sesquioleate | 0.4 |
| (6) Methylphenyl polysiloxane | 5.0 |
| (7) Glycerin | 5.0 |
| (8) 1,3-butylene glycol | 5.0 |
| (9) Xanthane gum | 0.1 |
| (10) Carrageenan | 0.05 |
| (11) Triethanol amine | 1.0 |
| (12) Antiseptic | approp. amount |
| (13) Purified water | balance |
| (14) Titanium oxide (Example 1-4 and Comparative Example 1-4) | 2.0 |
| (15) Yellow iron oxide (Example 1-5 and Comparative Example 1-5) | 1.0 |
| (16) Red iron oxide (Example 1-6 and Comparative Example 1-6) | 0.5 |
| (17) Black iron oxide (Example 1-7 and Comparative Example 1-7) | 0.1 |
| (18) Talc (Example 1-3 and Comparative Example 1-3) | 3.0 |

### (Producing method)

A: Ingredients (1) to (6) were heated and dissolved.
B: Ingredients (7) to (13) were homogeneously heated and dissolved.
C: The mixture obtained in B was added to the mixture obtained in A, and ingredients (14) to (18) were added thereto, which was homogeneously mixed, thereby producing a skin color emulsion.

### (Table 1-28)

**Table 1-28**

| Evaluation item | Compound (a1) | Compound (b1) | Compound (c1) | Compound (d1) | Compound (e1) |
|---|---|---|---|---|---|
| Usability | A | B | B | D | D |
| Uniform finish | B | A | A | D | D |
| Long-lasting property | A | A | A | B | B |

### (Example 1-25 and Comparative Example 1-25) Production of nail manicures

Manicures having a composition shown in Table 1-29 were produced by the following method. Evaluation results were shown in Table 1-30.

### (Table 1-29)

**Table 1-29**

| Ingredients | Parts by weight |
|---|---|
| (1) Nitro cellulose | 10.0 |
| (2) Alkyd resin | 10.0 |
| (3) Acetyl tributyl Citrate | 4.0 |
| (4) d1-Caffeine | 1.0 |
| (5) Organic-modified hectorite | 1.0 |
| (6) Ethyl acetate | 20.0 |
| (7) Butyl acetate | balance |
| (8) Isopropyl alcohol | 5.0 |
| (9) Red No. 202 (Example 1-14 and Comparative Example 1-14) | 0.1 |
| (10) Titanium oxide (Example 1-4 and Comparative Example 1-4) | 3.0 |
| (11) Pearl pigment (Example 1-12 and Comparative Example 1-12) | 5.0 |
| (12) Red iron oxide (Example 1-6 and Comparative Example 1-6) | 0.5 |

### (Producing method)

Ingredients (1) to (9) were mixed and stirred in a disperser. Ingredients (10) to (12) were added thereto, and a manicure was produced by mixing the resultant in the disperser for 10 minutes, and packed into a glass bottle.

### (Table 1-30)

**Table 1-30**

| Evaluation item | Compound (a1) | Compound (b1) | Compound (c1) | Compound (d1) | Compound (e1) |
|---|---|---|---|---|---|
| Usability | B | B | B | D | D |
| Uniform finish | B | B | B | D | D |
| Long-lasting property | A | A | A | B | B |

Meanwhile, the manicure into which the surface-treated powder coated with the specific fluorine-containing copolymer of the present invention was blended had good dispersion stability without becoming a hard cake with a lapse of time.

### (Example 1-26 and Comparative Example 1-26) Production of cheek colors

Cheek colors having a composition shown in Table 1-31 were produced by the following method. Evaluation results were shown in Table 1-32.

### (Table 1-31)

**Table 1-31**

| Ingredients | Parts by weight |
|---|---|
| (1) Mica (Example 1-2 and Comparative Example 1-2) | 14.0 |
| (2) Titanium oxide (Example 1-4 and Comparative Example 1-4) | 10.0 |
| (3) Yellow iron oxide (Example 1-5 and Comparative Example 1-5) | 1.0 |
| (4) Red iron oxide (Example 1-6 and Comparative Example 1-6) | 0.5 |
| (5) Black iron oxide (Example 1-7 and Comparative Example 1-7) | 0.1 |
| (6) Talc (Example 1-3 and Comparative Example 1-3) | balance |
| (7) Perfluoropolyether (FOMBLIN HC-04) | 7.0 |
| (8) Dimethylpolysiloxane (10CS) | 5.0 |

### (Producing method)

Ingredients (1) to (6) were homogeneously mixed and pulverized. After ingredients (7) and (8) were added and homogeneously mixed thereinto, a cheek color was produced by pulverizing.

### (Table 1-32)

**Table 1-32**

| Evaluation item | Compound (a1) | Compound (b1) | Compound (c1) | Compound (d1) | Compound (e1) |
|---|---|---|---|---|---|
| Usability | B | B | B | E | E |
| Uniform finish | A | A | B | D | D |
| Long-lastin g property | A | A | A | B | B |

### (Example 1-27 and Comparative Example 1-27) Production of antiperspirant cosmetics

Stick-shaped antiperspirant cosmetics having a composition shown in Table 1-33 were produced by the following method. Evaluation results were shown in Table 1-34.

### (Table 1-33)

**Table 1-33**

| Ingredients | Parts by weight |
|---|---|
| (1) Carnauba wax | 1.0 |
| (2) Ceresin | 6.0 |
| (3) Paraffin wax | 3.0 |
| (4) Sorbitan sesqui isostearate | 2.5 |
| (5) Cetyl 2-ethylhexanoate | 27.0 |
| (6) Squalane | 2.0 |
| (7) Decamethylcyclopentasiloxane | balance |
| (8) Aluminum hydroxychloride | 25.0 |
| (9) Sericite (Example 1-1 and Comparative Example 1-1) | 10.0 |
| (10) Talc (Example 1-3 and Comparative Example 1-3) | 8.0 |
| (11) Finely particulate zinc oxide (Example 1-11 and Comparative Example 1-11) | 2.0 |
| (12) Antioxidant | approp. amount |
| (13) Perfume | approp. amount |

### (Producing method)

Ingredients (1) to (7) were heated and homogeneously dissolved. After ingredients (8) to (12) were added and homogeneously mixed thereinto, a stick-shaped antiperspirant cosmetic was produced by adding an ingredient (13) thereto and cooling the resultant.

### (Table 1-34)

**Table 1-34**

| Evaluation item | Compound (a1) | Compound (b1) | Compound (c1) | Compound (d1) | Compound (e1) |
|---|---|---|---|---|---|
| Usability | B | A | B | E | E |
| Uniform finish | A | A | B | D | D |
| Long-lastin g property | A | A | A | B | B |

### (Example 1-28 and Comparative Example 1-28) Production of hair dressing agents

Hair dressing agents having a composition shown in Table 1-35 were produced by the following method. Evaluation results were shown in Table 1-36.

### (Table 1-35)

**Table 1-35**

| Ingredients | Parts by weight |
|---|---|
| (1) Carnauba wax | 1.0 |
| (2) Fluid paraffin | 10.0 |
| (3) Squalane | 5.0 |
| (4) Stearyl alcohol | 1.5 |
| (5) Dimethyl polysiloxane (10cs) | 3.0 |
| (6) Stearic acid | 6.0 |
| (7) Polyoxyethylene (3) stearyl alcohol | 4.5 |
| (8) Polyoxyethylene (150) cetyl ether | 2.0 |
| (9) Organic-modified bentonite | 0.2 |
| (10)Amihope LL (Example 1-13 and Comparative Example 1-13) | 2.0 |
| (11) 1,3-butylene glycol | 6.0 |
| (12) Antioxidant | approp. amount |
| (13) Perfume | approp. amount |
| (14) Purified water | balance |

### (Producing method)

A: Ingredients (1) to (9) were heated and homogeneously dissolved.
   An ingredient (10) was added and homogeneously dissolved thereinto.
B: Ingredients (11), (12) and (14) were mixed under heating.
C: The mixture obtained in B was added and mixed into the mixture obtained in A, and after cooling, an ingredient (13) was added thereto, which was cooled, thereby producing a hair dressing agent.

### (Table 1-36)

**Table 1-36**

| Evaluation item | Compound (a1) | Compound (b1) | Compound (c1) | Compound (d1) | Compound (e1) |
|---|---|---|---|---|---|
| Usability | B | A | B | E | E |
| Uniform finish | A | A | A | D | D |
| Long-last ing property | A | A | A | D | C |

### (Example 1-29 and Comparative Example 1-29) Production of skin color serum

Skin color moisturizing serums having a composition shown in Table 1-37 were produced by the following method. Evaluation results were shown in Table 1-38.

### (Table 1-37)

**Table 1-37**

| Ingredients | Parts by weight |
|---|---|
| (1) Purified water | balance |
| (2) Alkyl (C10-30) acrylate-methacrylate copolymer | 0.1 |
| (3) Carboxyvinyl polymer | 1.0 |
| (4) Antiseptic | approp.amount |
| (5) Finely particulate titanium oxide (Example 1-9 and Comparative Example 1-9) | 1.0 |
| (6) Triethanolamine | 0.7 |
| (7) Glycerin | 25.0 |
| (8) Titanium oxide (Example 1-4 and Comparative Example 1-4) | 2.0 |
| (9) Yellow iron oxide (Example 1-5 and Comparative Example 1-5) | 0.8 |
| (10) Red iron oxide (Example 1-6 and Comparative Example 1-6) | 0.3 |
| (11) Black iron oxide (Example 1-7 and Comparative Example 1-7) | 0.1 |
| (12) Talc (Example 1-3 and Comparative Example 1-3) | 3.5 |

### (Producing method)

A: After ingredients (1) to (4) are homogeneously dissolved under heating, and an ingredient (5) is added and homogeneously mixed thereinto. An ingredient (6) is added to neutralize the mixture.
B: After ingredients (8) to (12) are homogeneously mixed, an ingredient (7) is added and homogeneously mixed thereinto.
C: A skin color moisturizing serum was produced by adding and mixing the mixture obtained in B into the mixture obtained in A.

### (Table 1-38)

**Table 1-38**

| Evaluation item | Compound (a1) | Compound (b1) | Compound (c1) | Compound (d1) | Compound (e1) |
|---|---|---|---|---|---|
| Usability | B | B | B | E | E |
| Uniform finish | A | A | A | D | D |
| Longlasting property | A | A | A | C | B |

### (Example 1-30 and Comparative Example 1-30) Production of Liquid eye shadows

Aqueous liquid eye shadows having a composition shown in Table 1-39 were produced by the following method. Evaluation results were shown in Table 1-40.

### (Table 1-39)

**Table 1-39**

| Ingredients | Parts by weight |
|---|---|
| (1) Purified water | balance |
| (2) Alkyl (C10-30) acrylate-methacrylate copolymer | 0.1 |
| (3) Carboxyvinyl polymer | 1.0 |
| (4) Antiseptic | approp. amount |
| (5) Triethanolamine | 0.7 |
| (6) Glycerin | 25.0 |
| (7) Titanium oxide (Example 1-4 and Comparative Example 1-4) | 2.5 |
| (8) Red iron oxide (Example 1-6 and Comparative Example 1-6) | 2.0 |
| (9) Pearl pigment (Example 1-12 and Comparative Example 1-12) | 8.0 |

### (Producing method)

A: After ingredients (1) to (4) are homogeneously dissolved under heating, an ingredient (5) is added and homogeneously mixed thereinto.
B: After ingredients (7) to (9) are homogeneously mixed, an ingredient (6) is added and homogeneously mixed thereinto.
C: A liquid aqueous eye shadow was produced by adding and mixing the mixture obtained in B into the mixture obtained in A.

### (Table 1-40)

**Table 1-40**

| Evaluation item | Compound (a1) | Compound (b1) | Compound (c1) | Compound (d1) | Compound (e1) |
|---|---|---|---|---|---|
| Usability | B | B | B | D | D |
| Uniform finish | A | A | A | D | D |
| Long-last ing property | A | A | A | B | B |

### (Example 1-31 and Comparative Example 1-31) Production of whitening powders

Whitening powders having a composition shown in Table 1-41 were produced by the following method. Evaluation results were shown in Table 1-42.

### (Table 1-41)

**Table 1-41**

| Ingredients | Parts by weight |
|---|---|
| (1) Sodium ascorbic phosphate | 1.0 |
| (2) Sodium citrate | 1.0 |
| (3) 1,3-bytylene glycol | 5.0 |
| (4) Glycerin | 5.0 |
| (5) Purified water | balance |
| (6) Methyl paraoxybenzoate | 0.2 |
| (7) Mica (Example 1-2 and Comparative Example 1-2) | 5.0 |
| (8) Talc (Example 1-3 and Comparative Example 1-3) | 0.1 |
| (9) Organopolysiloxane elastomer powder | 3.0 |
| (10)Trifluoroalkyl dimethyl trimethyl siloxane | 3.0 |
| (11) Partially crosslinked type organopolysiloxane polymer *7 | 5.0 |
| (12) Red iron oxide (Example 1-6 and Comparative Example 1-6) | 0.5 |

| | |
|---|---|
| *7 KSG-16 (Shin-Etsu Chemical Co., Ltd.) | |

### (Producing method)

A: Ingredients (7) to (9) are mixed and pulverized.
B: After ingredients (11) to (12) are homogeneously mixed, the mixture obtained in the above A is added thereto.
C: Ingredients (1) to (6) are mixed and dissolved.
D: The mixture obtained in the above B and the mixture obtained in C were mixed, which was packed into a container, thereby obtaining a whitening powder.

### (Table 1-42)

**Table 1-42**

| Evaluation item | Compound (a1) | Compound (b1) | Compound (c1) | Compound (d1) | Compound (e1) |
|---|---|---|---|---|---|
| Usability | B | B | B | D | D |
| Uniform finish | A | A | A | D | D |
| Long-lastin g property | A | A | A | B | B |

### (Example 1-32 and Comparative Example 1-32) Production of sunscreen creams

Sunscreen creams having a composition shown in Table 1-43 were produced by the following method. Evaluation results were shown in Table 1-44.

### (Table 1-43)

**Table 1-43**

| Ingredients | Parts by weight |
|---|---|
| (1) Decamethylcyclopentasiloxane | 15.0 |
| (2) Long-chain alkyl-containing POE-modified silicone (ABIL EM-90: Degussa GmbH) | 2.0 |
| (3) Isononyl isononanoate | 5.0 |
| (4) Diethylhexyl succinate | 5.0 |
| (5) Avobenzone (Parsol 1789 : DSM Nutrition Japan Co., Ltd.) | 0.8 |
| (6) Octocrylene (Eusolex OCR : Merck Co., Ltd.) | 5.0 |
| (7) Finely particulate zinc oxide (Example 1-11 and Comparative Example 1-11) | 10.0 |
| (8) Purified water | balance |
| (9) Glycerin | 3.0 |
| (10) Ethanol | 5.0 |
| (11) Finely particulate titanium aqueous dispersion (Example 1-10 and Comparative Example 1-10) *8 | 10.0 |
| (12) Antiseptic 15 | approp. amount |

| | |
|---|---|
| *8 An aqueous dispersion was obtained by dispersing the composition of the finely particulate titanium oxide/purified water/glycerin = 45/50/5 in Example 1-10 and Comparative Example 1-10 with a sand grinder. The aqueous dispersion in Example was stable free of precipitating separation even with a lapse of time. A dispersed body in Comparative Example did not proceed with dispersing, so that a uniform and stable dispersion could not be produced. | |

### (Producing method)

A: Ingredients (3) to (7) are heated and mixed.
B: After ingredients (1), (2) and (6) are homogeneously mixed, the resultant is added to the mixture obtained in the above A.
C: Ingredients (8) to (12) are mixed and dissolved.
D: A sunscreen cream was obtained by adding the mixture obtained in the above C into the mixture obtained in B and emulsifying the resultant.

### (Table 1-44)

**Table 1-44**

| Evaluation item | Compound (a1) | Compound (b1) | Compound (c1) | Compound (d1) | Compound (e1) |
|---|---|---|---|---|---|
| Usability | A | A | A | E | E |
| Uniform finish | A | A | A | D | D |
| Long-lasting property | A | A | A | D | D |
| Quality Stability*9 | A | A | A | E | E |

| | | | | | |
|---|---|---|---|---|---|
| *9 Regarding the quality stability, a sample was left in a thermostatic bath at 50 °C for one week, and whether crystallization, separation and precipitation of the UV absorber occurred or not were observed. A result was judged good for one with no change in appearance such as crystallization, etc. The cosmetics into which the compounds (d1) and (e1) in Comparative Examples were blended caused the crystallization and separation the next day, so they had poor quality stability. | | | | | |

### (Example 1-33 and Comparative Example 1-33)

Into 500g of water prepared in a 2L glass beaker was put 100g of Talc JA-13R (Asada Milling Co., Ltd.), which is fully stirred and dispersed. After a liquid in which 3g as a solid content of the fluorine-containing copolymer in Producing Example 1-4 and the fluorine-containing copolymer of the compound (e1) in Comparative Example and 10g of IPA are dissolved is gradually added thereto, which is stirred for 15 minutes. The glass beaker is moved to a hot plate, and heated at a condition of 3°C/min., and its temperature is kept when it reaches 80°C. (Compounds (a1) to (d1) having oxyethylene in the molecular structure of the polymer of the surface-treating agent are dewatered during heating due to a cloud point phenomenon, so that they encompass and coat the powder particles.) After the stirring was kept for 15 minutes, the talc treated with each of the fluorine-containing compounds was obtained by pulverizing with an atomizer after dewatering, washing and drying (115°C/16 hours).

### (Example 1-34 and Comparative Example 1-34)

Into 500g of water prepared in a 2L glass beaker was put 100g of Talc JA-13R (Asada Milling Co., Ltd.), which was fully stirred and dispersed. After a liquid in which 3g as a solid content of the fluorine-containing copolymer in Producing Example 1-4 and the fluorine-containing copolymer of the compound (e1) in Comparative Example and 10g of IPA were dissolved is gradually added thereto, which is then stirred for 15 minutes. Next, a liquid in which 30g of NaCl is dissolved in 100g of water is gradually added thereto, while stirring is being maintained. (Compounds (a1) to (d1) having oxyethylene in the molecular structure of the polymer of the surface-treating agent are dewatered due to a salting-out phenomenon, so that they encompass and coat the powder particles.) After the stirring was kept for 15 minutes, the talc treated with each of the fluorine-containing compound was obtained by pulverizing with an atomizer after dewatering, washing and drying (115°C/16 hours).

### (Example 1-35 and Comparative Example 1-35)

Into 500g of water prepared in a 2L glass beaker is put 100g of Talc JA-13R (Asada Milling Co., Ltd.), which is fully stirred and dispersed. After a liquid in which 3g as a solid content of the fluorine-containing copolymer in Producing Example 1-4 and the fluorine-containing copolymer of the compound (e2) in Comparative Example and 10g of IPA are dissolved is gradually added thereto, which is then stirred for 15 minutes. The thus obtained slurry is fed to a spray dryer equipped with a twin-fluid spray nozzle. The temperature of hot air at an inlet of the spray dryer is set at 200°C, and a spraying pressure of the twin-fluid nozzle is set at 0.6MPa. In this matter, a talc treated with each of fluoride-containing compounds was obtained.

### (Example 1-36 and Comparative Example 1-36)

Talc JA-13R (Asada Milling Co., Ltd.), 5kg, and 5kg of water are converted to a slurry by kneading in a kneader. Into this slurry is put 3g as a solid content of the fluorine-containing polymer in Producing Example 1-4 and the fluorine-containing polymer of the compound (e1) Comparative Example, and the mixture is mechanochemically treated with a grindstone type friction grinding machine: Super mass Colloider (MKCA 6-2: MASUKO SANGYO CO.,LTD). The obtained slurry was dried with a vacuum vibration dryer. In this way, the talk treated with each of the fluorine-containing compound was obtained.

### (Example 1-37 and Comparative Example 1-37)

Crystals of titanyl sulfate was put into water, which was heated to produce a hydrolysate. To 350g (the content of titanium oxide: 100g) of a cake of the hydrous titanium hydroxide obtained by filtering and washing the hydrolysate was added 400g of a 48% aqueous solution of sodium hydroxide, which was heated and stirred for 2 hours in a range of 95 to 105° C. Next, a suspension of a hydrate of the resulting titanium dioxide was filtered and washed. A slurry was obtained by adding 500g of water to the washed cake, 140g of 35% hydrochloric acid was further added thereto, while being stirred, and the resultant was aged under heating at 95°C for 2 hours. The concentration of the thus obtained aqueous suspension of the finely particulate titanium oxide was adjusted to 70g/L. To this aqueous suspension liquid was added polyaluminum chloride (10 wt% relative to the finely particulate titanium oxide when calculated as Al2O3), and pH was adjusted to 5.0 by using caustic soda, followed by aging for 30 minutes.
After the aging, 7 wt% as a solid content of the fluorine-containing copolymer in Producing Example 1-4 and the fluorine-containing copolymer of the compound (e1) in Comparative Example was added, followed by aging for 30 minutes. The resultant was filtered, washed, dried, and pulverized by an Eck atomizer. In this manner, the finely particulate titanium oxide treated with each of the fluorine-containing compounds was obtained.

### (Example 1-38 and Comparative Example 1-38) Production of sunscreen gels

Sunscreen gels having a composition shown in Fig. 1-45 were produced by the following method. Evaluation results were shown in Table 1-46.

### (Table 1-45)

**Table 1-45**

| Ingredients | Parts by weight |
|---|---|
| (1) Glycerin | 5.0 |
| (2) Long-chain alkyl acrylate-methacryl copolymer ((Pemulen TR-1; BF Goodrich Corporation) | 0.3 |
| (3) Methyl paraffin | 0.2 |
| (4) Purified water | to 100.0 |
| (5) Potassium hydroxide | 0.2 |
| (6) Purified water | 5.0 |
| (7) Finely particulate titanium (Example 1-37 and Comparative Example 1-37) | 10.0 |
| (8) Paradimethylamino isooctyl benzoate | 5.0 |
| (9) Glyceryl monooleyl ether | 1.0 |

### (Producing method)

Ingredients (1) to (4) are heated and dissolved at 80°C. Ingredients (5) and (6) are heated and dissolved at 80°C. The former is neutralized by adding the heated and dissolved ingredients (5) and (6) thereto. Ingredients (7) to (9) are heated at 80°C, and mixed and dispersed. A sunscreen gel was obtained by gradually adding these ingredients to the above-neutralized ingredients and emulsifying the resultant with a homo mixer.

### (Table 1-46)

**Table 1-46**

| Evaluation item | Example | Comparative Example |
|---|---|---|
| Usability | A | D |
| Uniform finish | B | D |
| Long-lasting property | A | D |
| In-vitro SPF value | 20 | 15 |
| Temporary Stability*10 | B | E |

| | | |
|---|---|---|
| * 10 These formulations were stored at 50°C for one month. Those having no appearance changed were given B, and that having an appearance change was given E. | | |

(Example 1-39 and Comparative Example 1-39) Production of wet molded type powder foundations having a composition shown in Table 1-47 were produced by the following method. Evaluation results were shown in Table 1-48.

### (Table 1-47)

**Table 1-47**

| Ingredients | Parts by weight |
|---|---|
| (1) Talc JA-13R (Example 1-33 and Comparative Example 1-33) | to 100.0 |
| (2) MW treatment (hydrophilic treatment)PMMA(Miyoshi Kasei Inc.) | 10.0 |
| (3) Titanium oxide (Example 1-4 and Comparative Example 1-4) | 12.0 |
| (4) Yellow iron oxide (Example 1-5 and Comparative Example 1-5) | 2.5 |
| (5) Red iron oxide (Example 1-6 and Comparative Example 1-6) | 0.8 |
| (6) Black iron oxide (Example 1-7 and Comparative Example 1-7) | 0.3 |
| (7) 2-Ethylhexyl para methoxy cinnamate | 5.0 |
| (8) Polyoxyethylene (20mol) hydrogenated castor oil | 1.5 |

### (Producing method)

A: Ingredients (7) and (8) are heated to 75°C, and homogeneously dissolved.
B: Ingredients (1) to (6) are mixed with a mixer, and pulverized with an atomizer.
C: A is added and homogeneously dispersed into the above B, while B is being stirred.
D: To 100 parts by weight of the C component is added 100 parts by weight of purified water, which is changed to a slurry by homogeneously mixing.
E: The above D is packed into a metal bowl, and a part of purified water is removed by placing and pressing an absorbent paper onto a surface thereof.
F: The F was left in a thermostatic bath at 30° C for 24 hours, and a wet molded type powder foundation was obtained by completely removing purified water.

### (Table 1-48)

**Table 1-48**

| Evaluation item | Example | Comparative Example |
|---|---|---|
| Usability | A | D |
| Uniform finish | B | D |
| Long-lasting property | A | D |

### (Example 1-40 and Comparative Example 1-40) Production of F/W emulsion lotions

Lotions having a composition shown in Table 1-49 were produced by the following method. Evaluation results were shown in Table 1-50.

### (Table 1-49)

**Table 1-49**

| Ingredients | Parts by weight |
|---|---|
| (1) Perfluoropolyether (FOMBLIN HC/04: Solvay Co., Ltd.) | 25.0 |
| (2) Hydrofluoroether (CF-76: 3M Corporation) | 10.0 |
| (3) Talc JA-13R (Example 1-34 and Comparative Example 1-34) | 6.0 |
| (4) Ethanol | 10.0 |
| (5) Glycerin | 3.5 |
| (6) Purified water | to 100.0 |
| (7) Antiseptic | approp. amount |

### (Producing method)

A: Ingredients (1) to (3) are mixed and heated to 50°C.
B: Ingredients (4) to (7) are dispersed, dissolved and mixed in a homo mixer, which is heated at 50° C.
C: An F/W emulsion lotion was obtained by gradually adding the above A, while the above B was being stirred.

### (Table 1-50)

**Table 1-50**

| Evaluation item | Example | Comparative Example |
|---|---|---|
| Usability | A | D |
| Uniform finish | B | D |
| Long-lasting property | A | E |

### (Example 1-41 and Comparative 1-41) Production of W/F emulsion lotions

Lotions having a composition shown in Table 1-51 were produced by the following method. Evaluation results were shown in Table 1-52.

### (Table 1-51)

**Table 1-51**

| Ingredients | Parts by weight |
|---|---|
| (1) Perfluoropolyether (FOMBLIN HC/04: Solvay Co., Ltd.) | 45.0 |
| (2) Hydrofluoroether (CF-76: 3M Corporation) | 15.0 |
| (3) Talc (Example 1-35 and Comparative Example 1-35) | 5.5 |
| (4) Ethanol | 5.0 |
| (5) Glycerin | 3.5 |
| (6) Purified water | to 100.0 |
| (7) Antiseptic | approp. amount |

### (Producing method)

A: Ingredients (1) to (3) are homogeneously mixed and heated to 50°C.
B: Ingredients (4) to (7) are dispersed, dissolved and mixed in a homo mixer, which is heated to 50° C.
C: A W/F emulsion lotion was obtained by gradually adding the above B, while the above A was stirred with the honomixer.

### (Table 1-52)

**Table 1-52**

| Evaluation item | Example | Comparative Example |
|---|---|---|
| Usability | A | D |
| Uniform finish | B | D |
| Long-lasting property | A | E |

### (Example 1-42 and Comparative Example 1-42) Production of powder-emulsified sunscreen creams

Lotions having a composition shown in Table 1-53 were produced by the following method. Evaluation results were shown in Table 1-54.

### (Table 1-53)

**Table 1-53**

| Ingredients | Parts by weight |
|---|---|
| (1) Isohexadecane | 20.0 |
| (2) Isotridecyl isononanoate | 7.0 |
| (3) Finely particulate titanium oxide (Example 1-37 and Comparative Example 1-37) | 10.0 |
| (4) Talc JA-13R (Example 1-36 and Comparative Example 1-36) | 3.0 |
| (5) Dimethyl silylated silica (Aerosil R-972: Japan Aerosil Co., Ltd.) | 2.0 |
| (6) Ethanol | 5.0 |
| (7) Glycerin | 7.0 |
| (8) Purified water | to 100 |
| (9) Antiseptic | approp. amount |

### (Producing method)

A: Ingredients (1) to (5) are homogeneously, and heated to 50° C.
B: Ingredients (6) to (9) are heated to 50°C, and homogeneously mixed.
C: A powder-emulsified sunscreen cream was obtained by gradually adding the above B, while the above A was stirred with a homo mixer.

### (Table 1-54)

**Table 1-54**

| Evaluation item | Example | Comparative Example |
|---|---|---|
| Usability | A | D |
| Uniform finish | B | D |
| Long-lasting property | A | D |

### (Producing Example 2-1 to 2-3)

Producing examples of fluorine-containing copolymers to be used in the present invention will be shown below.

### (Producing Example 2-1)

Into a 100ml four-necked flask equipped with a reflex cooling tube, a nitrogen introduction tube, a thermometer and a stirrer were charged 18.6g of a fluorine-containing monomer CH2 = CHC(=O)O - CH2CH2C6F13 (hereinafter referred to as C6SFA(a)), 11.4g of polyethylene glycol acrylate CH2=CHC(=O)O-(CH2CH2O)n-H (BLEMMER AE 90, manufactured by NOF Corporation, the average value of n is 2, hereinafter referred to as AE90(b)), 0.3g of dodecane thiol and 45g of isopropanol , followed by bubbling with nitrogen for 30 minutes. The internal temperature was raised to 50 to 65°C under nitrogen stream, and 0.4g of perbutyl PV (hereinafter referred to as PV) was added to perform a reaction at 60 to 65 ° C for 10 hours. Isopropanol was distilled off from the resulting solution at about 70°C under a reduced pressure condition, thereby obtaining a residue of a light yellow polymer. Then, after 122.4g of water was added and the internal temperature was kept at about 80°C for not less than 1 hour, a water dispersion having about 20 wt% of a solid concentration was prepared by cooling.

### (Producing Example 2-2)

Into a 100ml four-necked flask equipped with a reflex cooling tube, a nitrogen introduction tube, a thermometer and a stirrer were charged 18.6g of C6SFA(a), 5.7g of AE90(b), 5.7g of polyethylene glycol acrylate CH2=CHC(=O)O-(CH2CH2O)n-H (BLEMMER AE200, manufactured by NOF Corporation, the average value of n is 4.5, hereinafter referred to as AE200(b), 0.3g of dodecane thiol and 45g of isopropanol, and a polymerization reaction was carried out in the same manner as in Producing Example 2-1, thereby preparing a water dispersion having about 20 wt% of a solid concentration.

### (Producing Example 2-3)

Into a 100ml four-necked flask equipped with a reflex cooling tube, a nitrogen introduction tube, a thermometer and a stirrer were charged 18.6g of C6SFA(a), 10.5g of AE350(b), 0.9g of polyethylene glycol diacrylate CH2=CHC(=O)O - (CH2CH2O)n - C(=O)CH=CH2 (BLEMMER ADE300, manufactured by NOF Corporation, the average value of n is 7), 0.3g of dodecane thiol and 45g of isopropanol, and a polymerization reaction was carried out in the same manner as in Producing Example 2-1, thereby preparing a water dispersion having about 20 wt% of a solid concentration.

### (Table 2-1)

**Table 2-1**

| Compound | Structure | Remarks |
|---|---|---|
| (a 2) | Fluoride-containing copolymer in Production Example 1 | treating agent to be used in the present invention |
| (b 2) | Fluoride-containing copolymer in Production Example 2 | treating agent to be used in the present invention |
| (c 2) | Fluoride-containing copolymer in Production Example 3 | treating agent to be used in the present invention |
| (d 2) | Fluoride-containing copolymer in Production Example 1 of JP-A 2000-290640 (17FA/HEMA) | Conventional treating agent (Comparative Example) |
| (e 2) | Fluoride-containing copolymer in Production Example 4 of JP-A 2005-213485 | Conventional treating agent (Comparative Example) |

### (Examples 2-1 to 2-14 and Comparative Examples 2-1 to 2-14)

(a2) to (c2) shown in Table 2-1 are respective fluorine-containing copolymers to be used in the present invention, and copolymers shown in (d2) and (e2) are conventional compounds (Comparative Examples). Powders surface-treated with these respective compounds were produced, and evaluations were performed regarding respective physical properties of these surface-treated powders. In the following, Producing Examples and evaluation items and methods for the surface-treated powders were shown. Note that the surface treatment with the fluorine-containing copolymers to be used in the present invention shown in the above Table 2-1 is taken as FAW treatment in Examples.

Evaluation methods for the treated powders were shown below.

### (Contact angle tests for water and squalane)

A powder is packed into a metal bowl, and molded by pressing it for 10 seconds under pressure of 6MPa, Water or squalane is dropwise dropped on a surface of the molded body, and an angle formed between the liquid drop and the surface of the powder is measured. The liquid was dropwise dropped on the surface of the molded powder (dropped amount 10µl) by a contact angle meter (CA-D) manufactured by Kyowa Interface Science Co., Ltd., and an angle formed by the liquid drop and the powder-coated face 20 seconds later was measured (n value was set to 5). When a contact angle could not be measured due to absorption of a liquid drop into the powder, it was taken as penetration.

### (Tests on usability and adhesion)

Usability and adhesion of each of the surface-treated powders for the identical powder were evaluated by 15 panelists capable of conducting organoleptic examinations of the surface-treated powders. Evaluations were performed by rubbing with balls of fingers and coating the powders on backs of hands and brachial regions. Evaluations were performed by absolute evaluations with each panelist, and their marks were defined as follows. A: Very good, B: good, C: ordinary, D: bad

### (Evaluation methods for water-repellency and hydrophilicity)

About 50cc of purified water is put into a 100cc glass beaker. About 0.1g of the treated powder is put on a water surface, which is stirred by a spatula at a rate of about 2 times per one second. Turbidity degrees of the water phase after stirring 50 times, 100 times and 150 times were evaluated according to 5-point scale.

### (Evaluation standard)

5 - No turbidity observed in the water phase (strong water repellency)
4 - Slight turbidity observed in the water phase
3 - Clear turbidity observed in the water
2 - Most powder moved into the water phase
1 - All powder moved into the water phase (Strong hydrophilicity)

### (Evaluation results)

Test results on contact angles of water and squalane were shown in columns of Examples and Comparative Examples of the respective treated powders. A list of evaluations was shown in Table 2-18 regarding test results on the usability and the adhesion.

### (Example 2-1 (FAW treated sericite) and Comparative Example 2-1)

A surface-treating amount as a solid content of each of the fluorine-containing copolymers shown in Table 2-1 was varied each time by 1 g from 1g (external percentage 1wt%) to 5g (external percentage 5wt%) with respect to 100g of Sericite FSE (Sanshin Mining Ind. Co., Ltd.). To each of the fluorine-containing copolymers was added 50g of water, which was kneaded for 30 minutes. The kneaded matter was dried at 80°Cfor 3 hours, and then further dried at 110 ° C for 10 hours. The sericite treated with each of the fluorine-containing copolymers was obtained by pulverization with the atomizer. Results were shown in Table 2-2 and Table 2-3.

### (Table 2-2)

**Table 2-2 (Results of Example 2-1 and Comparative Example 2-1) Contact angle tests on water/squalane of the sericite treated with each of the fluorine-containing copolymers**

| | 1% | 2% | 3% | 4% | 5% |
|---|---|---|---|---|---|
| Invention compound (a2) | 16°/P* | 50°/35° | 140°/130° | 72°/140° | P/145° |
| Invention compound (b2) | 18°/P | 48°/37° | 137°/133° | 64°/139° | P/142° |
| Invention compound (c2) | 15°/P | 55°/38° | 136°/122° | 75°/139° | P/140° |
| Compound in Comparative Example (d2) | 17°/P | 53°/42° | 137°/123° | 138°/135° | 145°/133° |
| Compound in Comparative Example (e2) | 20°/P | 83°/56° | 141°/128° | 143°/140° | 140°/142° |

| | | | | | |
|---|---|---|---|---|---|
| *P: Penetration | | | | | |

As shown in Table 2-2, in Comparative Examples, the water-repellent and oil-repellent properties increase in proportion to the treated amount. On the other hand, in the case of the powders coated with the fluorine-containing copolymers of the present invention, when the treated amount is not more than 2%, the water repellency and the oil repellency were low, whereas the highest water-repellent and oil-repellent properties were exhibited at the time of 3%. When the treated amount was 5%, the hydrophilic and oil-repellent properties appeared. Even when the treated amount was increased to over 5%, the oil-repellent property or the hydrophilic property did not change. Similar results were seen in those shown in the following Table 2-3.

### (Table 2-3)

**Table 2-3: Results of Example 2-1 and Comparative Example 2-1 Test results on the water repellency and hydrophilic property of the sericites treated with the fluorine-containing copolymers**

| | 1% | 2% | 3% | 4% | 5% |
|---|---|---|---|---|---|
| Invention compound (a2) | 1 | 3 | 4 | 2 | 1 |
| Invention compound (b2) | 1 | 3 | 4 | 2 | 1 |
| Invention compound (c2) | 1 | 3 | 4 | 2 | 1 |
| Compound in Compound Example (d2) | 1 | 3 | 4 | 5 | 5 |
| Compound in Comparative Example (e2) | 1 | 3 | 4 | 5 | 5 |

### (Example 2-2 (FAW treated sericite) and Comparative Example 2-2)

A surface-treating amount as a solid content of each of the fluorine-containing copolymers shown in Table 2-1 was varied each time by 1g from 1g (external percentage 1wt%) to 5g (external percentage 5wt%) with respect to 100g of Titanium CR-50 (Ishihara Sangyo Kaisha, Ltd.). Each of the fluorine-containing compounds was diluted with 10ml of water and added. After it was mixed with a mixer for 15 minutes, overheated steam at 250° C was introduced. When the interior of the mixer reached 200° C, stirring was stopped to obtain the titanium treated with each of the fluorine-containing copolymers. Results were shown in Table 2-4 and Table 2-5.

### (Table 2-4)

**Table 2-4: Results of Example 2-2 and Comparative Example 2-2 Contact angle tests of water/squalane of titanium treated with each of the fluorine-containing copolymers**

| | 1% | 2% | 3% | 4% | 5% |
|---|---|---|---|---|---|
| Invention compound (a2) | 16°/P* | 61°/35° | 140°/133° | 72°/141° | P/142° |
| Invention compound (b2) | 18°/P | 64°/37° | 143°/136° | 64°/140° | P/143° |
| Invention compound (c2) | 15°/P | 78°/38° | 141°/131° | 75°/137° | P/142° |
| Compound in Comparative Example (d2) | 20°/P | 89°/56° | 138°/135° | 147°/140° | 148°/142° |
| Compound in Comparative Example (e2) | 24°/P | 80°/42° | 137°/140° | 148°/142° | 143°/143° |

| | | | | | |
|---|---|---|---|---|---|
| * Penetration | | | | | |

As shown in Table 2-4, in the powders treated with fluorine-containing copolymers in Comparative Examples, the water-repellent and oil-repellent properties increase in proportion to the treated amount. On the other hand, in the powders coated with the fluorine-containing copolymers of the present invention, the water repellency and the oil repellency were low at the treated amount of not more than 2%, and the highest water-repellent and oil-repellent properties were exhibited at 3%. The hydrophilic and oil-repellent properties appeared at 5% of the treated amount. Even when the treated amount was increased to over 5%, the oil repellency or the hydrophilicity did not change. Similar results were also seen in results shown in Table 2-5.

### (Table 2-5)

**Table 2-5: Results of Example 2-2 and Comparative Example 2-2 Test Results on water repellency and hydrophilicity of titanium treated with each of the fluorine copolymers**

| | 1% | 2% | 3% | 4% | 5% |
|---|---|---|---|---|---|
| Invention compound (a2) | 1 | 2 | 4 | 2 | 1 |
| Invention compound (b2) | 1 | 3 | 5 | 3 | 1 |
| Invention compound (c2) | 1 | 2 | 4 | 2 | 1 |
| Compound in Comparative Example (d2) | 1 | 3 | 5 | 5 | 5 |
| Compound in Comparative Example (e2) | 1 | 3 | 5 | 5 | 5 |

### (Example 2-3 (FAW-treated yellow iron oxide) and Comparative Example 2-3)

A surface-treating amount as a solid content of each of the fluorine-containing copolymers shown in Table 2-1 was varied each time by 1g from 2g (external percentage 2wt%) to 6g (external percentage 6wt%) with respect to 100g of yellow iron oxide (Yellow LL-100P: Titan Kogyo, Ltd.). Each of the fluorine-containing compounds was diluted with 5ml of water and added. After it was mixed with a Henschel mixer for 15minutes and dried at 110°C for 10 hours, the yellow iron oxide treated with each of the fluorine-containing copolymers was obtained by pulverization with the atomizer. Results were shown in Table 2-6 and Table 2-7.

### (Table 2-6)

**Table 2-6: Results of Example 2-3 and Comparative Example 2-3 Contact angle tests of water/squalane of the yellow iron oxide treated with each of the fluorine-containing copolymers**

| | 2% | 3% | 4% | 5% | 6% |
|---|---|---|---|---|---|
| Invention compound (a2) | 16°/P* | 61°/35° | 132°/116° | 61°/141° | P/142° |
| Invention compound (b2) | 18°/P | 64°/37° | 131°/124° | 55°/140° | P/143° |
| Invention compound (c2) | 17°/P | 85°/42° | 130°/126° | 68°/138° | P/143° |
| Compound in Comparative Example (d2) | 20°/P | 89°/56° | 139°/135° | 148°/140° | 144°/142° |
| Compound in Comparative Example (e2) | 24°/P | 80°/42° | 138°/128° | 147°/142° | 145°/143° |

| | | | | | |
|---|---|---|---|---|---|
| * Penetration | | | | | |

As shown in Table 2-6, in the powders treated with fluorine-containing copolymers in Comparative Example, the water-repellent and oil-repellent properties increase in proportion to the treated amount. On the other hand, in the powders coated with the fluorine-containing copolymers of the present invention, the water repellency and the oil repellency were low at the treated amount of not more than 3%, and the highest water-repellent and oil-repellent properties were exhibited at 4%. The hydrophilic and oil-repellent properties appeared at 6% of the treated amount. Even when the treated amount was increased to not less than 6%, the oil repellency or the hydrophilicity did not change. Similar results were also seen in results shown in Table 2-7.

### (Table 2-7)

**Table 2-7: Results of Example 2-3 and Comparative Example 2-3 Test results on water repellency and hydrophilic property of yellow iron oxide treated with each of the fluorine-containing copolymers**

| | 2% | 3% | 4% | 5% | 6% |
|---|---|---|---|---|---|
| INVENTION COMPOUND (A2) | 1 | 2 | 4 | 2 | 1 |
| INVENTION COMPOUND (B2) | 1 | 2 | 5 | 3 | 1 |
| INVENTION COMPOUND (C2) | 1 | 2 | 4 | 2 | 1 |
| COMPOUND IN COMPARATIVE EXAMPLE (D2) | 1 | 3 | 5 | 5 | 5 |
| COMPOUND IN COMPARATIVE EXAMPLE (E2) | 1 | 3 | 5 | 5 | 5 |

### (Example 2-4 (FAW-treated red iron oxide) and Comparative Example 2-4)

A surface-treating amount as a solid content of each of the fluorine -containing copolymers shown in Table 2-1 was varied each time by 1g from 2g (external percentage 2wt%) to 6g (external percentage 6wt%) with respect to 100g of red iron oxide (Red R-516PS: Titan Kogyo, Ltd.). Red iron oxide treated with each of the fluorine-containing copolymers was obtained by the treatment in the same method as in the following Example 2-3. Results were shown in Table 2-8 and Table 2-9.

### (Table 2-8)

**Table 2-8: Results of Example 2-4 and Comparative Example 2-4 Contact angle tests on water/squalane of the red iron oxide treated with each of the fluorine-containing copolymers**

| | 2% | 3% | 4% | 5% | 6% |
|---|---|---|---|---|---|
| Invention compound (a2) | 18°/P* | 66°/35° | 140°/133° | 72°/141° | P/142° |
| Invention compound (b2) | 14°/P | 75°/38° | 141°/131° | 75°/137° | P/142° |
| Invention compound (c2) | 25°/P | 76°/42° | 141°/130° | 74°/138° | P/143° |
| Compound in Comparative Example (d2) | 26°/P | 80°/56° | 138°/135° | 143°/140° | 145°/142° |
| Compound in Comparative Example (e2) | 28°/P | 82°/42° | 137°/140° | 138°/142° | 143°/143° |

| | | | | | |
|---|---|---|---|---|---|
| * Penetration | | | | | |

As shown in Table 2-8, in the powders treated with fluorine-containing copolymers in Comparative Example, the water-repellent and oil-repellent properties increase in proportion to the treated amount. On the other hand, in the powders coated with the fluorine-containing copolymers of the present invention, the water repellency and the oil repellency were low at the treated amount of not more than 3%, and the highest water-repellent and oil-repellent properties were exhibited at 4%. The hydrophilic and oil-repellent properties appeared at 6% of the treated amount. Even when the treated amount was increased to over 6%, the oil repellency or the hydrophilicity did not change. Similar results were also seen in results shown in Table 2-9.

### (Table 2-9)

**Table 2-9: (Results of Example 2-4 and Comparative Example 2-4) Test results of water repellency and hydrophilicity of the red iron oxide treated with each of the fluorine-containing copolymers**

| | 2% | 3% | 4% | 5% | 6% |
|---|---|---|---|---|---|
| Invention compound (a2) | 1 | 3 | 4 | 2 | 1 |
| Invention compound (b2) | 1 | 3 | 4 | 3 | 1 |
| Invention compound (c2) | 1 | 2 | 4 | 2 | 1 |
| Compound in Comparative Example (d2) | 1 | 3 | 5 | 5 | 5 |
| Compound in Comparative Example (e2) | 1 | 3 | 5 | 5 | 5 |

### (Example 2-5 (FAW-treated black iron oxide) and Comparative Example 2-5)

A surface-treating amount as a solid content of each of the fluorine-containing copolymers shown in Table 2-1) was varied each time by 1g from 1g (external percentage 1wt%) to 5g (external percentage 5wt%) with respect to 100g of black iron oxide (Black BL-100P: Titan Kogyo, Ltd.). The black iron oxide treated with each of the fluorine-containing copolymers was obtained by the treatment in the same method as in the following Example 2-3. Results were shown in Table 2-10 and Table 2-11.

### (Table 2-10)

**Table 2-10: Results of Example 2-5 and Comparative Example 2-5 Tests on contact angles of water/squalane of the black iron oxide treated with each of the fluorine-containing copolymers**

| | 1% | 2% | 3% | 4% | 5% |
|---|---|---|---|---|---|
| Invention compound (a2) | 35°/P* | 134°/128° | 68°/133° | 12°/141° | P/142° |
| Invention compound (b2) | 40°/P | 136°/125° | 70°/136° | 13°/140° | P/143° |
| Invention compound (c2) | 45°/P | 138°/130° | 64°/138° | 15°/137° | P/142° |
| Compound in Comparrative Example (d2) | 68°/P | 140°/134° | 146°/145° | 145°/140° | 145°/142° |
| Compound in Comparative Example (e2) | 57°/P | 142°/133° | 144°/140° | 146°/142° | 143°/143° |

| | | | | | |
|---|---|---|---|---|---|
| * Penetration | | | | | |

As shown in Table 2-10, in the powders treated with fluorine-containing copolymers in Comparative Example, the water-repellent and oil-repellent properties increase in proportion to the treated amount. On the other hand, in the powders coated with the fluorine-containing copolymers of the present invention, the water repellency and the oil repellency were low at the treated amount of not more than 1%, and the highest water-repellent and oil-repellent properties were exhibited at 2%. The hydrophilic and oil-repellent properties were exhibited at 4% and 5% of the treated amount. Even when the treated amount was increased to over 5%, the oil repellency or the hydrophilicity did not change. Similar results were seen in results shown in Table 2-11.

### (Table 2-11)

**Table 2-11: (Results of Example 2-5 and Comparative Example 2-5) Test results of water repellency and hydrophilicity of the black iron oxide treated with each of the fluorine-containing copolymers**

| | 1% | 2% | 3% | 4% | 5% |
|---|---|---|---|---|---|
| Invention compound (a2) | 1 | 3 | 5 | 2 | 1 |
| Invention compound (b2) | 2 | 3 | 5 | 2 | 1 |
| Invention compound (c2) | 1 | 3 | 5 | 2 | 1 |
| Compound in Comparative Example (d2) | 2 | 3 | 5 | 5 | 5 |
| Compound in Comparative Example (e2) | 2 | 3 | 5 | 5 | 5 |

### (Example 2-6 (FAW treated finely particulate titanium oxide) and Comparative Example 2-6)

Fine particles of titanium oxide (MT-500SA: TAYCA Corporation), 100g, was put into 1500ml of deionized water (containing 0.05g of sodium hexamethaphosphate), and each of fluorine-containing compounds which was diluted at a solid content of 6g with 500ml IPA was added thereinto. The mixture was dispersed under circulation for 15 minutes with a sand grinder (DYNO-Mill: 1.4L Zirconia Vessel & Blade, 0.5mm in diameter zirconia beads at a packed rate 85%). The dispersion liquid was heated to 80°C under stirring, and a 10% HCl aqueous solution wad added dropwise to adjust pH at 4.5. After the resultant was dewatered by centrifugal separation, the residue was dried at 120°C for 16 hours, and pulverized with a JET atomizer, thereby obtaining the finely particulate titanium oxide treated with each of the fluorine-containing compounds. Further, surface-treated powders in which the amount of each of the fluoride-containing copolymer was varied each by 1% from 6% to 10% were prepared in trial. Results were shown in Table 2-12 and Table 2-13.

### (Table 2-12)

**Table 2-12: Results of Example 2-6 and Comparative Example 2-6 Tests on contact angles of water/squalane of the finely particulate iron oxide treated with each of the fluorine-containing copolymers**

| | 6% | 7% | 8% | 9% | 10% |
|---|---|---|---|---|---|
| Invention compound (a2) | 35°/P* | 134°/128° | 88°/133° | 29°/141° | P/142° |
| Invention compound (b2) | 40°/P | 136°/125° | 80°/136° | 25°/143° | P/143° |
| Invention compound (c2) | 45°/P | 138°/130° | 94°/138° | 25°/139° | P/142° |
| Compound in Comparative Example (d2) | 68°/P | 140°/134° | 146°/145° | 145°/140° | 145°/142° |
| Compound in Comparative Example (e2) | 57°/P | 142°/133° | 144°/140° | 146°/142° | 143°/143° |

| | | | | | |
|---|---|---|---|---|---|
| * Penetration | | | | | |

As shown in Table 2-12, in the powders treated with fluorine-containing copolymers in Comparative Example, the water-repellent and oil-repellent properties increase in proportion to the treated amount. On the other hand, in the powders coated with the fluorine-containing copolymers of the present invention, the water repellency and the oil repellency were low at the treated amount of not more than 6%, and the highest water-repellent and oil-repellent properties were exhibited at 7%. The hydrophilic and oil-repellent properties were exhibited at 10% of the treated amount. Even when the treated amount was increased to over 10%, the oil repellency or the hydrophilicity did not change. Similar results were also seen in results shown in Table 2-13.

### (Table 2-13)

**Table 2-13: Results of Example 2-6 and Comparative Example 2-6 Test results of water repellency and hydrophilicity of the finely particulate iron oxide treated with each of the fluorine-containing copolymers**

| | 6% | 7% | 8% | 9% | 10% |
|---|---|---|---|---|---|
| Invention compound (a2) | 2 | 5 | 4 | 2 | 1 |
| Invention compound (b2) | 2 | 5 | 4 | 2 | 1 |
| Invention compound (c2) | 2 | 5 | 4 | 2 | 1 |
| Compound in Comparative Example (d2) | 3 | 5 | 5 | 5 | 5 |
| Compound in Comparative Example (e2) | 3 | 5 | 5 | 5 | 5 |

### (Example 2-7 (FAW treated/SA treated finely particulate zinc oxide) and Comparative Example 2-7)

Into 20g of water and 50g of IPA were added 100g of finely particulate zinc oxide (MZ-300: TAYCA Corporation), each of 5g to 9g as a solid content of each of the fluorine-containing copolymers shown in Table 2-1, 3g of a straight-chain dimethyl polysiloxane (SA treated) having a triethoxy group at one end and a polymerization degree of 15, which was kneaded for 30 minutes. Further, 10g of deionized water was added thereto, which was kneaded for 30 minutes and dried at 105°C for 16 hours. Thereafter, the resultant was pulverized with a JET atomizer, thereby obtaining the finely particulate zinc oxide treated in complex with each of the fluorine-containing copolymers and the silicone. Results were shown in Table 2-14 and Table 2-15.

### (Table 2-14)

**Table 2-14: Results of Example 2-7 and Comparative Example 2-7 Tests on contact angles of water/squalane of the finely particulate iron oxide treated with each of the fluorine-containing copolymers**

| | 5% | 6% | 7% | 8% | 9% |
|---|---|---|---|---|---|
| Invention compound (a2) | 75°/25° | 144°/135° | 98°/133° | 51°/141° | P*/142° |
| Invention compound (b2) | 85°/26° | 148°/136° | 102°/138° | 62°/137° | P/142° |
| Invention compound (c2) | 77°/27° | 145°/137° | 87°/137° | 54°/138° | P/143° |
| Compound in Comparative Example (d2) | 88°/30° | 140°/140° | 146°/145° | 145°/140° | 145°/142° |
| Compound in Comparative Example (e2) | 97°/38° | 142°/142° | 144°/140° | 146°/142° | 143°/143° |

As shown in Table 2-14, in the powders treated with fluorine-containing copolymers in Comparative Example, the water-repellent and oil-repellent properties increase in proportion to the treated amount. On the other hand, in the powders coated with the fluorine-containing copolymers of the present invention, the water repellency and the oil repellency were low at the treated amount of not more than 5%, and the highest water-repellent and oil-repellent properties were exhibited at 6%. The hydrophilic and oil-repellent properties were exhibited at 9% of the treated amount. Even when the treated amount was increased to not less than 9%, the oil repellency or the hydrophilicity did not change. Similar results were also seen in results shown in Table 2-15.

### (Table 2-15)

**Table 2-15: Results of Example 2-7 and Comparative Example 2-7 Test results of water repellency and hydrophilicity of the finely particulate zinc oxide treated with each of the fluorine-containing polymers**

| | 5% | 6% | 7% | 8% | 9% |
|---|---|---|---|---|---|
| Invention compound (a2) | 3 | 5 | 3 | 2 | 1 |
| Invention compound (b2) | 3 | 5 | 3 | 2 | 1 |
| Invention compound (c2) | 3 | 5 | 3 | 2 | 1 |
| Compound in Comparative Example (d2) | 4 | 5 | 5 | 5 | 5 |
| Compound in Comparative Example (e2) | 4 | 5 | 5 | 5 | 5 |

### (Example 2-8 (FAW treated/NAI treated pearls) and Comparative Example 2-8)

Into 1000ml of deionized water was added 100g of a pearl pigment (Flamenco Gold: Angel Heart Co., Ltd.) and then added 1.0g of disodium N-stearoyl glutamate (Ajinomoto Co., Inc.: Amino soft HS-21P) (NAI treated), which is dissolved and dispersed. Into the resultant was added each of the fluorine-containing copolymer shown in Table 2-1 in an amount of from 1g to 5g, which was heated to 80°C and further kneaded with a kneader for 30 minutes. Then, 0.5mol/L of an aqueous solution of A12 (S04)3 was added to adjust pH of the mixture to 5.0. The resultant was dried at 140°C for 8 hours, thereby obtaining the pearl pigment treated in complex with each of the fluorine-containing copolymers and the acylated amino acid. Results were shown in Table 2-16 and Table 2-17.

### (Table 2-16)

**Table 2-16: Results of Example 2-8 and Comparative Example 2-8 Tests on contact angles of water/squalane of the fine pearl treated with each of the fluorine-containing copolymers**

| | 1% | 2% | 3% | 4% | 5% |
|---|---|---|---|---|---|
| Invention compound (a2) | 50°/15° | 144°/136° | 98°/133° | 51°/137° | P*/42° |
| Invention compound (b2) | 46°/10° | 146°/132° | 90°/136° | 45°/141° | P/143° |
| Invention compound (c2) | 40°/16° | 148°/133° | 102°/138° | 52°/140° | P/142° |
| Compound in Comparative Example (d2) | 88°/30° | 148°/145° | 149°/146° | 145°143° | 145°/142° |
| Compound in Comparative Example (e2) | 97°/38° | 148°/142° | 146°/141° | 146°/142° | 143°/143° |

| | | | | | |
|---|---|---|---|---|---|
| * Penetration | | | | | |

As shown in Table 2-16, in the powders treated with fluorine-containing copolymers in Comparative Example, the water-repellent and oil-repellent properties increase in proportion to the treated amount. On the other hand, in the powders coated with the fluorine-containing copolymers of the present invention, the water repellency and the oil repellency were low at the treated amount of not more than 1%, and the highest water-repellent and oil-repellent properties were exhibited at 2%. The hydrophilic and oil-repellent properties appeared at 5% of the treated amount. Even when the treated amount was increased to over 5%, the oil repellency or the hydrophilicity did not change. Similar results were also seen in results shown in Table 2-17.

### (Table 2-17)

**Table 2-17: Results of Example 2-8 and Comparative Example 2-8 Test results of water repellency and hydrophilicity of the fine pearl treated with each of the fluorine-containing copolymers**

| | 1% | 2% | 3% | 4% | 5% |
|---|---|---|---|---|---|
| Invention compound (a2) | 2 | 5 | 3 | 2 | 1 |
| Invention compound (b2) | 2 | 5 | 3 | 2 | 1 |
| Invention compound (c2) | 2 | 5 | 3 | 2 | 1 |
| Compound in Comparative Example (d2) | 3 | 5 | 5 | 5 | 5 |
| Compound in Comparative Example (e2) | 3 | 5 | 5 | 5 | 5 |

### (Example 2-9 (FAW-treated lauroyl lysin) and Comparative Example 2-9)

Into 100g of Amihope LL (lauroyl lysin: Ajinomoto Co., Inc.) was added a mixed liquid of an aqueous IPA solution (= 15g/10g) of which pH was adjusted to pH= about 8.0 with NaOH and further added with 1g to 5g of each of the fluorine-containing copolymers shown in Table 2-1. Surface treatment was carried out in the same manner as Example 2-8 and Comparative Example 2-8, thereby obtaining Amihope LL treated with each of the fluorine-containing compounds. Results were shown in Table 2-18 and Table 2-19.

### (Table 2-18)

**Table 2-18: Results of Example 2-9 and Comparative Example 2-9 Tests on contact angles of water/squalane of the lauroyl lysin treated with each of the fluorine-containing copolymers**

| | 1% | 2% | 3% | 4% | 5% |
|---|---|---|---|---|---|
| Invention compound (a2) | 75°/25° | 144°/135° | 98°/133° | 51°/141° | P*142° |
| Invention compound (b2) | 85°/36° | 148°/136° | 102°/138° | 62°/137° | P/142° |
| Invention compound (c2) | 77°/27° | 145°/137° | 87°/137° | 54°/138° | P/143° |
| Compound in Comparative Example (d2) | 88°/30° | 140°/140° | 146°/145° | 145°/140° | 145°/142° |
| Compound in Comparative Example (e2) | 97°/38° | 142°/142° | 144°/140° | 146°/142° | 143°/l43° |

| | | | | | |
|---|---|---|---|---|---|
| * Penetration | | | | | |

As shown in Table 2-18, in the powders treated with the fluorine-containing copolymers in Comparative Example, the water-repellent and oil-repellent properties increase in proportion to the treated amount. On the other hand, in the powders coated with the fluorine-containing copolymers of the present invention, the water repellency and the oil repellency were low at the treated amount of not more than 1%, and the highest water-repellent and oil-repellent properties were exhibited at 2%. The hydrophilic and oil-repellent properties appeared at 5% of the treated amount. Even when the treated amount was increased to not less than 5%, the oil repellency or the hydrophilicity did not change. Similar results were also seen in results shown in Table 2-19.

### (Table 2-19)

**Table 2-19: Results of Example 2-9 and Comparative Example 2-9 Test results of water repellency and hydrophilicity of the lauroyl lysin treated with each of the fluorine-containing copolymers**

| | 1% | 2% | 3% | 4% | 5% |
|---|---|---|---|---|---|
| Invention compound (a2) | 4 | 5 | 3 | 2 | 1 |
| Invention compound (b2) | 4 | 5 | 3 | 2 | 1 |
| Invention compound (c2) | 4 | 5 | 3 | 2 | 1 |
| Compound in Comparative Example (d2) | 4 | 5 | 5 | 5 | 5 |
| Compound in Comparative Example (e2) | 4 | 5 | 5 | 5 | 5 |

### (Example 2-10 (FAW treated Red No. 202) and Comparative Example 2-10)

Into a mixed solution of IPA/deionized water = 40g/100g was put 100g of Red No. 202 ((Kishi Kasei Co., Ltd.), which was dispersed with an ultrasonic homogenizer (Japan Siber Hegner & Co.), while being stirred with a propeller stirrer. After 5g to 9g of each of the fluorine-containing treating agents shown in Table 2-1 and 5g of IPA were dropwise added thereto and the mixture was aged, the solvent was distilled off by heating under vacuum, and the resultant was dried and pulverized at 105°C for 16 hours, thereby obtaining the Red No. 202 treated with each of the fluorine-containing compounds. Results were shown in Table 2-20 and Table 2-21.

### (Table 2-20)

**Table 2-20: Results of Example 2-10 and Comparative Example 2-10 Tests on contact angles of water/squalane of the Red No. 202 treated with each of the fluorine-containing copolymers**

| | 5% | 6% | 7% | 8% | 9% |
|---|---|---|---|---|---|
| Invention compound (a2) | 75°/25° | 144°/135° | 98°/133° | 51°/141° | P*/142° |
| Invention compound (b2) | 85°/36° | 148°/136° | 102°/138° | 62°/137° | P/142° |
| Invention compound (c2) | 77°/27° | 145°/137° | 87°/137° | 54°/138° | P/143° |
| Compound in Comparative Example (d2) | 88°/30° | 140°/140° | 146°/145° | 145°/140° | 145°/142° |
| Compound in Comparative Example (e2) | 97°/38° | 142°/142° | 144°/140° | 146°/142° | 143°/143° |

| | | | | | |
|---|---|---|---|---|---|
| * Penetration | | | | | |

As shown in Table 2-20, in the powders treated with fluorine-containing copolymers in Comparative Example, the water-repellent and oil-repellent properties increase in proportion to the treated amount. On the other hand, in the powders coated with the fluorine-containing copolymers of the present invention, the water repellency and the oil repellency were low at the treated amount of not more than 5%, and the highest water-repellent and oil-repellent properties were exhibited at 6%. The hydrophilic and oil-repellent properties appeared at 9% of the treated amount. Even when the treated amount was increased to not less than 9%, the oil repellency or the hydrophilicity did not change. Similar results were also seen in results shown in Table 2-21.

### (Table 2-21)

**Table 2-21: Results of Example 2-10 and Comparative Example 2-10 Test results of water repellency and hydrophilicity of the Red No. 202 treated with each of the fluorine-containing copolymers**

| | 5% | 6% | 7% | 8% | 9% |
|---|---|---|---|---|---|
| Invention compound (a2) | 3 | 5 | 4 | 3 | 1 |
| Invention compound (b2) | 3 | 5 | 4 | 3 | 1 |
| Invention compound (c2) | 3 | 5 | 4 | 3 | 1 |
| Compound in Comparative Example (d2) | 4 | 5 | 5 | 5 | 5 |
| Compound in Comparative Example (e2) | 4 | 5 | 5 | 5 | 5 |

### (Example 2-11 (FAW treatment/ALS treatment methicone/mixture of vinyl dimethicone and titanium oxide) and Comparative Example 2-11)

Into a high speed mixer was charged 100g of a mixture of dimethicone/vinyl dimethicone and titanium oxide (Dow Corning Corporation EP-9261 TI Cosmetic Powder), to which are added 1g to 5g as a solid content of each of the fluorine-containing copolymers shown in Table 2-1 and a liquid in which 1g of triethoxy capryl silane (Dow Corning Corporation: Z-6341) (ALS treatment) is dissolved in 10g of IPA (isopropyl alcohol), followed by stirring for 5 minutes. While stirring was being maintained, the interior of the chamber was reduced in pressure, it was heated to 50°C and stirred for 15 minutes. The obtained powder was pulverized with the atomizer, thereby obtaining a mixed powder of dimethicone/vinyl dimethicone and titanium oxide treated in complex with each of the fluorine-containing polymer and the alkyl silane. Results were shown in Table 2-22 and Table 2-23.

### (Table 2-22)

**Table 2-22: Results of Example 2-11 and Comparative Example 2-11 Tests on contact angles of water/squalane of the mixture of methicone/vinyl dimethicone and titanium oxide treated with each of the fluorine-containing polymers**

| | 1% | 2% | 3% | 4% | 5% |
|---|---|---|---|---|---|
| Invention compound (a2) | 45°/P* | 94°/65° | 145°/133° | 51°/141° | P*140° |
| Invention compound (b2) | 41°/P | 98°/66° | 148°/138° | 62°/137° | P/141° |
| Invention compound (c2) | 42°/P | 85°/57° | 145°/137° | 54°/138° | P/138° |
| Compound in Compative Example (d2) | 58°/P | 90°/70° | 146°/145° | 144°/140° | 142°/142° |
| Compound in Comparative Example (e2) | 67°/P | 102°/82° | 144°/140° | 146°/142° | 143°/143° |

| | | | | | |
|---|---|---|---|---|---|
| * Penetration | | | | | |

As shown in Table 2-22, in the powders treated with fluorine-containing copolymers in Comparative Example, the water-repellent and oil-repellent properties increase in proportion to the treated amount. On the other hand, in the powders coated with the fluorine-containing copolymers of the present invention, the water repellency and the oil repellency were low at the treated amount of not more than 2%, and the highest water-repellent and oil-repellent properties were exhibited at 3%. The hydrophilic and oil-repellent properties appeared at 5% of the treated amount. Even when the treated amount was increased to not less than 5%, the oil repellency or the hydrophilicity did not change. Similar results were also seen in results shown in Table 2-23.

### (Table 2-23)

**Table 2-23: Results of Example 2-11 and Comparative Example 2-11 Test results of water repellency and hydrophilicity of the mixture of methicone/vinyl dimethicone and titanium oxide treated with each of the fluorine-containing polymers**

| | 1% | 2% | 3% | 4% | 5% |
|---|---|---|---|---|---|
| Inwention compound (a2) | 2 | 3 | 4 | 2 | 1 |
| Invention compound (b2) | 2 | 3 | 5 | 2 | 1 |
| Invention compound (c2) | 2 | 3 | 5 | 2 | 1 |
| Compound in Comparative Example (d2) | 2 | 3 | 5 | 5 | 5 |
| Compound in Comparative Example (e2) | 2 | 3 | 5 | 5 | 5 |

### (Example 2-12 (FAW treated/C6F silane- treated finely particulate zinc oxide) and Comparative Example 2-12)

Finely particulate zinc oxide (MZ-300: TAYCA Corporation), 100g, and 3g to 6g as a solid content of each of the fluorine-containing copolymers shown in Table 2-1 and 5g of tridecafluoro octyl triethoxy silane (DYNASYLAN F-8261; Evoik Degussa GmbH.) (C6F silane treatment) were added to 60g of a liquid of water/IPA=1/1. The resultant was kneaded in a kneader for 60 minutes and dried at 130 ° C for 16 hours. The finely particulate zinc oxide treated in complex with each of the fluorine-containing copolymer and the C6F silane was obtained by pulverizing with the atomizer. Results were shown in Table 2-24 and Table 2-25.

### (Table 2-24)

**Table 2-24: Results of Example 2-12 and Comparative Example 2-12 Tests on contact angles of water/squalane of the finely particulate zinc oxide treated with each of the fluorine-containing polymers**

| | 3% | 4% | 5% | 6% | 7% |
|---|---|---|---|---|---|
| Invention compound (a2) | 149°/144° | 108°/145° | 89°/145° | 41°/150° | p*/141° |
| Invention compound (b2) | 150°/146° | 102°/143° | 78°/147° | 32°/148° | p/143° |
| Invention compound (c2) | 158°/147° | 110°/142° | 80°/140° | 34°/140° | p/140° |
| Compound in Comparative Example (d2) | 154°/149° | 154°/146° | 146°/145° | 144°/140° | 142°/142° |
| Compound in Comparative Example (e2) | 156°/146° | 157°/145° | 144°/145° | 146°/142° | 143°/143° |

| | | | | | |
|---|---|---|---|---|---|
| * Penetration | | | | | |

As shown in Table. 2-24, even when the treating amount is increased in the case of the powders treated with the fluoride-containing copolymers in Comparative Examples, the water-repellent and oil-repellent properties does not change. However, the powders coated with the fluoride-containing copolymers according to the present invention, the water-repellent property and the oil-repellent property are high at not more than 3% of the treated amount of the fluoride-containing copolymer. The highest water-repellent and oil-repellent properties appeared at 5% in the treatment with the C6F silane and 3% in the case of the fluid-containing copolymer. When the treated amount of the fluoride-containing copolymer was 7%, the hydrophilic and oil-repellent properties were exhibited. Even when the treated amount was increased to not less than 7%, the oil-repellency or the hydrophilic property did not change. Similar result were also seen in the following Table 2-25.

### (Table 2-25)

**Table 2-25: Results of Example 2-12 and Comparative Example 2-12 Test results on water repellency and hydrophilicity of the finely particulate zinc oxide treated with each of the fluorine-containing copolymers**

| | 3% | 4% | 5% | 6% | 7% |
|---|---|---|---|---|---|
| Invention compound (a2) | 5 | 4 | 3 | 2 | 1 |
| Invention compound (b2) | 5 | 4 | 3 | 2 | 1 |
| Invention compound (c2) | 5 | 4 | 3 | 2 | 1 |
| Compound in Comparative Example (d2) | 5 | 5 | 5 | 5 | 5 |
| Compound in Comparative Example (e2) | 5 | 5 | 5 | 5 | 5 |

### (Example 2-13 (FAW treated sericite) and Comparative Example 2-13)

Sericite treated with a fluorine-containing copolymer was obtained by the same powder and method as in Example 2-1 except that 50g of the mixed solution of IPA and water (50:50wt%) was adjusted to pH8.5 with NaOH. Results were shown in Table 2-26 and Table 2-27.

### (Table 2-26)

**Table 2-26: Results of Example 2-13 and Comparative Example 2-13 Tests on contact angles of water/squalane of the sericite treated with each of fluorine-containing copolymers**

| | 1% | 2% | 3% | 4% | 5% |
|---|---|---|---|---|---|
| Invention compound (a2) | 26°/P* | 30°/45° | P/140° | P/140° | P/145° |
| Invention compound (b2) | 28°/P | 38°/42° | P/141° | P/139° | P/142° |
| Invention compound (c2) | 25°/P | 35°/40° | P/140° | P/139° | P/140° |
| Compound in Comparative Example (d2) | 28°/P | 53°/52° | 101°/143° | 138°/145° | 145°/143° |
| Compound in Comparative Example (e2) | 25°/P | 43°/59° | 105°/145° | 143°/142° | 140°/142° |

| | | | | | |
|---|---|---|---|---|---|
| * Penetration | | | | | |

As shown in Table 2-26, in the powders treated with fluorine-containing copolymers in Comparative Example, the water-repellent and oil-repellent properties increased in proportion to the treated amount. On the other hand, in the powders coated with the fluorine-containing copolymers of the present invention, the water repellency and the oil repellency were low at the treated amount of not more than 2%, and the high water-repellent and oil-repellent properties were exhibited at not less than 3%. Even when the treated amount was increased to not less than 5%, the oil repellency or the hydrophilicity did not change. Similar results were also seen in results shown in Table 2-27.

### (Table 2-27)

**Table 2-27: Results of Example 2-13 and Comparative Example 2-13 Test results on water repellency and hydrophilicity of the sericite treated with each of fluorine-containing copolymers**

| | 1% | 2% | 3% | 4% | 5% |
|---|---|---|---|---|---|
| Invention compound (a2) | 2 | 2 | 1 | 1 | 1 |
| Invention compound (b2) | 2 | 2 | 1 | 1 | 1 |
| Invention compound (c2) | 2 | 2 | 1 | 1 | 1 |
| Compound in Comparative Example (d2) | 2 | 3 | 4 | 5 | 5 |
| Compound in Comparative Example (e2) | 2 | 3 | 4 | 5 | 5 |

### (Example 2-14 (FAW treated talc) and Comparative Example 2-14)

Into 500g of water prepared in a 2L glass beaker is put 100g of Talk JA-13R (Asada Milling Co., Ltd.), which is fully stirred and dispersed. After a liquid in which 1g to 5g as a solid content of each of the fluorine-containing copolymers shown in Table 2-1 and 10g of IPA were dissolved is gradually added thereto, which is then stirred for 15 minutes. The thus obtained slurry is fed to a spray dryer equipped with a twin-fluid spray nozzle. The temperature of hot air at an inlet of the spray dryer is set at 200°C, and a spraying pressure of the twin-fluid nozzle is set at 0.6MPa. In this matter, a talc treated with each of fluoride-containing compounds was obtained. Results were shown in Table 2-28 and Table 2-29.

### (Table 2-28)

**Table 2-28: Results of Example 2-14 and Comparative Example 2-14 Tests on contact angles of water/squalane of the talc treated with each of fluorine-containing copolymers**

| | 1% | 2% | 3% | 4% | 5% |
|---|---|---|---|---|---|
| Invention compound (a2) | 29°/P* | 48°/65° | 55°/95° | 61°/100° | P/101° |
| Invention compound (b2) | 30°/P | 42°/63° | 67°/87° | 72°/108° | P/103° |
| Invention compound (c2) | 38°/P | 40°/62° | 58°/86° | 64°/100° | P/103° |
| Compound in Comparative Example (d2) | 44°/P | 94°/76° | 143°/97° | 144°/110° | 142°/112° |
| Compound in Comparative Example (e2) | 56°/P | 97°/75° | 144°/95° | 146°/112° | 143°/113° |

| | | | | | |
|---|---|---|---|---|---|
| * Penetration | | | | | |

As shown in Table 2-28, in the powders treated with fluorine-containing copolymers in Comparative Example, the water-repellent and oil-repellent properties increase in proportion to the treated amount. On the other hand, in the powders coated with the fluorine-containing copolymers of the present invention, the water repellency and the oil repellency were low at the treated amount of not more than 2%, and the highest water-repellent and oil-repellent properties were exhibited at 3%. The hydrophilic and oil-repellent properties appeared at 5% of the treated amount. Even when the treated amount was increased to not less than 5%, the oil repellency or the hydrophilicity did not change. Similar results were also seen in results shown in Table 2-29.

### (Table 2-29)

**Table 2-29: Results of Example 2-14 and Comparative Example 2-14 Test results on water repellency and hydrophilicity of the talc treated with each of the fluorine-containing copolymers**

| | 1% | 2% | 3% | 4% | 5% |
|---|---|---|---|---|---|
| Invention compound (a2) | 2 | 2 | 3 | 3 | 1 |
| Invention compound (b2) | 2 | 2 | 3 | 3 | 1 |
| Invention compound (c2) | 2 | 2 | 3 | 3 | 1 |
| Compound in Comparative Example (d2) | 2 | 3 | 5 | 5 | 5 |
| Compound in Comparative Example (e2) | 2 | 3 | 5 | 5 | 5 |

### (Example 2-15 (FAW treated finely particulate titanium oxide) and Comparative Example 2-15)

Crystals of titanyl sulfate were put into water, which was heated to produce a hydrolysate. To 350g of a cake of the hydrous titanium hydroxide (the content of titanium oxide: 100g) obtained by filtering and washing the hydrolysate was added 400g of a 48% aqueous solution of sodium hydroxide under stirring, which was heated and stirred for 2 hours in a range of 95 to 105 ° C. Next, a suspension of this hydrate of the titanium dioxide was filtered and washed. A slurry was obtained by adding 500g of water to the washed cake, 140g of 35% hydrochloric acid was further added thereto, while being stirred, and the resultant was aged under heating at 95°C for 2 hours. The concentration of the thus obtained aqueous suspension liquid of the finely particulate titanium oxide was adjusted to 70g/L. To this aqueous suspension liquid was added polyaluminum chloride (10 wt% relative to the finely particulate titanium oxide when calculated as A1203), and then H was adjusted to 5.0 by using caustic soda, followed by aging for 30 minutes. After the aging, 6 to 10 wt% as a solid content of each of the fluorine-containing copolymers shown in Table 2-1 was added to the finely particulate titanium oxide, followed by aging for 30 minutes. The resultant was filtered, washed, dried, and pulverized by an Eck atomizer. In this manner, the titanium oxide treated with each of the fluorine-containing compounds was obtained. Results were shown in Table 2-30 and Table 2-31.

### (Table 2-30)

**Table 2-30: Results of Example 2-15 and Comparative Example Tests on contact angles of water/squalane of the finely particulate titanium oxide treated with each of the fluorine-containing copolymers**

| | 6% | 7% | 8% | 9% | 10% |
|---|---|---|---|---|---|
| Invention compound (a2) | 75°/35° | 144°/135° | 98°/133° | 51°/141° | P*/142° |
| Invention compound (b2) | 85°/46° | 148°/136° | 102°/138° | 62°/137° | P/142° |
| Invention compound (c2) | 77°/37° | 145°/137° | 87°/137° | 54°/138° | P/143° |
| Compound in Comparative Example (d2) | 88°/40° | 140°/140° | 146°/145° | 145°/140° | 145°/142° |
| Compound in Comparative Example (e2) | 97°/48° | 142°/142° | 144°/140° | 146°/142° | 143°/143° |

| | | | | | |
|---|---|---|---|---|---|
| * Penetration | | | | | |

As shown in Table 2-30, in the powders treated with fluorine-containing copolymers in Comparative Example, the water-repellent and oil-repellent properties increase in proportion to the treated amount. On the other hand, in the powders coated with the fluorine-containing copolymers of the present invention, the water repellency and the oil repellency were low at the treated amount of not more than 6%, and the highest water-repellent and oil-repellent properties were exhibited at 7%. The hydrophilic and oil-repellent properties appeared at 10% of the treated amount. Even when the treated amount was increased to not less than 10%, the oil repellency or the hydrophilicity did not change. Similar results were also seen in results shown in Table 2-31.

### (Table 2-31)

**Table 2-31: Results of Example 2-15 and Comparative Example 2-15 Test results on water repellency and hydrophilicity of the finely particulate titanium oxide treated with each of the fluorine-containing copolymers**

| | 6% | 7% | 8% | 9% | 10% |
|---|---|---|---|---|---|
| Invention compound (a2) | 3 | 5 | 3 | 2 | 1 |
| Invention compound (b2) | 3 | 5 | 3 | 2 | 1 |
| Invention compound (c2) | 3 | 5 | 3 | 2 | 1 |
| Compound in Comparative Example (d2) | 4 | 5 | 5 | 5 | 5 |
| Compound in Comparative Example (e2) | 4 | 5 | 5 | 5 | 5 |

A list of Examples 2-1 to 2-15 and Comparative Example 2-1 to 2-15 were shown in Table 2-32.

### (Table 2-32)

**Table 2-32 A list of Examples and Comparative Examples**

| Powder to be surface-treated | Compound (a2) | Compound (b2) | Compound (c2) | Compound (d2) | Compound(e2) |
|---|---|---|---|---|---|
| Sericite FSE | Example 2-1 | Example 2-1 | Example 2-1 | Comparative Example 2-1 | Comparative Example2-1 |
| Titanium CR-50 | Example 2-2 | Example 2-2 | Example 2-2 | Comparative Example 2-2 | Comparative Example2-2 |
| Yellow - LL-100P | Example 2-3 | Example 2-3 | Example 2-3 | Comparative Example 2-3 | Comparative Example2-3 |
| Red R -516PS | Example 2-4 | Example 2-4 | Example 2-4 | Comparative Example 2-4 | Comparative Example2-4 |
| BlackBL-100P | Example 2-5 | Example 2-5 | Example 2-5 | Comparative Example 2-5 | Comparative Example2-5 |
| Finely particulate titanium oxide | Example 2-6 | Example 2-6 | Example 2-6 | Comparative Example 2-6 | Comparative Example2-6 |
| Finely particulate zinc oxide | Example 2-7 | Example 2-7 | Example 2-7 | Comparative Example2-7 | Comparative Example2-7 |
| Pearl pigment | Example 2-8 | Example 2-8 | Example 2-8 | Comparative Example2-8 | Comparative Example2-8 |
| AmihopeLL | Example 2-9 | Example 2-9 | Example 2-9 | Comparative Example2-9 | Comparative Example2-9 |
| Red No. 202 | Example 2-10 | Example 2-10 | Example 2-10 | Comparative Example2-10 | Comparative Example2-10 |
| Mixture of methicone/vinyl dimethicone and titanium oxide | Example 2-11 | Example 2-11 | Example 2-11 | Comparative Example2-11 | Comparative Example 2-11 |
| Finely particulate zinc oxide | Example 2-12 | Example 2-12 | Example 2-12 | Comparative Example2-12 | Comparative Example2-12 |
| SericiteFSE | Example 2-13 | Example 2-13 | Example 2-13 | Comparative Example2-13 | Comparative Example2-13 |
| Talc JA-13R | Example 2-14 | Example 2-14 | Example 2-14 | Comparative Example2-14 | Comparative Example2-14 |
| Finely particulate titanium oxide | Example 2-15 | Example 2-15 | Example 2-15 | Comparative Example2-15 | Comparative Example2-15 |

In Examples, the treated powders were evaluated by the minimum coated amount which gave the hydrophilic and oil-repellent properties. In Comparative Examples, the powders coated in the same treating amount as in the hydrophilic and oil-repellent powder in Example were used.

### (Table 2-33)

**Table 2-33: Test results on usability/adhesion**

| Powder to be surface-treated | Compound (a2) | Compound (b2) | Compound (c2) | Compound (d2) | Compound (e2) |
|---|---|---|---|---|---|
| Sericite FSE | B/A | B/A | B/A | C/C | D/D |
| Titanium CR-50 | B/A | B/A | B/A | C/C | D/D |
| Yellow - LL-100P | A/A | A/A | A/A | B/D | C/D |
| RedR-516PS | B/A | B/A | B/A | C/D | D/D |
| BlackBL-100P | B/A | B/A | B/A | C/D | D/D |
| Finely particulate titanium oxide | B/A | B/A | B/A | C/D | D/D |
| Finely particulate zinc oxide | B/A | B/A | B/A | C/D | D/D |
| Pearl pigment | B/A | B/A | B/A | B/C | D/D |
| AmihopeLL | A/A | A/A | A/A | B/D | C/D |
| Red No. 202 | A/A | A/A | A/A | B/D | C/D |
| Mixture of methicone/vinyl dimethicone and titanium oxide | B/A | B/A | B/A | C/C | D/D |
| Finely particulate zinc oxide | A/A | A/A | A/A | C/C | D/D |
| SericiteFSE | A/A | A/A | A/A | C/C | D/D |
| Talc JA-13R | B/A | B/A | B/A | C/C | D/D |
| Finely particulate titanium oxide | B/A | B/A | B/A | C/D | D/D |

### (Verification of moisture-retaining property due to an effect of suppressing moisture)

After the surface-treated powder of the present invention was coated onto a skin, whether or not there was an effect of suppressing moisture loss from the skin due to the possession of the moisturizing effect was verified by the following method. As a sample, the treated powders tested with respect to the usability and the adhesion were used. Test method: A 10% solution of sodium lauryl sulfate was applied in a closed fashion onto to forearm flexor sides of 30 panelists having sound skins for 2 hours, thereby forming skin disorder models. As a sample to be applied, a mixture of vaseline (Nikko Rica Corporation): coating powder = 1:1 wt% was prepared. As a comparative sample, a powder before coating in each Example was tested as a non-coated powder. Further, vaseline only was applied as a blank. A face of 2cm x 4cm to be applied was defined in a skin disordered portion, the sample was applied under a requirement of 2mg/cm2. This operation was performed twice per day, morning and evening, continuously for 5 days, and a moisture content in a skin cuticle was measured by using SKICON-200 (IBS Co., Ltd.). A ratio in low frequency conductivity as a moisturizing effect between before the application and 5 days after the application was determined as a relative conductivity by the following calculation. The skin disorder is due to disturbance originated from skin hills and skin grooves, and slip feeling and rough feeling on a skin surface were evaluated by the panelist himself or herself. An evaluation standard was digitized as shown below, and a total value was obtained. Results were shown in Table 2-34. Relative conductivity = low frequency conductivity after application/ low frequency conductivity x 100(%).

### (Table 2-34)

**Table 2-34: Test results on moisture loss-suppressing effect: Relative conductivity**

| Powder to be surface-treated | Compound (a2) | Compound (b2) | Compound (c2) | Compound (d2) | Compound (e2) |
|---|---|---|---|---|---|
| Sericite FSE | 116% | 120% | 123% | 103% | 101% |
| Titanium CR-50 | 112% | 123% | 124% | 102% | 103% |
| Yellow - LL-100P | 110% | 123% | 124% | 103% | 103% |
| RedR-516PS | 116% | 120% | 123% | 100% | 103% |
| BlackBL-100P | 111% | 114% | 124% | 103% | 104% |
| Finely particulate titanium oxide | 110% | 116% | 113% | 102% | 103% |
| Finely particulate zinc oxide | 115% | 115% | 113% | 95% | 96% |
| Pearl pigment | 113% | 122% | 114% | 100% | 97% |
| AmihopeLL | 119% | 125% | 123% | 103% | 104% |
| Red No. 202 | 118% | 119% | 118% | 102% | 103% |
| Mixture of methicone/vinyl dimethicone and titanium oxide | 115% | 117% | 117% | 103% | 104% |
| Finely particulate zinc oxide | 115% | 117% | 119% | 98% | 97% |
| SericiteFSE | 120% | 113% | 114% | 103% | 100% |
| Talc JA-13R | 120% | 123% | 123% | 103% | 100% |
| Finely particulate titanium oxide | 110% | 112% | 114% | 97% | 99% |

The relative conductivity of blank only was 102%. As shown in Table 2-34, it was recognized that the powders coated with the fluorine-containing copolymers of the present invention had the moisture loss-suppressing effect.

### (Examples 2-16 to 2-38 and Comparative Examples 2-16 to 2-38)

Next, Examples of cosmetics into which the surface-treated powders of the present invention were blended will be explained. Various cosmetics were produced by the following producing methods based on respective compositions in the following tables.

Further, with respect to a cosmetic obtained in each of Examples and Comparative examples, 20 special panelists were prepared for each of evaluation items of usability, cosmetic finish and long-lasting property, and they used the cosmetic for one day, and made evaluations according to evaluation standards shown in Table 2-35 to Table 2-37 as given below. An evaluation mark was obtained by dividing total marks of all the panelists by 20. In this case, as evaluation items for the usability, spreading easiness and smoothness were evaluated.

### (Table 2-35)

**Table 2-35: Evaluation standard for usability**

| Standard | Mark |
|---|---|
| Very good usability | A |
| Good usability | B |
| Ordinary usability | C |
| Bad usability | D |
| Very bad usability | E |

### (Table 2-36]

**Table 2-36:Evaluation standard for cosmetic finish**

| Standard | Mark |
|---|---|
| High effect felt | A |
| Effect felt | B |
| Effect somewhat felt | C |
| Effect merely slightly felt | D |
| No effect felt | E |

Regarding finish items, finish uniformity, covering power, natural shinny feeling and uniformity of a cosmetic film were evaluated for makeups and skin care cosmetics; absence of stickiness and absence of oily feeling were evaluated for antiperspirant cosmetics; shinny feeling, dry feeling, smoothness and combing smoothness were evaluated for hair cosmetics.

### (Table 2-37)

**Table 2-37: Evaluation standard for long-lasting property**

| Standard | Mark |
|---|---|
| High effect felt | A |
| Effect felt | B |
| Effect somewhat felt | C |
| Effect merely slightly felt | D |
| No effect felt | E |

As to items of the long-lasting property, three items of color dullness preventing effect, secondary attachment (color transfer) preventing effect and shinning preventing effect were taken. Further, as to the manicure, peeling difficulty was evaluated.

### (Example 2-16 and Comparative Example 2-16) Production of powder foundations

Powder foundations having a composition shown in Table 2-38 were produced by the following method. Evaluation results were shown in Table 2-39.

### (Table 2-38)

**Table 2-38**

| Ingredients | wt. parts |
|---|---|
| ( 1 ) Sericite (5% treated product in Example 2-1 and Comparative Example 2-1) | 35.0 |
| (2) Talc (5% treated product in Example 2-14 and Comparative Example 2-14)) | to100.0 |
| ( 3 ) Silicone-treated titanium oxide | 8.5 |
| ( 4 ) Silicone-treated yellow iron oxide | 3.5 |
| ( 5 ) Silicone-treated red iron oxide | 1.8 |
| ( 6 ) Silicone-treated black iron oxide | 0.2 |
| ( 7 ) hydroxyapatite | 5.0 |
| ( 8 ) Octyldodecyl myristate | 4.0 |
| ( 9 ) Squalane | 3.5 |
| ( 10 ) Methylphenyl polysiloxane | 1.5 |
| ( 11 ) Antiseptic | approp. amount |
| ( 12 ) Perfume | approp. amount |

### (Producing method)

The above ingredients (1) to (7) were mixed, and pulverized through a grinding mill. The resultant was moved to a high speed blender, and a mixture in which ingredients (8) to (12) mixed and homogenized under heating were added thereto, followed by being further mixed and homogenized. After the resultant was passed through the mill and screened to adjust particle sizes, the powder was compression molded in an aluminum bowl under a surface pressing pressure of 10MPa, thereby producing a 2-way powder foundation.

With respect to a foundation bulk press molded above, a contact angle of squalane was measured by an underwater oil-repellency testing method described in JP-A-2003-253077.

### (Table 2-39)

**Table 2-39**

| Evaluation item | Compound (a2) | Compound (b2) | Compound (c2) | Compound (d2) | Compound (e2) |
|---|---|---|---|---|---|
| Usability | B | B | A | D | D |
| Uniform finish | B | B | A | C | D |
| Long-lasting property | A | A | A | B | B |
| Underwater oil repellency | 66 ° | 72 ° | 67 ° | 36 ° | 20 ° |

### (Example 2-17 and Comparative Example 2-17) Production of emulsion type foundations

W/O type liquid foundations having a composition shown in Table 2-40 were produced by the following method. Evaluation results were shown in Table 2-41.

### (Table 2-40)

**Table 2-40**

| Ingredient | Wt. parts |
|---|---|
| (1)Decamethylcyclopentasiloxane | 20.0 |
| (2)Vaseline | 0.5 |
| (3)Methyl phenyl polysiloxane | 2.3 |
| (4)Squalane | 4.2 |
| (5)Isotridecyl isononanoate | 4.5 |
| (6)Polyether-modified silicone *11 | 3.0 |
| (7)Silicone-treated red iron oxide | 1.3 |
| (8)Silicone-treated yellow iron oxide | 2.4 |
| (9)Silicone-treated black iron oxide | 0.1 |
| (10)Silicone-treated titanium oxide | 8.0 |
| (11)Silicone-treated talc | 2.5 |
| (12)Ethanol | 5.0 |
| (13)1,3-butylene glycol | 5.0 |
| (14)Sodium chloride | 2.0 |
| (15)Purified water | to100.0 |
| (16)AmihopeLL(5% treated product in Example 2-9 and Comparative Example 2-9) | 5.0 |
| (17)Antiseptic | approp. amount |
| (18)Perfume | approp. amount |

### (Producing method)

The above ingredients (7) to (11) were preliminarily mixed and pulverized. The mixture of the preliminarily pulverized ingredients (7) to (11) was added to an oily phase in which ingredients (1) to (6) were homogeneously dissolved and mixed at 70 ° C, and the resultant was homogeneously dispersed with a homodisperser. A water phase in which ingredients (12) to (17) were homogeneously mixed and dissolved at 70°C was gradually added to the oily phase, which was uniformly dispersed in a homo mixer and cooled. Then, an ingredient (18) was added thereto, and emulsion particles were adjusted to produce a liquid foundation.

### (Table 2-41)

**Table 2-41**

| Evaluation item | Compound (a2) | Compound (b2) | Compound (c2) | Compound (d2) | Compound (e2) |
|---|---|---|---|---|---|
| Usability | A | B | A | Not uniformly dispersed in the aqueous ingredients, and no formulation possible | |
| Uniform finish | B | A | B | | |
| Long-lasting property | A | A | A | | |
| Quality Stability*12 | Good | Good | Good | | |

### (Example 2-18 and Comparative Example 2-18) Production of eye shadows

Eye shadows having a composition shown in Table 2-42 were produced by the following method. Evaluation results were shown in Table 2-43.

### (Table 2-42)

**Table 2-42**

| Ingredient | wt. parts |
|---|---|
| 1. Decamethylcyclopentasiloxane | 25.0 |
| 2.Dimethylpolysiloxane (6 c s ) | 10.0 |
| 3.Polyether-modified silicone * 1 3 | 2.0 |
| 4.PEG (10) lauryl ether | 0.5 |
| 5.Titanium oxide (5% treated product in Example 2-2 and Comparative Example 2-2) | 2.5 |
| 6.Red iron oxide (6% treated product in Example 2-4 and Comparative Example 2-4) | 3.5 |
| 7.Pearl pigment (5% treated product in Example 2-8 and Comparative Example 2-8) | 6.0 |
| 8.Sodium chloride | 2.0 |
| 9.Propylene glycol | 8.0 |
| 10.antiseptic | approp. amount |
| 11.Perfume | approp. amount |
| 12.Purified water | to 100.0 |

| | |
|---|---|
| *13 KF6026 (Shin-Etsu Chemical Co., Ltd.) | |

### (Producing method)

(1) Ingredients 1 to 4 are mixed and dissolved.
(2) Ingredients 5 to 10 and an ingredient 12 are mixed and homogeneously dispersed.
(3) Under stirring, the mixture obtained in (2) was gradually added to the mixture obtained in (1), and an emulsified ingredient 11 was added thereto, thereby obtaining an eye shadow.

### (Table 2-43)

**Table 2-43**

| Evaluation item | Compound (a2) | Compound (b2) | Compound (c2) | Compound (d2) | Compound (e2) |
|---|---|---|---|---|---|
| Usability | B | B | B | Not uniformly dispersed in the aqueous ingredients, and no formulation possible | |
| Uniform finish | B | B | A | | |
| Long-lasting property | A | A | B | | |

### (Example 2-19 and Comparative Example 2-19) Production of oily solid foundations

Oily solid foundations having a composition shown in Table 2-44 were produced by the following method. Evaluation results were shown in Table 2-45.

### (Table 2-44)

**Table 2-44**

| Ingredient | wt. parts |
|---|---|
| (1)Isopropyl palmitate | 20.0 |
| (2)Cetanol | 7.0 |
| (3)Squalane | 15.5 |
| (4)Polyglyceryl triisostearate | 5.0 |
| (5)Volatile fluid paraffin | to100.0 |
| (6)Ceresin wax | 3.8 |
| (7)Candelilla wax | 5.5 |
| (8)Red iron oxide (6% treated product in Example 2-4 and Comparative Example 2-4) | 0.8 |
| (9)Red iron oxide (5% treated product in Example 2-2 and Comparative Example 2-2) | 11.5 |
| (10)Talc (5% treated product in Example 2-14 and Comparative Example 2-14) | 8.0 |
| (11)Antiseptic | approp. amount |
| (12)Perfume | approp. amount |

### (Producing method)

The above ingredients (8) to (10) were preliminarily mixed and pulverized. The preliminarily comminuted ingredients (8) to (11) were added to an oily phase in which ingredients (1) to (7) were mixed and dissolved at 85°C, which was dispersed homogeneously by a homodisperser. A perfume was added thereto, which was packed into a metal bowl and cooled, thereby producing an oily solid foundation.

### (Table 2-45)

**Table 2-45**

| Evaluation item | Compound (a2) | Compound (b2) | Compound (c2) | Compound (d2) | Compound (e2) |
|---|---|---|---|---|---|
| Usability | A | B | B | D | E |
| Uniform finish | B | A | A | D | E |
| Long-lasting property | B | B | B | D | D |

### (Example 2-20 and Comparative Example 2-20) Production of emulsion type sunscreen creams

Emulsion type sunscreen creams having a composition shown in Table 2-46 were produced by the following method. Evaluation results were shown in Table 2-47.

### (Table 2-46)

**Table 2-46**

| Ingredient | wt. parts |
|---|---|
| 1 . Volatile fluid isoparaffin (isododecane) | 15.0 |
| 2 . Dimethicone (6cs) | 3.0 |
| 3 . Isononyl isononate | 4.5 |
| 4. Cetanol | 1.0 |
| 5. Squalane | 5.0 |
| 6 . Polyethylene monostearate glycol (4EO) | 1.0 |
| 7 . Hexaglyceryl polyricinoleate | 3.5 |
| 8. Aqueous dispersion of finely particulate titanium oxide * 14 | 10.0 |
| 9 . Aqueous dispersion of finely particulate zinc oxide *15 | 15.0 |
| 1 0 . Purified water | to100.0 |
| 1 1. Glycerin | 5.0 |
| 1 2 . 1,3-butylene glycol | 5.0 |
| 1 3 . Sodium pyrrolidone carboxylate | 2.5 |
| 1 4 . Antiseptic | approp. amount |
| 1 5 . Perfume | approp. amount |

| | |
|---|---|
| *14 Dispersions were obtained by dispersing the finely particulate titanium oxides treated at 10% in Example 2-6 and Comparative Example 2-6 and purified water in a composition of 30:70 (parts by weight) in a sand grinder. The surface-treated powder coated with the specific fluorine-containing copolymer of the present invention had high pigment dispersing power and storage stability. *15 Dispersions were obtained by dispersing the finely particulate zinc oxides treated at 9% in Example 2-7 and Comparative Example 2-7 and purified water in a composition of 35:65 (parts by weight) in a sand grinder. The surface-treated powder coated with the specific fluorine-containing copolymer of the present invention had high pigment dispersing power and storage stability. | |

### (Producing method)

Oily phase ingredients (1) to (7) were dissolved at 75°C. Oily phase ingredients (8) to (14) were dissolved, dispersed and homogenized at 75 ° C, which was added to the oily phase ingredients and emulsified in the homo mixer. Finally, an ingredient (15) was added thereto, followed by cooling, thereby producing a sunscreen cream.

### (Table 2-47)

**Table 2-47**

| Evaluation item | Compound (a2) | Compound (b2) | Compound (c2) | Compound (d2) | Compound (e2) |
|---|---|---|---|---|---|
| Usability | A | B | B | Not uniformly dispersed in the aqueous ingredients, and no formulation possible | |
| Uniform finish | B | A | A | | |
| Long-lasting property | A | A | A | | |

### (Example 2-21 and Comparative Example 2-21) Production of eyeliners

Eyeliners having a composition shown in Table 2-48 were produced by the following method. Evaluation results were shown in Table 2-49.

### (Table 2-48)

**Table 2-48**

| Ingredient | wt. parts |
|---|---|
| (1)Decamethylcyclopentasiloxane | 25.0 |
| (2)Dimethyl polysiloxane (6cs) | 4.0 |
| (3)Jojoba oil | 2.5 |
| (4)Polyether-modified silicone * 16 | 1.0 |
| (5)Black iron oxide (5% treated product in Example 2-5 and Comparative Example 2-5) | 20.0 |
| (6)Ethanol | 5.0 |
| (7)Antiseptic | approp. amount |
| (8)Purified water | to 100.0 |

| | |
|---|---|
| *16 KF6026 (Shin-Etsu Chemical Co., Ltd.) | |

### (Producing method)

(1) Ingredients 1 to 4 are mixed at 70°C.
(2) Ingredients 5 to 8 are heated, mixed and dissolved, and an ingredient (5) is added and homogeneously dispersed thereinto.
(3) Under stirring, the mixture obtained in (2) was gradually added to the mixture obtained in (1), which was emulsified to obtain an eyeliner.

### (Table 2-49)

**Table 2-49**

| Evaluation item | Compound (a2) | Compound (b2) | Compound (c2) | Compound (d2) | Compound (e2) |
|---|---|---|---|---|---|
| Usability | B | B | B | Not uniformly dispersed in the aqueous ingredients, and no formulation possible. | |
| Uniform finish | B | A | A | | |
| Long-lasting property | A | A | A | | |

### (Example 2-22 and Comparative Example 2-22) Production of lipsticks

Lipsticks having a composition shown in Table 2-50 were produced by the following method. Evaluation results were shown in Table 2-51.

### (Table 2-50)

**Table 2-50**

| Ingredient | wt. parts |
|---|---|
| (1)Diisostearyl malate | 15.0 |
| (2)Glyceryl triisooctanoate | to 100.0 |
| (3)Phytosterol octyldodecanol glutamate | 15.0 |
| (4)Carnauba wax | 3.5 |
| (5)Candelilla Wax | 5.5 |
| (6)Ceresin wax | 5.0 |
| (7)Titanium oxide (5% treated product of Example 2-2 and Comparative Example 2-2) | 8.0 |
| (8)Red No. 202 (9% treated product of Example 2-10 and Comparative Example 2-10) | 0.7 |
| (9)Black iron oxide (5% treated product of Example 2-5 and Comparative Example 2-5) | 0.2 |
| (10)Antiseptic | approp. amount |

### (Producing method)

(1) Ingredients 7 to 9 were added to an ingredient 1, which was kneaded and dispersed by rollers.
(2) After the other ingredients were heated, mixed and dissolved, the above ingredients (1) was added thereto, which was homogeneously dispersed by a homo mixer.
(3) After deaeration, the dispersion was poured into a mold, thereby obtaining a stick-shaped lipstick.

### (Table 2-51)

**Table 2-51**

| Evaluation item | Compound (a2) | Compound (b2) | Compound (c2) | Compound (d2) | Compound (e2) |
|---|---|---|---|---|---|
| Usability | A | B | B | D | D |
| Uniform finish | B | B | A | D | E |
| Longlasting property | A | A | A | D | D |

### (Example 2-23 and Comparative Example 2-23) Production of lipstick overcoats

Lipstick overcoats having a composition shown in Table 2-52 were produced by the following method. Evaluation results were shown in Table 2-53.

### (Table 2-52)

**Table 2-52**

| Ingredient | wt. parts |
|---|---|
| (1)Perfluoropolyether (FOMBLIN HC-04) | 91.0 |
| (2)Silica (Aerosil R-972) *17 | 2.0 |
| (3)Finely particulate titanium oxide (10% treated product of Example 2-15 and Comparative Example 2-15) | 6.0 |
| (4)Glycerin | 1.0 |

| | |
|---|---|
| *17 Silica surface treated at 5% with each of the fluorine-containing compounds (a2) to (e2) in the method of Example 2-1 and Comparative Example 2-1 was blended. | |

### (Producing method)

After an ingredient (2) and an ingredient (3) were added and homogeneously dispersed in an ingredient (1), and an ingredient (4) was added thereto, thereby producing a lipstick overcoat.

### (Table 2-53)

**Table 2-53**

| Evaluation item | Compound (a2) | Compound (b2) | Compound (c2) | Compound (d2) | Compound (e2) |
|---|---|---|---|---|---|
| Usability | B | B | B | B | C |
| Uniform finish | B | A | A | D | E |
| Long-lasting property | A | A | A | D | B |
| Transfer resistance *18 | B | B | B | E | D |

| | | | | | |
|---|---|---|---|---|---|
| *18 When each panelist applied the present lipstick overcoat after having applied the lipsticks in Example 2-22 and Comparative Example 2-22, a state in which the lipstick was transferred to a cup was observed. The following marks were given. Not transferred B, Slightly transferred D, Transferred E. | | | | | |

### (Example 2-24 and Comparative Example 2-24) Production of emulsion type liquid foundations

O/W type liquid foundations having a composition shown in Table 2-54 were produced by the following method. Evaluation results were shown in Table 2-55.

### (Table 2-54)

**Table 2-54**

| Ingredient | wt. parts |
|---|---|
| (1)Stearic acid | 1.5 |
| (2)Cetanol | 1.0 |
| (3)Polyoxyethylene sorbitan monooleate (3EO) | 1.0 |
| (4)Sorbitan sesquioleate | 1.0 |
| (5)Fluid paraffin | 5.0 |
| (6)Glyceryl 2-ethylhexanoate | 5.0 |
| (7)Glycerin | 5.0 |
| (8)1,3-butylene glycol | 5.0 |
| (9)Alkyl (C10-30) acrylate-methacrylate copolymer | 0.05 |
| (10)Locust bean gum | 0.2 |
| (11)Triethanolamine | 0.8 |
| (12)Antiseptic | approp. amount |
| (13)Purified water | to100.0 |
| (14)Titanium oxide (5% treated product of Example 2-2 and Comparative Example 2-2) | 5.0 |
| (15)Yellow iron oxide (6% treated product of Example 2-3 and Comparative Example 2-3) | 1.0 |
| (16)Red iron oxide (6% treated product of Example 2-4 and Comparative Example 2-4) | 0.3 |
| (17)Black iron oxide (5% treated product of Example 2-5 and Comparative Example 2-5) | 0.1 |
| (18)Amihope LL (5% treated product of Example 2-9 and Comparative Example 2-9) | 5.0 |

### (Producing method)

A: Ingredients (1) to (6) were heated and dissolved.
B: After ingredients (7) to (13) were homogeneously heated and dissolved, ingredients (14) to (18) were added thereto, which was homogeneously mixed.
C: The mixture obtained in B was added to the mixture obtained in A, which was cooled to then produce a liquid foundation.

### (Table 2-55)

**Table 2-55**

| Evaluation item | Compound (a2) | Compound (b2) | Compound (c2) | Compound (d2) | Compound (e2) |
|---|---|---|---|---|---|
| Usability | B | B | B | Not uniformly dispersed in the aqueous ingredients, and no formulation possible | |
| Uniform finish | B | A | A | | |
| Long-lasting property | A | A | A | | |

### (Example 2-25 and Comparative Example 2-25) Production of skin color emulsions

Skin color emulsions having a composition shown in Table 2-56 were produced by the following method. Evaluation results were shown in Table 2-57.

### (Table 2-56)

**Table 2-56**

| Ingredient | wt. parts |
|---|---|
| (1)N-stearoyl-L-glutamate | 0.5 |
| (2)Cetanol | 0.5 |
| (3)Polyoxyethylene (10mol) monostearate | 0.8 |
| (4)Decaglyceryl monoisostearate | 1.0 |
| (5)Sorbitan sesquioleate | 0.4 |
| (6)Methylphenyl polysiloxane | 5.0 |
| (7)Glycerin | 5.0 |
| (8)1,3-butylene glycol | 5.0 |
| (9)Xanthane gum | 0.1 |
| (10)Carrageenan | 0.05 |
| (11)Triethanolamine | 1.0 |
| (12)Antiseptic | approp. amount |
| (13)Purified water | to100.0 |
| (14)Titanium oxide (5% treated product in Example 2-2 and Comparative Example 2-2) | 2.0 |
| (15)Yellow iron oxide (5% treated product in Example 2-3 and Comparative Example 2-3) | 1.0 |
| (16)Red iron oxide (6% treated product in Example 2-4 and Comparative Example 2-4) | 0.5 |
| (17)Black iron oxide (5% treated product in Example 2-5 and Comparative Example 2-5) | 0.1 |
| (18)Talc (5% treated product in Example 2-14 and Comparative Example 2-14) | 3.0 |

### (Producing method)

A: Ingredients (1) to (6) were heated and dissolved.
B: After ingredients (7) to (13) were homogeneously heated and dissolved, ingredients (14) to (18) were added and homogeneously mixed.
C: The mixture obtained in B was added to the mixture obtained in A, which was cooled to then produce a skin color emulsion.

### (Table 2-57)

**Table 2-57**

| Evaluation item | Compound (a2) | Compound (b2) | Compound (c2) | Compound (d2) | Compound (e2) |
|---|---|---|---|---|---|
| Usability | A | B | B | Not uniformly dispersed in the aqueous ingredients, and no formulation possible | |
| Uniform finish | B | A | A | | |
| Long-lasting property | A | A | A | | |

### (Example 2-26 and Comparative Example 2-26) Production of manicures

Manicures having a composition shown in Table 2-58 were produced by the following method. Evaluation results were shown in Table 2-59.

### (Table 2-58)

**Table 2-58**

| Ingredient | wt. parts |
|---|---|
| (1)Nitrocellulose | 10.0 |
| (2)Alkyd resin | 10.0 |
| (3)Acetyl tributyl citrate | 4.0 |
| (4)D1-camphor | 1.0 |
| (5)Organically modified hectorite | 1.0 |
| (6)Ethyl acetate | 20.0 |
| (7)Butyl acetate | to100.0 |
| (8)Isopropyl alcohol | 5.0 |
| (9)Red No. 202 (9% treated product in Example 2-10 and Comparative Example 2-10) | 0.1 |
| (10)Titanium oxide (9% treated product in Example 2-10 and Comparative Example 2-10) | 3.0 |
| (11)Pearl pigment (5% treated product in Example 2-8 and Comparative Example 2-10) | 5.0 |
| (12)Read iron oxide (6% treated product in Example 2-4 and Comparative Example 2-4) | 0.5 |

### (Producing method)

Ingredients (1) to (9) were mixed and stirred in a disperser. Ingredients (10) to (12) were added thereto, and the resultant was mixed in the disperser for 10 minutes to produce a manicure, which was packed into a glass bottle.

### (Table 2-59)

**Table 2-59**

| Evaluation item | Compound (a2) | Compound (b2) | Compound (c2) | Compound (d2) | Compound (e2) |
|---|---|---|---|---|---|
| Usability | B | B | B | D | D |
| Uniform finish | B | B | B | D | D |
| Long-lasting property | A | A | A | B | B |

In addition, the manicures into which the surface-treated powders coated with the specific fluorine-containing copolymers of the present invention were blended had good dispersion stability without becoming a hard cake with a lapse of time.

### (Example 2-27 and Comparative Example 2-27) Production of Cheek colors

Cheek colors having a composition shown in Table 2-60 were produced by the following method. Evaluation results were shown in Table 2-61.

### (Table 2-60)

**Table 2-60**

| Ingredient | wt. parts |
|---|---|
| (1)Sericite (3% treated product in Example 2-13 and Comparative Example 2-13) | 14.0 |
| (2)Titanium oxide (5% treated product in Example 2-2 and Comparative Example 2-2) | 10.0 |
| (3)Yellow iron oxide (5% treated product in Example 2-3 and Comparative Example 2-3) | 1.0 |
| (4)Red iron oxide (6% treated product in Example 2-4 and Comparative Example 2-4) | 0.5 |
| (5)Black iron oxide (5% treated product in Example 2-5 and Comparative Example 2-5) | 0.1 |
| (6)Talc (5% treated product in Example 2-14 and Comparative Example 2-14) | to 100.0 |
| (7)Excel Mica JP-2 (Miyoshi Kasei, Inc.) | 7.0 |
| (8)Perfluoropolyether (FOMBLIN HC-04) | 7.0 |
| (9)Dimethylpolysiloxane (10CS) | 5.0 |

### (Producing method)

Ingredients (1) to (7) were homogeneously mixed and pulverized. After ingredients (8) and (9) were added thereto and homogeneously mixed, the mixture was pulverized, thereby producing a cheek color.

### (Table 2-61)

**Table 2-61**

| Evaluation item | Compound (a2) | Compound (b2) | Compound (c2) | Compound (d2) | Compound (e2) |
|---|---|---|---|---|---|
| Usability | B | B | B | E | E |
| Uniform finish | A | A | B | D | D |
| Long-lasting property | A | A | A | B | B |

### (Example 2-28 and Comparative Example 2-28) Production of antiperspirant cosmetics

Stick-shaped antiperspirant cosmetics having a composition shown in Table 2-62 were produced by the following method. Evaluation results were shown in Table 2-63.

### (Table 2-62)

**Table 2-62**

| Ingredient | wt. parts |
|---|---|
| (1)Carnauba wax | 1.0 |
| (2)Ceresin | 6.0 |
| (3)Paraffin wax | 3.0 |
| (4)Sorbitan sesqui isostearate | 2.5 |
| (5)Cetyl 2-ethylhexanoate | 27.0 |
| (6)Squalane | 2.0 |
| (7)Decamethyl cyclopentasiloxane | to 100.0 |
| (8)Aluminum hydroxychloride | 25.0 |
| (9)Sericite (3% treated product in Example 2-13 and Comparative Example 2-13) | 5.0 |
| (10)Silicone-treated talc | 8.0 |
| (11)Powder Lavie (Miyoshi Kasei, Inc.) | 5.0 |
| (12)Finely particulate zinc oxide (7% treated product in Example 2-1 and Comparative Example 2-12) | 2.0 |
| (13)Antioxidant | approp. amount |
| (14)Perfume | approp. amount |

### (Producing method)

Ingredients (1) to (7) were heated and homogeneously dissolved. Ingredients (8) to (13) were added thereto, which was homogeneously mixed. Then, an ingredient (14) was added thereto, which was cooled to produce a stick-shaped antiperspirant cosmetic.

### (Table 2-63)

**Table 2-63**

| Evaluation item | Compound (a2) | Compound (b2) | Compound (c2) | Compound (d2) | Compound (e2) |
|---|---|---|---|---|---|
| Usability | B | A | B | E | E |
| Uniform finish | A | A | B | D | D |
| Long-lasting property | A | A | A | B | B |

### (Example 2-29 and Comparative Example 2-29) Production of hair dressing agents

Hair dressing agents having a composition shown in Table 2-64 were produced by the following method. Evaluation results were shown in Table 2-65.

### (Table 2-64)

**Table 2-64**

| Ingredient | wt. parts |
|---|---|
| (1)Ethylene glycol distearate | 1.0 |
| (2)Liquid paraffin | 10.0 |
| (3)Squalane | 5.0 |
| (4)Stearyl alcohol | 1.5 |
| (5)Dimethylpolysiloxane (10cs) | 3.0 |
| (6)Stearic acid | 6.0 |
| (7)Polyoxyethylene (3) stearyl alcohol | 4.5 |
| (8)Polyoxyethylene (150) cetyl ether | 2.0 |
| (9)Organically modified bentonite | 0.2 |
| (10)AmihopeLL (5% treated product in Example 2-9 and Comparative Example 2-9) | 2.0 |
| (11)1,3-butylene glycol | 6.0 |
| (12)Antiseptic | approp. amount |
| (13)Purified water | to100.0 |
| (14)Perfume | approp. amount |

### (Producing method)

A: Ingredients (1) to (9) were heated and homogeneously dissolved.
B: Ingredients (10) to (13) were heated, mixed and dispersed.
C: After the mixture obtained in B was added and mixed into the mixture obtained in A, followed by cooling, and an ingredient (14) was added thereto, which was cooled to produce a hair dressing agent.

### (Table 2-65)

**Table 2-65**

| Evaluation item | Compound (a2) | Compound (b2) | Compound (c2) | Compound (d2) | Compound (e2) |
|---|---|---|---|---|---|
| Usability | B | A | B | Not uniformly dispersed in the aqueous ingredients, and no formulation possible | |
| Uniform finish | A | A | A | | |
| Long-lasting property | A | A | A | | |

### (Example 2-30 and Comparative Example 2-30) Production of skin color moisturizing lotions

Skin color moisturizing lotions having a composition shown in Table 2-66 were produced by the following method. Evaluation results were shown in Table 2-67.

### (Table 2-66)

**Table 2-66**

| Ingredient | wt. parts |
|---|---|
| (1)Purified water | to100.0 |
| (2)Alkyl (C10-30) acrylate-methacrylate copolymer | 0.1 |
| (3)Carboxyvinyl polymer | 1.0 |
| (4)Antiseptic | approp. amount |
| (5)Finely particulate titanium oxide (10% treated product in Example 1-15 and Comparative Example 2-15) | 1.0 |
| (6)Triethanolamine | 0.7 |
| (7)Glycerin | 25.0 |
| (8)Titanium oxide (5% treated product in Example 2-2 and Comparative Example 2-2) | 2.0 |
| (9)Yellow iron oxide (5% treated product in Example 2-3 and Comparative Example 2-3) | 0.8 |
| (10)Red iron oxide (6% treated product in Example 2-4 and Comparative Example 2-4) | 0.3 |
| (11)Black iron oxide (5% treated product in Example 2-5 and Comparative Example 2-5) | 0.1 |
| (12)Talc(5% treated product in Example 2-14 and Comparative Example 2-14) | 3.5 |

### (Producing method)

A: After ingredients (1) to (4) are heated and homogeneously dissolved, an ingredient (5) is added thereto, which is homogenously dispersed and mixed. An ingredient (6) is added to neutralize the resultant.
B: After ingredients (8) to (12) are homogeneously mixed, an ingredient (7) is added thereto, which is homogeneously mixed.
C: A skin color moisturizing lotion was produced by adding and mixing the mixture obtained in B to the mixture obtained in A.

### (Table 2-67)

**Table 2-67**

| Evaluation item | Compound (a2) | Compound (b2) | Compound (c2) | Compound (d2) | Compound (e2) |
|---|---|---|---|---|---|
| Usability | B | B | B | Not uniformly dispersed in the aqueous ingredients, and no formulation possible | |
| Uniform finish | A | A | A | | |
| Long-lasting property | A | A | A | | |

### (Example 2-31 and Comparative Example 2-31) Production of liquid eye shadows

Aqueous liquid eye shadows having a composition shown in Table 2-68 were produced by the following method. Evaluation results were shown in Table 2-69.

### (Table 2-68)

**Table 2-68**

| Ingredient | wt. parts |
|---|---|
| (1)Purified water | to100.0 |
| (2)Alkyl (C10-30) acrylate-methacrylate copolymer | 0.1 |
| (3)Carboxyvinyl polymer | 1.0 |
| (4)Antiseptic | approp. amount |
| (5)Triethanolamine | 0.7 |
| (6)Glycerin | 25.0 |
| (7)Titanium oxide (5% treated product in Example 2-2 and Comparative Example 2-2) | 2.5 |
| (8)Red iron oxide (6% treated product in Example 2-3 and Comparative Example 2-3) | 2.0 |
| (9)Pearl pigment (5% treated product in Example 2-8 and Comparative Example 2-8) | 8.0 |

### (Producing method)

A: Ingredients (1) to (4) are heated and uniformly dissolved, and then an ingredient (5) is added and uniformly mixed thereinto.
B: After ingredients (7) to (9) are uniformly mixed, an ingredient (6) is added and uniformly mixed thereinto.
C: A liquid aqueous eye shadow was produced by adding and mixing the mixture obtained in B into the mixture obtained in A.

### (Table 2-69)

**Table 2-69**

| Evaluation item | Compound (a2) | Compound (b2) | Compound (c2) | Compound (d2) | Compound (e2) |
|---|---|---|---|---|---|
| Usability | B | B | B | Not uniformly dispersed in the aqueous ingredients, and no formulation possible | |
| Uniform finish | A | A | A | | |
| Long-lasting property | A | A | A | | |

### (Example2-32 and Comparative Example 2-32) Production of whitening powders

Whitening powders having a composition shown in Table 2-70 were produced by the following method. Evaluation results were shown in Table 2-71.

### (Table 2-70)

**Table 2-70**

| Ingredient | wt. parts |
|---|---|
| (1)Magnesium ascorbyl phosphate | 1.0 |
| (2)Sodium citrate | 1.0 |
| (3)1,3-butylene glycol | 5.0 |
| (4)Glycerin | 5.0 |
| (5)Purified water | to100.0 |
| (6)Methyl parahydroxybenzoate | 0.2 |
| (7)Sericite (5% treated product in Example 2-1 and Comparative Example 2-1) | 5.0 |
| (8)Talc (5% treated product in Example 2-14 and Comparative Example 2-14) | 0.1 |
| (9)Powder of organopolysiloxane elastomer | 3.0 |
| (10)Partially crosslinked organopolysiloxane polymer *19 | 5.0 |
| (11)Partially crosslinked organopolysiloxane polymer | 0.5 |
| (12) Trifluoroalkyldimethyl Trimethylsiloxysilicate | 3.0 |

### (Producing method)

A: Ingredients (1) to (8) are mixed and dispersed.
B: Ingredients (9) to (12) are uniformly mixed.
C: A whitening powder was obtained by mixing the mixture obtained in B with the mixture obtained in A and packing the resultant into a container.

### (Table 2-71)

**Table 2-71**

| Evaluation item | Compound (a2) | Compound (b2) | Compound (c2) | Compound (d2) | Compound (e2) |
|---|---|---|---|---|---|
| Usability | B | B | B | Not uniformly dispersed in the aqueous ingredients, and no formulation possible | |
| Uniform finish | A | A | A | | |
| Long-lasting property | A | A | A | | |

### (Example 2-33 and Comparative Example 2-33) Production of sunscreen creams

Sunscreen creams having a composition shown in Table 2-72 were produced by the following method. Evaluation results were shown in Table 2-73.

### (Table 2-72)

**Table 2-72**

| Ingredient | wt. parts |
|---|---|
| (1)Decamethyl cyclopentasiloxane | 15.0 |
| (2)Long-chain alkyl-containing POE-modified silicone (ABIL EM90:Evonik Degussa GmbH) | 2.0 |
| (3)Isononyl isononate | 5.0 |
| (4)Diethylhexyl succinate | 5.0 |
| (5)Avobenzone(Parsol1789:DSM Nutrition Japan K.K.) | 0.8 |
| (6)Octocrylene(EusolexOCR:Merck & Co., Inc.) | 5.0 |
| (7)Finely particulate zinc oxide (7% treated product in Example 2-12 and Comparative Example 2-12) | 10.0 |
| (8)Purified water | to100.0 |
| (9)Glycerin | 3.0 |
| (10)Ethanol | 5.0 |
| (11)Aqueous dispersion of finely particulate titanium oxide *20 | 10.0 |
| (12)Antiseptic | approp. amount |

| | |
|---|---|
| *10 A water dispersion was obtained by dispersing a composition of the finely particulate titanium oxide treated at 10% in Example 2-6 and Comparative Example 2-6/purified water/glycerin = 45/50/5 in a sand grinder. The water dispersions in Examples were stable without precipitation separation even with a lapse of time. The dispersions in Comparative Examples did not become dispersed, which could not produce a homogeneous and stable dispersion. | |

### (Producing method)

A: Ingredients (3) to (6) are heated and mixed.
B: After ingredients (1) and (2) are uniformly mixed, the resultant is added to the mixture obtained in A.
C: Ingredients (7) to (12) are mixed and dissolved.
D: A sunscreen cream was obtained by adding and emulsifying the mixture obtained in the above B into the mixture obtained in C.

### (Table 2-73)

**Table 2-73**

| Evaluation item | Compound (a2) | Compound (b2) | Compound (c2) | Compound (d2) | Compound (e2) |
|---|---|---|---|---|---|
| Usability | A | A | A | Not uniformly dispersed in the aqueous ingredients, and no formulation possible | |
| Uniform finish | A | A | A | | |
| Long-lasting property | A | A | A | | |
| Quality Stability *21 | A | A | A | | |

| | | | | | |
|---|---|---|---|---|---|
| *21 Regarding the quality stability, a sample was left in a thermostatic bath at 50 ° C for one week, and whether crystallization, separation and precipitation of the UV absorber occurred or not were observed. A result was judged good for one with no change in appearance such as crystallization and the like. The cosmetics into which the compounds (d2) and (e2) in Comparative Examples were blended caused the crystallization and separation the next day, so they had poor stability. | | | | | |

### (Example 2-34 and Comparative Example 2-34) Production of sunscreen gels

Sunscreen gels having a composition shown in Table 2-74 were produced by the following method. Evaluation results were shown in Table 2-75.

### (Table 2-74)

**Table 2-74**

| Ingredient | wt. parts |
|---|---|
| (1)Glycerin | 5.0 |
| (2)Long-chain alkyl acrylate-methacrylate copolymer (PemulenTR-1:BF Goodrich Corporation) | 0.3 |
| (3)Methylparaben | 0.2 |
| (4)Purified water | to 100.0 |
| (5)Potassium hydroxide | 0.2 |
| (6)Purified water | 5.0 |
| (7)Finely particulate zinc oxide (7% treated product in Example 2-12 and Comparative Example 2-12) | 10.0 |
| (8)Isooctyl paradimethylaminobenzoate | 5.0 |
| (9)Glyceryl monooleyl ether | 1.0 |

### (Producing method)

After ingredients (1) to (4) are heated at 80°C, an ingredient (7) is added thereto, which is dispersed in a disperser. Ingredients (5) and (6) are heated and dissolved at 80°C. The heated and dissolved ingredients (5) and (6) are added to the above ingredients, followed by neutralization. Ingredients (8) and (9) are mixed, and heated at 80°C. These ingredients were gradually added to those neutralized above, which was emulsified in a homo mixer to obtain a sunscreen gel.

### (Table 2-75)

**Table 2-75**

| Evaluation item | Compound (a2) | Compound (b2) | Compound (c2) | Compound (d2) | Compound (e2) |
|---|---|---|---|---|---|
| Usability | A | A | A | The treated powder was not dispersed in an aqueous layer, so no gel could not be obtained. | |
| Uniform finish | B | A | A | | |
| Long-lasting property | B | B | A | | |
| SPF value of Formulation | 18 | 20 | 19 | | |
| Temporal stability *22 | A | A | A | | |

| | | | | | |
|---|---|---|---|---|---|
| *22 The present formulations were stored at 50°C for one month. One free from change in appearance was taken as A. The cosmetics blended with the surface-treated powders of the present invention had no reactivity with the aqueous polymer, so that the stable formulations could be obtained. | | | | | |

### (Example 2-35 and Comparative Example 2-35) Production of wet-molded type powder foundations

Foundations having a composition shown in Table 2-76 were produced by the following method. Evaluation results were shown in Table 2-77.

### (Table 2-76)

**Table 2-76**

| Ingredient | wt. parts |
|---|---|
| (1)Sericite (3% treated product in Example 2-13 and Comparative Example 2-13) | to100.0 |
| (2)MW treatment ((hydrophilicizing treatment)PMMA (Miyoshi Kasei, Inc.) | 10.0 |
| (3)Titanium oxide (5% treated product in Example 2-2 and Comparative Example 2-2) | 12.0 |
| (4)Yellow iron oxide (5% treated product in Example 2-3 and Comparative Example 2-3) | 2.5 |
| (5)Red iron oxide (6% treated product in Example 2-4 and Comparative Example 2-4) | 0.8 |
| (6)Black iron oxide (5% treated product in Example 2-5 and Comparative Example 2-5) | 0.3 |
| (7)2-Ethylhexyl para methoxy cinnamate | 3.0 |
| (8)Polyoxyethylene (20mol)hydrogenated castor oil | 1.5 |

### (Producing method)

A: Ingredients (7) and (8) are heated at 75°C, and uniformly dissolved.
B: Ingredients (1) to (6) are mixed in a mixer and pulverized in an atomizer.
C: The above A is added and uniformly dispersed, while the above B is being stirred.
D: To 100 parts by weight of the ingredients in C is added 100 parts by weight of purified water, which is mixed uniformly to obtain a slurry.
E: The above D is packed in metal bowl, and a part of purified water is removed by placing and pressing an absorbent paper onto a surface thereof.
F: The F was left in a thermostatic bath at 50°C for 24 hours, and a wet molded type powder foundation was obtained by completely removing purified water.

### (Table 2-77)

**Table 2-77**

| Evaluation item | Compound (a2) | Compound (b2) | Compound (c2) | Compound (d2) | Compound (e2) |
|---|---|---|---|---|---|
| Usability | B | B | A | Not uniformly dispersed in the aqueous ingredients, and no formulation possible | |
| Uniform finish | A | A | B | | |
| Long-lasting property | A | A | A | | |

### (Example 2-36 and Comparative Example 2-36) Production of F/W emulsified lotion

Lotions having a composition shown in Table 2-78 were produced by the following method. Evaluation results were shown in Table 2-79.

### (Table 2-78)

**Table 2-78**

| Ingredient | wt. parts |
|---|---|
| (1)Perfluoropolyether(FOMBLIN HC/04 : Solvay SA) | 25.0 |
| (2)Hydrofluoroether(CF-76:3M Corporation) | 10.0 |
| (3)Silicone-treated talc | 1.5 |
| (4)Ethanol | 8.0 |
| (5)Glycerin | 3.5 |
| (6)Finely particulate zinc oxide (7% treated product in Example 2-12 and Comparative Example 2-12) | 5.0 |
| (7)Purified water | to100.0 |
| (8)Antiseptic | approp. amount |

### (Producing method)

A: Ingredients (1) to (3) are uniformly mixed, and heated at 50°C.
B: Ingredients (4) to (8) are dispersed, dissolved and mixed in a homo mixer, which is heated at 50°C.
C: an F/W emulsified lotion was obtained by gradually adding the above A, while the above B was being stirred.

### (Table 2-79)

**Table 2-79**

| Evaluation item | Compound (a2) | Compound (b2) | Compound (c2) | Compound (d2) | Compound (e2) |
|---|---|---|---|---|---|
| Usability | B | B | A | The treated powder was not uniformly dispersed, so no emulsion was produced. | |
| Uniform finish | B | B | A | | |
| Long-lasting property | A | A | A | | |

### (Example 2-37 and Comparative Example 2-37) Production of W/F emulsified lotions

Lotions having a composition shown in Table 2-80 were produced by the following method. Evaluation results were shown in Table 2-81.

### (Table 2-80)

**Table 2-80**

| Ingredient | wt. parts |
|---|---|
| (1)Perfluoropolyether(FOMBLIN HC/04 : Solvay SA) | 45.0 |
| (2)Hydrofluoroether(CF-76:3M Corporation) | 15.0 |
| (3)Silicone-treated talc | 1.5 |
| (4)Ethanol | 8.0 |
| (5)Glycerin | 3.5 |
| (6)Finely particulate zinc oxide(7% treated product in Example 2-12 and Comparative Example 2-12) | 5.0 |
| (7)Purified water | to100.0 |
| (8)Antiseptic | approp. amount |

### (Producing method)

A: Ingredients (1) to (3) are homogeneously mixed, and heated at 50°C.
B: Ingredients (4) to (8) are dispersed, dissolved and mixed in a homo mixer, which is heated at 50°C.
C: a W/F emulsified lotion was obtained by gradually adding the above B, while the above A was being stirred.

### (Table 2-81)

**Table 2-81**

| Evaluation item | Compound (a2) | Compound (b2) | Compound (c2) | Compound (d2) | Compound (e2) |
|---|---|---|---|---|---|
| Usability | A | B | B | The treated powder was not uniformly dispersed, so no emulsion was produced.. | |
| Uniform finish | B | B | B | | |
| Long-lasting property | A | A | A | | |

### (Example 2-38 and Comparative Example 2-38) Production of emulsion type sunscreen creams

Emulsion type sunscreen creams having a composition shown in Table 2-82 were produced by the following method. Evaluation results were shown in Table 2-83.

### (Table 2-82)

**Table 2-82**

| Ingredient | wt. parts |
|---|---|
| (1)Isohexadecane | 20.0 |
| (2)Isotridecyl isononanoate | 7.0 |
| (3)Dimethyl silylated silica (Aerosil R972: Japan Aerosil Co., Ltd.) | 3.5 |
| (4)Finely particulate titanium oxide (10% treated product in Example 2-15 and Comparative Example 2-15) | 5.0 |
| (5)Finely particulate zinc oxide (7% treated product in Example 2-12 and Comparative Example 2-12) | 10.0 |
| (6)Ethanol | 5.0 |
| (7)Glycerin | 7.0 |
| (8)Purified water | to100.0 |
| (9)Antiseptic | approp. amount |

### (Producing method)

A: Ingredients (1) to (3) are uniformly mixed, and heated at 50 ° C in a disperser.
B: Ingredients (4) to (9) are heated at 50°C, and uniformly mixed and dispersed.
C: A powder-emulsified sunscreen cream was obtained by gradually adding the above B, while the above A was being stirred.

### (Table 2-83)

**Table 2-83**

| Evaluation item | Compound (a2) | Compound (b2) | Compound (c2) | Compound (d2) | Compound (e2) |
|---|---|---|---|---|---|
| Usability | B | B | B | The treated powder was not uniformly dispersed, so no emulsion was produced. | |
| Uniform finish | B | B | A | | |
| Long-lasting property | A | A | A | | |

### [Industrially applicability]

The surface-treated powders of the present invention can be used preferably as the surface-treated powders for cosmetic uses, but they can be applied to not only the cosmetics but also various fields of such as inks, paints, resin master batches, powder fillers to be blended into papers, etc., ceramic materials, magnetic materials, rare earths, optical materials, electroconductive materials, piezoelectric materials and the like.

## Claims

1. A surface-treated powder in which surfaces of particles of a powder to be surface-treated is coated with a surface-treating agent, **characterized in that** the surface-treating agent is a fluorine-containing copolymer obtained by copolymerizing monomers essentially comprising (a) a fluorine-containing monomer expressed by the following general formula (I) with (b) an alkoxy group-containing monomer expressed by the following general formula (II).
[Chemical formula 1] CH2=C(-X)-C(=O)-Y-[-(CH2)m-Z-]ₚ-(CH2)n-Rf (I)
wherein X is a hydrogen atom, a methyl group, a fluorine atom, a chlorine atom, a bromine atom, an iodine atom, a CFX1X2 in which X1 and X2 are a hydrogen atom, a fluorine atom or a chlorine atom, a cyano group, a straight-chain or branched-chain fluoroalkyl group having 1 to 20 carbon atoms, or a substituted or non-substituted benzyl group, a substituted or non-substituted phenyl group; Y is -O- or -NH-; Z is a direct bond, -S- or -SO2-; Rf is a fluoroalkyl group having 1 to 6 carbon atoms; m is 1 to 10, n is 0 to 10, and p is 0 or 1.
[Chemical structure 2] CH2=C(X1)-C(=O)-O-(RO)_{q}-X2 (II)
wherein X1 is a hydrogen atom or a methyl group; X2 is a hydrogen atom or an unsaturated or saturated hydrocarbon group having 1 to 22 carbon atoms; R is an alkylene group having 2 to 4 carbon atoms in which a part of or an entire part of hydrogen atoms may be replaced by a hydroxyl group or hydroxyl groups; and q is an integer of 1 to 50.

2. The surface-treated powder set forth in set forth in claim 1, wherein X 2 in the above formula (II) is a hydrogen atom.

3. The surface-treated powder set forth in claim 1 or 2, wherein the surface-treated powder has water-repellent and oil-repellent properties.

4. The surface-treated powder set forth in claim 1 or 2, wherein the surface-treated powder has hydrophilic and oil-repellent properties.

5. The surface-treated powder set forth in any one of any one of claims 1 to 4, wherein a crosslinkable monomer represented by the following general formula (III) is contained in constituting components of the fluorine-containing copolymer.
[Chemical structure 3] CH2=C(X3)-C(=O)-O-(R10)q-C(=O)-C(X3)=CH2 (III)
wherein each of the X3 groups is a hydrogen atom or a methyl group; Rl is an alkylene group having 2 to 10 carbon atoms in which a part or an entire part of hydrogen atoms may be replaced by a hydroxyl group or hydroxyl groups; and q is an integer of 1 to 50.

6. The surface-treated powder set forth in any one of claims 1 to 5, wherein the weight average molecular weight of the fluorine-containing copolymer is about 1,000 to about 1,000,000.

7. The surface-treated powder set forth in any one of claims 1 to 6, wherein the alkoxy group-containing monomer (b) is 10 to 400 parts by weight relative to 100 parts by weight of the fluorine-containing monomer (a) in the fluorine-containing copolymer.

8. The surface-treated powder set forth in any one of claims 1 to 7, wherein a coated amount of the fluorine-containing copolymer is 0.01 to 40 parts by weight relative to 100 parts by weight of the powder to be surface-treated.

9. The surface-treated powder set forth in any one of claims 1 to 8, wherein the surface-treating agent further contains one or more kinds of compounds selected from the group consisting of the following compounds: other fluorine-containing compound or compounds rather than the fluorine-containing copolymer, an organopolysiloxane, an alkylsilane, a polyether-modified silane, an organic titanate, a polyolefin, a hydrogenated lecithin (including a form of a salt), an acylated amino acid (including a form of a salt or a composition), an acidic ester oil, a fatty acid (including a form of a salt) and a dextrin fatty acid ester.

10. The surface-treated powder set forth in claims 9, wherein the fluorine-containing compound is at least one kind of a perfluoroalkyl phosphoric acid ester and a perfluoroalkyl silane having six or less carbon atoms, perfluoropolyether phosphoric acid ester and a perfluoropolyether silane.

11. The surface-treated powder set forth in any one of claims 1 to 10, wherein 0.01 to 39.99 parts by weight of the fluorine-containing copolymer and 39.99 to 0.01 parts by weight of one or more kinds of the group of the above-mentioned compounds are coated for 100 parts by weight of the powder to be surface-treated,

12. The surface-treated powder set forth in any one of claims 1 to 11, wherein the powder to be surface-treated is a powder usable for cosmetics.

13. The surface-treated powder set forth in claim 12, wherein the powder usable for the cosmetics is an inorganic powder usable for the cosmetics.

14. The surface-treated powder set forth in claim 13, wherein the inorganic powder is any one or more kinds of sericite, mica, kaolin, talc, silica, barium sulfate, boron nitride, titanium oxide, zinc oxide, iron oxide and a pearl pigment.

15. The surface-treated powder set forth in claim 12, wherein the powder usable for the cosmetics is an organic powder usable for the cosmetics.

16. A cosmetic in which the surface-treated powder set forth in any one of claims 1 to 15 is blended.

17. The cosmetic set forth in claim 16, which further contains at least one of an oily ingredient, an aqueous ingredient and a surface-active agent as constituting ingredients.

18. The cosmetic set forth in claim 16 or 17, which is any of a skin-care cosmetic, a hair cosmetic, a makeup cosmetic and an UV rays protecting cosmetic.

19. The cosmetic set forth in any one of claims 16 to 18, wherein a product formulation is any of a liquid form, an emulsion form, a creamy form, a solid form, a paste form, a gel form, a powdery form, a multi-layer form, a mousse form and a spray form.
